(19) Europäisches Patentamt
European Patent Office
Office européen des brevets

(11) EP 0 906 271 B1

(12) EUROPÄISCHE PATENTSCHRIFT

(45) Veröffentlichungstag und Bekanntmachung des Hinweises auf die Patenterteilung:
**06.02.2002 Patentblatt 2002/06**

(21) Anmeldenummer: **97925946.2**

(22) Anmeldetag: **26.05.1997**

(51) Int Cl.[7]: **C07C 271/22**, A01N 47/12

(86) Internationale Anmeldenummer:
**PCT/EP97/02687**

(87) Internationale Veröffentlichungsnummer:
**WO 97/46518 (11.12.1997 Gazette 1997/53)**

(54) **CARBAMOYLCARBONSÄUREAMIDOXIME**

CARBAMOYL CARBOXYLIC ACID AMIDE OXIMES

AMIDOXIMES D'ACIDE CARBAMOYLCARBOXYLIQUE

(84) Benannte Vertragsstaaten:
**AT BE CH DE DK ES FI FR GB GR IE IT LI LU MC NL PT SE**

(30) Priorität: **31.05.1996 DE 19621841**

(43) Veröffentlichungstag der Anmeldung:
**07.04.1999 Patentblatt 1999/14**

(73) Patentinhaber: **BASF AKTIENGESELLSCHAFT 67056 Ludwigshafen (DE)**

(72) Erfinder:
- **WETTERICH, Frank**
**D-67112 Mutterstadt (DE)**
- **EICKEN, Karl**
**D-67157 Wachenheim (DE)**
- **KIRSTGEN, Reinhard**
**D-67434 Neustadt (DE)**
- **AMMERMANN, Eberhard**
**D-64646 Heppenheim (DE)**
- **LORENZ, Gisela**
**D-67434 Hambach (DE)**
- **STRATHMANN, Siegfried**
**D-67117 Limburgerhof (DE)**

(56) Entgegenhaltungen:
**WO-A-95/23784** **WO-A-95/23786**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Carbamoylcarbonsäureamidoxime der Formel I

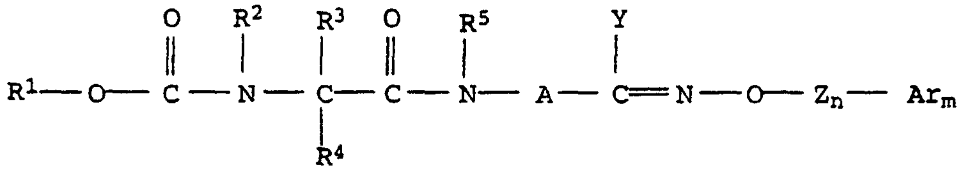

(I)

sowie deren Salze, in denen die Variablen die folgende Bedeutung haben:

$R^1$ $C_1$-$C_8$-Alkyl, $C_2$-$C_8$-Alkenyl oder $C_2$-$C_8$-Alkinyl, wobei diese Reste partiell oder vollständig halogeniert sein und/oder eine, oder unabhängig voneinander zwei oder drei der folgenden Gruppen tragen können: Cyano, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, $C_3$-$C_7$-Cycloalkyl, $C_3$-$C_7$-Cycloalkenyl, Aryl, Aryloxy und Heteroaryl, wobei die cyclischen Reste ihrerseits einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy und Heteroaryl,

$C_3$-$C_7$-Cycloalkyl oder $C_3$-$C_7$-Cycloalkenyl, wobei diese Reste partiell oder vollständig halogeniert sein und/oder eine, oder unabhängig voneinander zwei oder drei der folgenden Gruppen tragen können: Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy und Aryl- ($C_1$-$C_4$)-alkyl, wobei die cyclischen Gruppen einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy,

einen nicht-aromatischen 4- bis 8gliedrigen Ring, welcher als Ringglieder neben Kohlenstoff noch eines oder zwei der Heteroatome Sauerstoff, Schwefel und Stickstoff enthalten kann, wobei die Kohlenstoffatome im Ring eine oder zwei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy und wobei das zweite und jedes weitere Stickstoffatom als Heteroatom im Ring Wasserstoff oder eine $C_1$-$C_4$-Alkylgruppe trägt,

Aryl oder Heteroaryl, wobei diese Reste eine, oder unabhängig voneinander zwei oder drei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy und Heteroaryl, in denen die cyclischen Substituenten ihrerseits einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Alkoxycarbonyl,

$W^1W^2C$=N-, wobei $W^1$ $C_1$-$C_8$-Alkyl bedeutet, welches partiell oder vollständig halogeniert sein und/oder eine, oder unabhängig voneinander zwei oder drei der folgenden Gruppen tragen kann: Cyano, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, Aryl, Aryloxy und Heteroaryl, wobei die cyclischen Gruppen ihrerseits einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy,

$C_2$-$C_8$-Alkenyl oder $C_2$-$C_8$-Alkinyl, wobei diese Reste partiell oder vollständig halogeniert sein und/oder eine, oder unabhängig voneinander zwei oder drei der folgenden Gruppen tragen können: Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy, wobei die cyclischen Gruppen ihrerseits einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy,

$C_3$-$C_7$-Cycloalkyl oder $C_3$-$C_7$-Cycloalkenyl, wobei diese Reste eine, oder unabhängig voneinander zwei oder drei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryl-($C_1$-$C_4$)-alkyl, wobei die Gruppen, welche Aryl enthalten, einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy,

Aryl oder Heteroaryl, wobei diese Reste eine, oder unabhängig voneinander zwei oder drei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy und

$W^2$ Wasserstoff oder unabhängig von diesen eine der Gruppen $W^1$;

$R^2$ Wasserstoff oder $C_1$-$C_8$-Alkyl oder $C_3$-$C_7$-Cycloalkyl, welche partiell oder vollständig halogeniert sein können;

$R^3$ $C_1$-$C_8$-Alkyl, $C_3$-$C_7$-Cycloalkyl, wobei diese Reste eine, oder unabhängig voneinander zwei oder drei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Alkoxycarbonyl oder Phenyl-($C_1$-$C_4$)-alkyl, wobei der Phenylrest eine, oder unabhängig voneinander zwei oder drei der folgenden Gruppen tragen kann: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy;

$R^4$ Wasserstoff oder eine der bei $R^3$ genannten Gruppen oder

$R^3$ und $R^4$ gemeinsam mit dem C-Atom an das sie gebunden sind einen 4- bis 8gliedrigen Ring, welcher als Ringglieder neben Kohlenstoff noch ein oder zwei der Heteroatome Sauerstoff, Schwefel und Stickstoff enthalten kann, wobei die Kohlenstoffatome im Ring eine oder zwei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy und wobei Stickstoff als Heteroatom Wasserstoff oder eine $C_1$-$C_4$-Alkylgruppe trägt;

$R^5$ einen Rest $R^2$;

A $C_1$-$C_8$-Alkylen, wobei diese Reste partiell oder vollständig halogeniert sein und/oder eine, oder unabhängig voneinander zwei oder drei der folgenden Gruppen tragen können: Cyano, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Alkoxycarbonyl, Aryl und Aryloxy, oder
$C_3$-$C_7$-Cycloalkylen, wobei diese Reste partiell oder vollständig halogeniert sein und/oder eine, oder unabhängig voneinander zwei oder drei der folgenden Gruppen tragen können: Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy und Aryl-($C_1$-$C_4$)-alkyl, wobei die cyclischen Gruppen ihrerseits einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy;

Y Wasserstoff,

$C_1$-$C_8$-Alkyl, wobei dieser Rest partiell oder vollständig halogeniert sein und/oder eine, oder unabhängig voneinander zwei oder drei der folgenden Gruppen tragen kann: Cyano, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Alkoxycarbonyl, Aryl und Aryloxy,

$C_3$-$C_7$-Cycloalkyl, wobei dieser Rest partiell oder vollständig halogeniert sein und/oder eine, oder unabhängig voneinander zwei oder drei der folgenden Gruppen tragen kann: Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy und Aryl-($C_1$-$C_4$)-alkyl, wobei die cyclischen Gruppen ihrerseits einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy oder

Aryl, wobei diese Reste eine, oder unabhängig voneinander zwei oder drei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy,

$C_1$-$C_4$-Alkylthio, $C_1$-$C_4$-Alkoxycarbonyl, Aryl und Aryloxy;

Z $C_1$-$C_8$-Alkylen, wobei dieser Rest partiell oder vollständig halogeniert sein und/oder eine, oder unabhängig voneinander zwei oder drei der folgenden Gruppen tragen kann: Cyano, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Alkoxycarbonyl, Aryl und Aryloxy,

$C_1$-$C_8$-Alkylenoxy, wobei dieser Rest partiell oder vollständing halogeniert sein und/oder eine, oder unabhängig voneinander zwei oder drei der folgenden Gruppen tragen kann: Cyano, $C_1$-$C_4$-Alkylthio und $C_1$-$C_4$-Alkoxycarbonyl, Aryl und Aryloxy,

$C_3$-$C_7$-Cycloalkylen wobei dieser Rest partiell oder vollständig halogeniert sein und/oder eine, oder unabhängig voneinander zwei oder drei der folgenden Gruppen tragen kann: Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy und Aryl-($C_1$-$C_4$)-alkyl, wobei die cyclischen Gruppen ihrerseits einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy;

n 0, 1 oder 2;

Ar Aryl oder Heteroaryl, wobei diese Reste eine, oder unabhängig voneinander, zwei oder drei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy und Heteroaryl, in denen die cyclischen Substituenten ihrerseits einen, oder unabhängig voneinander zwei oder drei der folgenden Substituenten tragen können: Harogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Alkoxycarbonyl;

m die Zahl 1 bedeutet.

Daneben betrifft die Erfindung Verfahren zur Herstellung der Verbindungen I, die Verbindungen I enthaltende Mittel sowie die Verwendung der Verbindungen I und der Mittel zur Bekämpfung von Schadpilzen.
Carbamoylcarbonsäureamide und -hydrazide mit fungizider Wirkung sind dem Fachmann bekannt (vgl. WO-A 95/23 784, WO-A 95/23 785, WO-A 95/23 786, WO-A 95/33 710, EP-A 672 654, WO-A 96/7 638 und die ältere deutsche Anmeldung mit dem Aktenzeichen 195 31 814.5).
In WO 95/23786 und WO 95/23784 werden Carbamoylcarbonsäureamid-Derivate beschrieben. Die erfindungsgemäßen Carbamoylcarbonsäureamidoxime unterscheiden side von den in WO 95/23786 und WO 95/23784 beschriebenen Verbindungen dadurch, daß die Amidgruppe durch eine substituierte Oximgruppe substituiert ist.
Die Wirkung der in den vorgenannten Druckschriften beschriebenen Verbindungen gegen Schadpilze kann jedoch noch nicht befriedigen.
Der vorliegenden Erfindung lagen daher neue Carbamoylcarbonsäure-Derivate mit verbesserten Eigenschaften bei der Bekämpfung von Schadpilzen als Aufgabe zugrunde.
Demgemäß wurden die eingangs definierten Verbindungen I, Verfahren zu ihrer Herstellung, sie enthaltende Mittel und ihre Verwendung und die Verwendung der Mittel zur Bekämpfung von Schadpilzen gefunden.
Die Verbindungen I können in an sich bekannter Weise ausgehend von den entsprechenden Carbamoylcarbonsäuren II hergestellt werden. Bevorzugt erhält man die Verbindungen I nach den im folgenden beschriebenen Verfahren A bzw. B (die Literaturzitate "Houben-Weyl" beziehen sich auf: Houben-Weyl, Methoden der Organischen Chemie, 4. Auflage, Thieme Verlag, Stuttgart).

Verfahren A

Die Carbamoylcarbonsäureamidoxime I erhält man (vgl. das folgende Schema 1), indem man die Carbamoylcarbonsäuren II mit den Aminen III zu den Verbindungen IV umsetzt, die Verbindungen IV zu Carbonylverbindungen V oxidiert diese wiederum mit Oxyaminen VI zur Reaktion bringt.

Schema 1

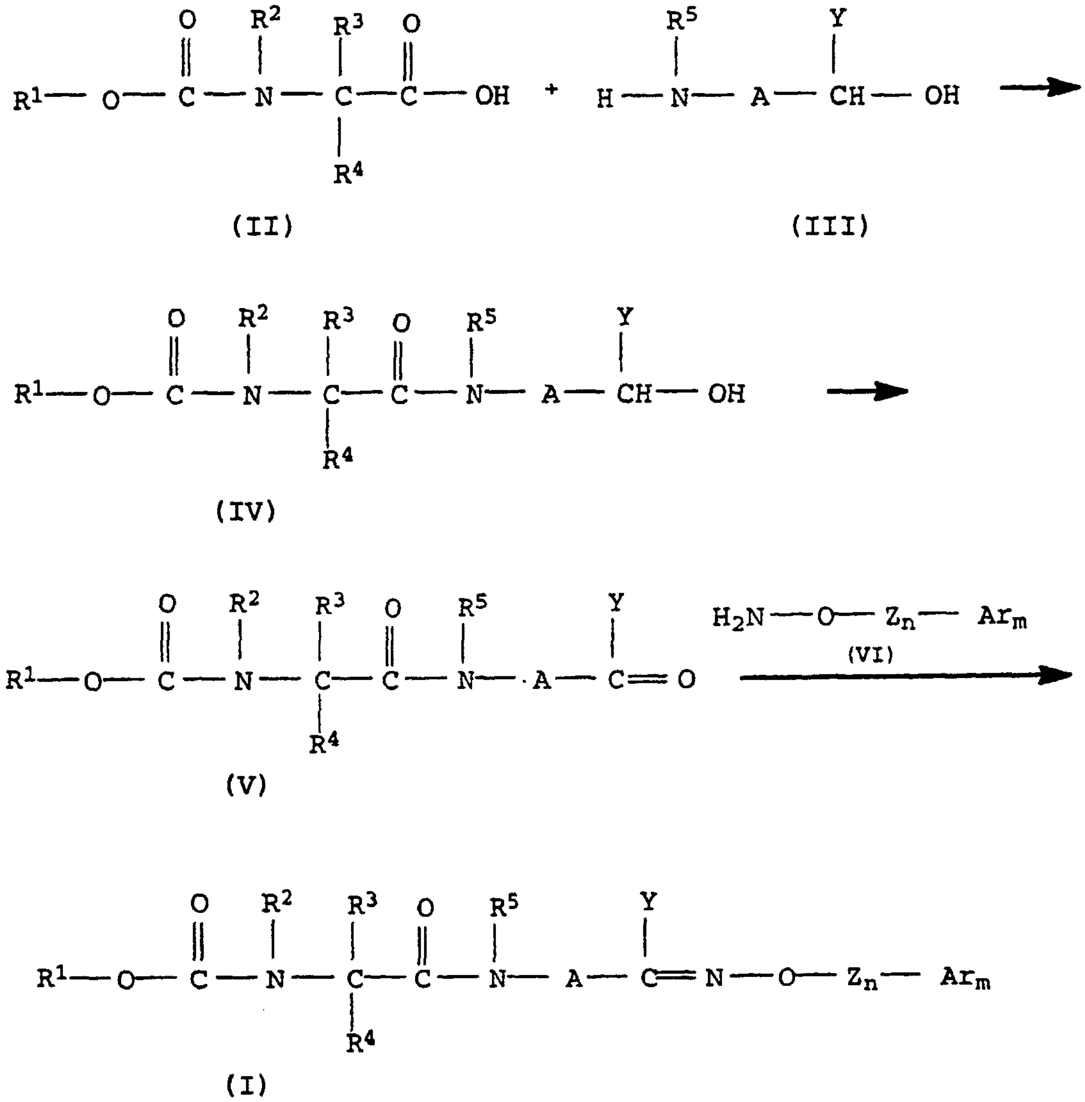

(II) (III)

(IV)

(VI)

(V)

(I)

Die Carbamoylcarbonsäuren II sind bekannt oder können nach bekannten Methoden, vor allem ausgehend von den zugrundeliegenden Aminosäuren, hergestellt werden (vgl. "Houben-Weyl", Band 15/1, Seite 46 bis Seite 305, vor allem Seite 117 bis Seite 125).

Die Amine III sind ebenfalls bekannt oder können leicht erhalten werden (vgl. J. Amer. Chem. Soc. 110, Seite 5195 ff. (1988); Tetrahedron 28, Seite 3475 ff. (1972)).

Vorzugsweise arbeitet man bei der Darstellung der Amide IV so, daß man zunächst die Carbamoylcarbonsäuren II in carboxyaktivierte Derivate, vor allem in Acylcyanide oder Anhydride, überführt (vgl. Tetrahedron Letters, Band 18, Seite 1595 bis Seite 1598 (1973), bzw. "Houben-Weyl". Band 15/1, Seite 28 bis Seite 32). Diese Derivate werden dann mit den Aminen III in Gegenwart von Basen zur Reaktion gebracht.

Zur Herstellung der carboxyaktivierten Acylcyanide eignet sich z.B. die Reaktion der Carbamoylcarbonsäuren II mit Cyanphosphonsäurediethylester, vor allem in einem inerten Lösungsmittel wie Tetrahydrofuran oder Toluol.

Zur Herstellung der carboxyaktivierten Anhydride ist die Umsetzung der Carbamoylcarbonsäure II mit Kohlensäurechloriden wie Chlorameisensäure-iso-butylester in Gegenwart von Basen und gegegebenfalls in einem inerten Lösungsmittel wie Toluol oder Tetrahydrofuran bevorzugt.

Die Umsetzung der Amine III mit den carboxyaktivierten Carbamoylcarbonsäuren II erfolgt vorzugsweise in einem Lösungsmittel wie Dichlormethan, Tetrahydrofuran oder Toluol.

Als Basen können insbesondere die Amine III selbst dienen, wobei man sie aus dem Rohprodukt üblicherweise zurückgewinnt.

In einer bevorzugten Ausführungsform dieser Verfahrensstufe werden die Carbamoylcarbonsäure II, das Amin III, das zur Erzeugung des caboxyaktivierten Derivates der Carbamoylcarbonsäure II geeignete Reagens und die Base im Eintopfverfahren, gegebenenfalls in einem inerten Lösungsmittel, zur Reaktion gebracht und das Rohprodukt anschließend in an sich bekannter Weise auf das Carbamoylcarbonsäureamid I aufgearbeitet.

Die Oxidation der Amide IV zu den Verbindungen V kann in an sich bekannter Weise, insbesondere mittels Natriumhypochlorit in gepufferter Lösung und in Gegenwart katalytischer Mengen von 2,2,6,6-Tetramethylpiperidin-N-oxid (TEMPO) (vgl. J. Org. Chem. 52, Seite 2559 ff. (1987); Org. Synth. 69, Seite 212 ff. (1990)) erfolgen.

Die Oxyamine VI sind nach allgemein bekannten Methoden erhältlich (vgl. DE-A 36 15 473). Die Umsetzung mit den Verbindungen V ist beschrieben in EP-A 588 146.

Eine Variante dieses Verfahrensschrittes von den Verbindungen V zu den Verbindungen I besteht darin, daß man zunächst (vgl. Schema 2) die Verbindungen V mit einem Salz des Hydroxylamins in Gegenwart einer Base zu den Oximen VIII umsetzt und letztere nach Deprotonierung mit Verbindungen IX, in der X' eine übliche nucleofuge Abgangsgruppe wie Brom bedeutet, zur Reaktion bringt.

Schema 2

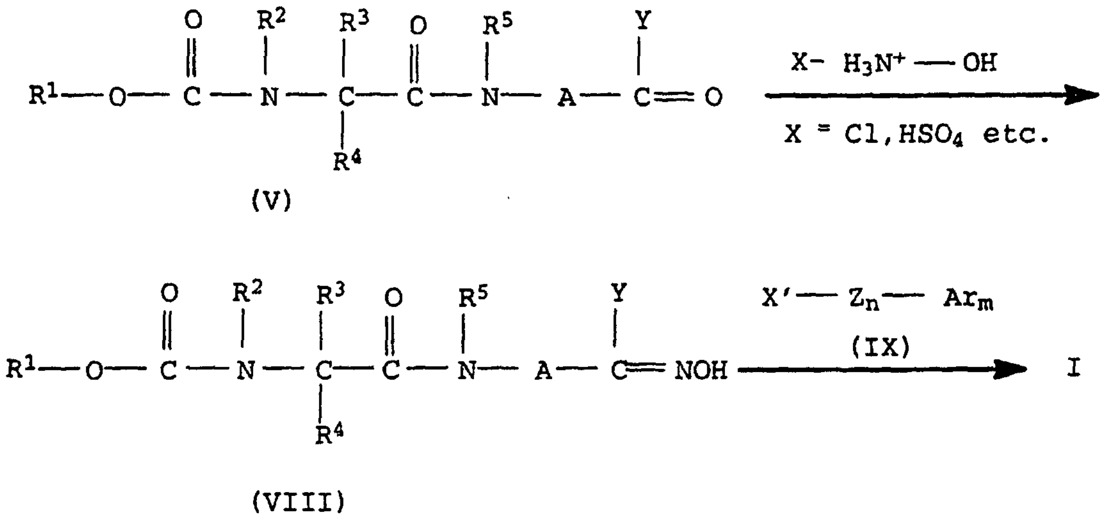


(V)

(VIII)

Verfahren B

Die Carbamoylcarbonsäureamide I, erhält man, indem man die Carbamoylcarbonsäureamide I, in denen die Gruppe $R^1$-O-(CO) für eine Schutzgruppe steht, die in an sich bekannter Weise abgespalten werden kann, in Aminosäureamide IV überführt und diese mit Chlorformyloximen V in Gegenwart von Basen umsetzt.

Stufe Ba: Herstellung der Aminosäureamide VII

Die Abspaltung der Gruppe $R^1$-O-(CO) aus den Carbamoylcarbonsäureamidoximen I (vgl. Schema 3) kann in an sich bekannter Weise durchgeführt werden (vgl. "Houben-Weyl", Band 15/1, Seite 46 bis Seite 305, vor allem Seite 126 bis Seite 129).

Schema 3

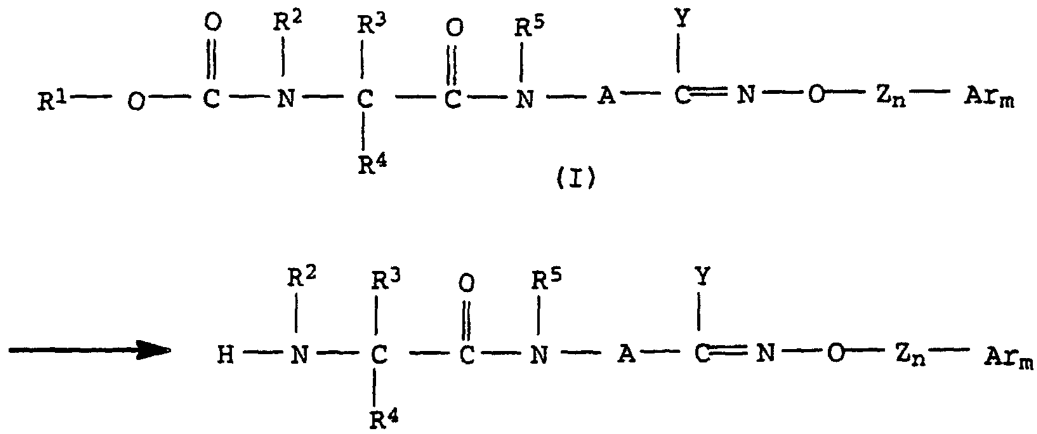

(I)

(VII)

Geeignete abspaltbare Gruppen enthalten als Rest $R^1$ eine tert.-Butyl- oder die Benzylgruppe.

Im Falle von $R^1$ = tert.-Butyl beispielsweise erfolgt die Abspaltung üblicherweise durch Umsetzung mit einer Säure, insbesondere einer Protonensäure wie z.B. Salzsäure oder Trifluoressigsäure (ibid., Seite 126 bis Seite 129).

Die als Ausgangsstoffe geeigneten Carbamoylcarbonsäureamide I können nach bekannten Verfahren (vgl. "Houben-Weyl", Band 15/1, Seite 28 bis Seite 32) oder insbesondere nach dem erfindungsgemäßen Verfahren A gewonnen werden.

Stufe Bb: Herstellung der Carbamoylcarbonsäureamide I

Die aus der Synthesestufe (Ba) resultierenden Aminosäureamide VII werden mit den Chlorformyloximen VIII in Gegenwart von Basen umgesetzt (Schema 4).

Schema 4

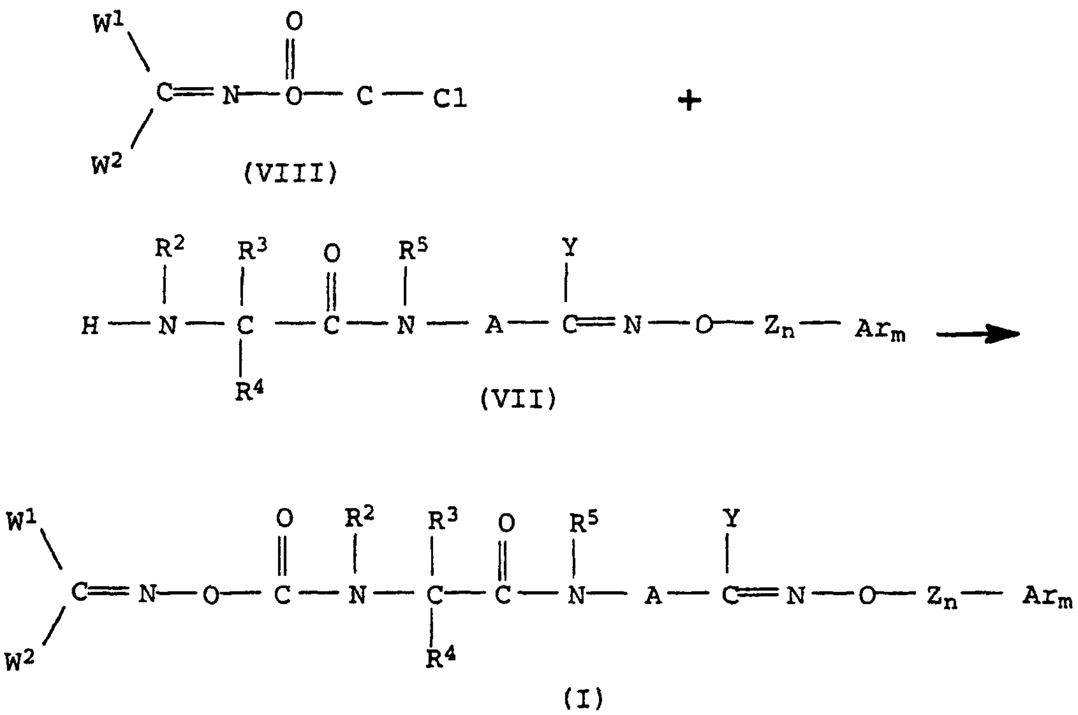

(VIII) (VII) (I)

Die Chlorformyloxime VIII sind bekannt oder können nach bekannten Verfahren, wie z.B. durch Phosgenierung von Oximen, hergestellt werden (vgl. z.B. Zeitschrift für Chemie, Band 9, Seite 344 bis Seite 345 (1967)).

Die Umsetzung wird vorzugsweise in einem organischen Lösungsmittel, vor allem Toluol, Methylenchlorid oder Tetrahydrofuran, oder Gemischen dieser Lösungsmittel durchgeführt.

Als Basen kommen anorganische und organische Basen gleichermaßen in Betracht, wobei organische Basen und hierunter wiederum tertiäre Amine wie Triethylamin, Pyridin und N-Methylpiperidin bevorzugt sind.

Die Umsetzung wird in der Regel bei Temperaturen von (-40) bis 50, vorzugsweise (-10) bis 20°C durchgeführt.

Im übrigen ist die Durchführung dieser Reaktion dem Fachmann geläufig, so daß es keiner weiteren Ausführungen hierzu bedarf (vgl. "Houben-Weyl", Band 15/1, Seite 117 bis Seite 125, bzw. Dev. Endocrinol., 13 (Neurohypophyseal Pept. Horm. Other Biol. Act. Pept.), Seite 37 bis Seite 47 (1981)).

Die nach den Verfahren A bzw. B erhaltenen Reaktionsgemische werden in üblicher Weise aufgearbeitet, z. B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit werden können. Sofern die Zwischen- und Endprodukte als Feststoffe erhalten werden, kann die Reinigung auch beispielsweise durch Umkristallisieren oder Digerieren erfolgen.

Die Verbindungen der Formel I können gegebenenfalls in Abhängigkeit von der Art der Substituenten als geometrische und/oder optische Isomere oder Isomerengemische vorliegen. Sowohl die reinen Isomeren als auch die Gemische der Isomeren weisen die fungizide Wirkung auf.

Teil der Erfindung sind auch die Salze der säurebeständigen Verbindungen I, welche basische Zentren, vor allem basische Stickstoffatome enthalten, insbesondere mit Mineralsäuren wie Schwefelsäure und Phosphorsäure oder Lewis-Säuren wie Zinkchlorid. Üblicherweise kommt es hierbei auf die Art des Salzes nicht an. Im Sinne der Erfindung sind solche Salze bevorzugt, die die von Schadpilzen oder tierischen Schädlingen freizuhaltenden Pflanzen, Flächen, Materialien oder Räume nicht schädigen und die Wirkung der Verbindungen I nicht beeinträchtigen. Besonders bedeutsam sind derartige Salze, welche für landwirtschaftliche Zwecke geeignet sind.

Die Salze der Verbindungen I sind in an sich bekannter Weise zugänglich, vor allem durch Umsetzen der entsprechenden Verbindungen I mit den genannten Säuren in Wasser oder einem inerten organischen Lösungsmittel bei Temperaturen von (-80) bis 120, vorzugsweise 0 bis 60 °C.

Bei den eingangs angegebenen Definitionen der Verbindungen I wurden Sammelbegriffe verwendet, die allgemein repräsentativ für die folgenden Gruppen stehen:

Halogen: Fluor, Chlor, Brom und Iod;

Alkyl: geradkettige oder verzweigte Alkylgruppen mit 1 bis 8 Kohlenstoffatomen, z.B. $C_1$-$C_6$-Alkyl wie Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 2,2-Dimethylpropyl, 1,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 2, 2-Dimethylbutyl, 3,3-Dimethylbutyl, 1,2-Dimethylbutyl, 1,3-Dimethylbutyl, 2,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;

Halogenalkyl bzw. partiell oder vollständig halogeniertes Alkyl: geradkettige oder verzweigte Alkylgruppen mit 1 bis 4 bzw. 8 Kohlenstoffatomen (wie vorstehend genannt), wobei in diesen Gruppen die Wasserstoffatome partiell oder vollständig durch Halogenatome (wie vorstehend genannt) ersetzt sein können, z.B. $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;

Alkoxy: geradkettige oder verzweigte Alkoxygruppen mit 1 bis 4 Kohlenstoffatomen, z.B. $C_1$-$C_3$-Alkoxy wie Methyloxy, Ethyloxy, Propyloxy und 1-Methylethyloxy;

Alkoxyalkyl: geradkettige oder verzweigte Alkylgruppen mit 1 bis 8 Kohlenstoffatomen (wie vorstehend genannt), welche in einer beliebigen Position eine geradkettige oder verzweigte Alkoxygruppe (wie vorstehend genannt) mit im Falle von $C_1$-$C_4$-Alkoxyalkyl 1 bis 4 Kohlenstoffatomen tragen, wie Methoxymethyl, Ethoxymethyl, n-Propoxymethyl, n-Butoxymethyl, 1-Methoxyethyl, 2-Methoxyethyl, 1-Ethoxyethyl, 2-Ethoxyethyl, 2-n-Propoxyethyl und 2-Butoxyethyl;

Halogenalkoxy: geradkettige oder verzweigte Alkoxygruppen mit 1 bis 4 Kohlenstoffatomen (wie vorstehend genannt), wobei in diesen Gruppen die Wasserstoffatome partiell oder vollständig durch Halogenatome (wie vorstehend genannt) ersetzt sein können, z.B. $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;

Alkylthio: geradkettige oder verzweigte Alkylgruppen mit 1 bis 4 Kohlenstoffatomen (wie vorstehend genannt), welche über ein Schwefelatom (-S-) an das Gerüst gebunden sind, z.B. $C_1$-$C_4$-Alkylthio wie Methylthio, Ethylthio, Propylthio, 1-Methylethylthio, n-Butylthio und tert.-Butylthio;

Alkoxycarbonyl: geradkettige oder verzweigte Alkoxygruppen mit 1 bis 4 C-Atomen (wie vorstehend genannt), welche über eine Carbonylgruppe (-CO-) an das Gerüst gebunden sind;

Alkenyl: geradkettige oder verzweigte Alkenylgruppen mit 2 bis 8 Kohlenstoffatomen und einer Doppelbindung in einer beliebigen Position, z.B. $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 2-Methyl-1-propenyl, 1-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;

Alkinyl: geradkettige oder verzweigte Alkinylgruppen mit 2 bis 8 Kohlenstoffatomen und einer Dreifachbindung in einer beliebigen Position, z.B. $C_2$-$C_6$-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-lmethyl-2-propinyl;

Cycloalkyl: monocyclische Alkylgruppen mit 3 bis 7 Kohlenstoffringgliedern, z.B. $C_3$-$C_7$-Cycloalkyl wie Cyclopropyl, Cyclobutyl, Cyclopentyl, Cyclohexyl und Cycloheptyl;

Cycloalkenyl: monocyclische Alkylgruppen mit 5 bis 7 Kohlenstoffringgliedern die eine oder mehrere Doppelbindungen enthalten z.B. $C_5$-$C_7$-Cycloalkenyl wie Cyclopentenyl, Cyclohexenyl und Cycloheptenyl;

nicht-aromatische 4- bis 8gliedrigen Ringe, welcher als Ringglieder neben Kohlenstoff noch ein oder zwei Sauerstoff-, Schwefel- oder Stickstoffatome enthalten wie gesättigte 5- oder 6gliedrige Ringe mit 1 oder 2 Stickstoff- und/oder Sauerstoffatomen wie 3-Tetrahydrofuranyl, 1-Piperidinyl, 2-Piperidinyl, 3-Piperidinyl, 4-Piperidinyl, 2-Tetrahydropyranyl, 3-Tetrahydropyranyl, 4-Tetrahydropyranyl, 2-Morpholinyl und 3-Morpholinyl;

Aryl: monocyclische oder polycyclische aromatische Gruppen mit 6 bis 10 C-Atomen wie Phenyl und Naphthyl;

Arylalkyl: Arylgruppen (wie vorstehend genannt), welche im Falle von Aryl-($C_1$-$C_4$)-alkyl über Alkylgruppen mit 1 bis 4 Kohlenstoffatomen (wie vorstehend genannt) an das Gerüst gebunden sind, z.B. Phenyl - ($C_1$-$C_4$)-alkyl wie Benzyl, 2-Phenylethyl, 3-Phenylpropyl, 4-Phenylbutyl, 1-Phenylethyl, 1-Phenylpropyl und 1-Phenylbutyl;

Aryloxy: Arylgruppen (wie vorstehend genannt), welche über ein Sauerstoffatom (-O-) an das Gerüst gebunden sind wie Phenoxy, 1-Naphthoxy und 2-Naphthoxy;

Heteroaryl: aromatische mono- oder polycyclische Reste, welche neben Kohlenstoffringgliedern zusätzlich 1 bis 4 Stickstoffatome oder 1 bis 3 Stickstoffatome und ein Sauerstoff- oder ein Schwefelatom oder ein Sauerstoff- oder ein Schwefelatom enthalten können, z.B.:

- 5gliedriges Heteroaryl, enthaltend 1 bis 3 Stickstoffatome: 5-Ring-Heteroarylgruppen, welche neben Kohlenstoffatomen 1 bis 3 Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;

- 5gliedriges Heteroaryl, enthaltend 1 bis 4 Stickstoffatome oder 1 bis 3 Stickstoffatome und 1 Schwefelatom oder Sauerstoffatom oder 1 Sauerstoff- oder 1 Schwefelatom: 5-Ring-Heteroarylgruppen, welche neben Kohlenstoffatomen 1 bis 4 Stickstoffatome oder 1 bis 3 Stickstoffatome und 1 Schwefel- oder Sauerstoffatom oder 1 Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl;

- benzokondensiertes 5gliedriges Heteroaryl, enthaltend 1 bis 3 Stickstoffatome oder 1 Stickstoffatom und/oder ein Sauerstoff- oder Schwefelatom: 5-Ring-Heteroarylgruppen, welche neben Kohlenstoffatomen 1 bis 4 Stickstoffatome oder 1 bis 3 Stickstoffatome und 1 Schwefel- oder Sauerstoffatom oder 1 Sauerstoff- oder ein Schwefelatom als Ringglieder enthalten können, und in welchen 2 benachbarte Kohlenstoffringglieder oder 1 Stickstoff- und 1 benachbartes Kohlenstoffringglied durch eine Buta-1,3-dien-1,4-diylgruppe verbrückt sein können;

- über Stickstoff gebundenes 5gliedriges Heteroaryl, enthaltend 1 bis 4 Stickstoffatome, oder über Stickstoff gebundenes benzokondensiertes 5gliedriges Heteroaryl, enthaltend 1 bis 3 Stickstoffatome: 5-Ring-Heteroarylgruppen, welche neben Kohlenstoffatomen 1 bis 4 Stickstoffatome bzw. 1 bis 3 Stickstoffatome als Ringglieder enthalten können, und in welchen 2 benachbarte Kohlenstoffringglieder oder ein Stickstoffund ein benachbartes Kohlenstoffringglied durch eine Buta-1,3-dien-1,4-diylgruppe verbrückt sein können, wobei diese

Ringe über eines der Stickstoffringglieder an das Gerüst gebunden sind;

- 6gliedriges Heteroaryl, enthaltend 1 bis 3 bzw. 1 bis 4 Stickstoffatome: 6-Ring-Heteroarylgruppen, welche neben Kohlenstoffatomen 1 bis 3 bzw. 1 bis 4 Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;

- benzokondensiertes 6gliedriges Heteroaryl, enthaltend 1 bis 4 Stickstoffatome: 6-Ring-Heteroarylgruppen, in welchen 2 benachbarte Kohlenstoffringglieder durch eine Buta-1,3-dien-1,4-diylgruppe verbrückt sein können, z.B. Chinolin, Isochinolin, Chinazolin und Chinoxalin.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.

Im Hinblick auf ihre biologische Wirkung gegen Schadpilze und tierische Schädlinge sind Verbindungen I bevorzugt, in denen die Reste in den folgenden Bedeutungen, und zwar für sich allein oder in Kombination, stehen:

$R^1$ iso-Propyl, sec-Butyl, tert.-Butyl oder Phenyl;

$R^2$ Wasserstoff;

$R^3$ und $R^4$: Einer dieser Reste Wasserstoff und der andere iso-Propyl, wobei das Kohlenstoffatom welches die Reste $R^3$ und $R^4$ trägt, insbesondere bei diesen und daneben bei jeglichen Bedeutungen der beiden Reste $R^3$ und $R^4$ vorzugsweise die S-Konfiguration aufweist;

$R^5$ Wasserstoff;

A $CH_2CH_2$, vorzugsweise $CH_2$ und $C(CH_3)_2$, insbesondere $CH(CH_3)CH_2$ und vor allem $CH(CH_3)$, wobei der Rest $CH(CH_3)$ vorzugsweise racemisch ist oder die R-Konfiguration aufweist;

Y Phenyl und insbesondere Wasserstoff oder Methyl;

Z $CH_2O$, vorzugsweise $CH_2CH_2$, insbesondere $CH(CH_3)$ und vor allem $CH_2$;

n 1 oder 2;

Ar gebenenfalls, vor allem durch Chlor, Methyl, Methoxy oder Cyano, substituiertes Phenyl;

m 1.

Im Hinblick auf ihre biologische Wirkung ganz besonders bevorzugt sind die in der folgenden Tabelle A wiedergegebenen Verbindungen I'.

Tabelle A

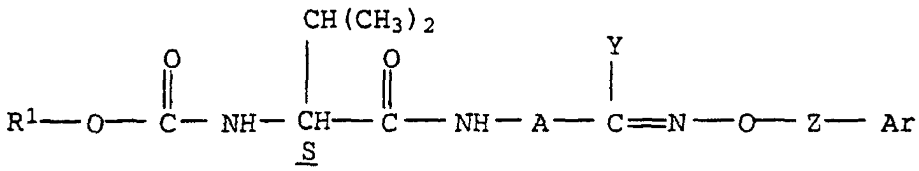

(I')

Tabelle A

| Nr. | $R^1$ | A | Y | Z | Ar |
|---|---|---|---|---|---|
| 1.1. | $CH(CH_3)_2$ | $CH(CH_3)$ | $CH_3$ | $CH_2$ | $4\text{-}Cl\text{-}C_6H_4$ |
| 1.2. | $CH(CH_3)_2$ | $CH(CH_3)$ | $CH_3$ | $CH_2$ | $4\text{-}CH_3\text{-}C_6H_4$ |
| 1.3. | $CH(CH_3)_2$ | $CH(CH_3)$ | $CH_3$ | $CH_2$ | $4\text{-}CH_3O\text{-}C_6H_4$ |
| 1.4. | $CH(CH_3)_2$ | $CH(CH_3)$ | $CH_3$ | $CH_2$ | $4\text{-}CN\text{-}C_6H_4$ |
| 1.5. | $CH(CH_3)_2$ | $CH(CH_3)$ | $CH_3$ | $CH_2$ | $3\text{-}Cl\text{-}C_6H_4$ |
| 1.6. | $CH(CH_3)_2$ | $CH(CH_3)$ | $CH_3$ | $CH_2$ | $3\text{-}CH_3\text{-}C_6H_4$ |
| 1.7. | $CH(CH_3)_2$ | $CH(CH_3)$ | $CH_3$ | $CH_2$ | $3\text{-}CH_3O\text{-}C_6H_4$ |
| 1.8. | $CH(CH_3)_2$ | $CH(CH_3)$ | $CH_3$ | $CH_2$ | $3\text{-}CN\text{-}C_6H_4$ |
| 1.9. | $CH(CH_3)_2$ | $CH(CH_3)$ | $CH_3$ | $CH_2$ | $2\text{-}Cl\text{-}C_6H_4$ |
| 1.10. | $CH(CH_3)_2$ | $CH(CH_3)$ | $CH_3$ | $CH_2$ | $2\text{-}CH_3\text{-}C_6H_4$ |
| 1.11. | $CH(CH_3)_2$ | $CH(CH_3)$ | $CH_3$ | $CH_2$ | $2\text{-}CH_3O\text{-}C_6H_4$ |
| 1.12. | $CH(CH_3)_2$ | $CH(CH_3)$ | $CH_3$ | $CH_2$ | $2\text{-}CN\text{-}C_6H_4$ |
| 1.13. | $CH(CH_3)_2$ | $CH(CH_3)$ | $CH_3$ | $CH_2$ | $3,4\text{-}(CH_3O)_2C_6H_3$ |
| 1.14. | $CH(CH_3)_2$ | $CH(CH_3)$ | $CH_3$ | $CH_2$ | $2,4\text{-}Cl_2\text{-}C_6H_3$ |
| 1.15. | $CH(CH_3)_2$ | $CH(CH_3)$ | $CH_3$ | $CH_2$ | $2,4\text{-}(CH_3)_2\text{-}C_6H_3$ |
| 1.16. | $CH(CH_3)_2$ | $CH(CH_3)$ | $CH_3$ | $CH_2$ | $3,4\text{-}(CH_3)_2C_6H_3$ |
| 1.17. | $CH(CH_3)_2$ | $CH(CH_3)$ | $CH_3$ | $CH_2$ | $4\text{-}NO_2\text{-}C_6H_4$ |
| 1.18. | $CH(CH_3)C_2H_5$ | $CH(CH_3)$ | $CH_3$ | $CH_2$ | $4\text{-}Cl\text{-}C_6H_4$ |
| 1.19. | $CH(CH_3)C_2H_5$ | $CH(CH_3)$ | $CH_3$ | $CH_2$ | $4\text{-}CH_3\text{-}C_6H_4$ |
| 1.20. | $CH(CH_3)C_2H_5$ | $CH(CH_3)$ | $CH_3$ | $CH_2$ | $4\text{-}CH_3O\text{-}C_6H_4$ |
| 1.21. | $CH(CH_3)C_2H_5$ | $CH(CH_3)$ | $CH_3$ | $CH_2$ | $4\text{-}CN\text{-}C_6H_4$ |
| 1.22. | $CH(CH_3)C_2H_5$ | $CH(CH_3)$ | $CH_3$ | $CH_2$ | $3\text{-}Cl\text{-}C_6H_4$ |
| 1.23. | $CH(CH_3)C_2H_5$ | $CH(CH_3)$ | $CH_3$ | $CH_2$ | $3\text{-}CH_3\text{-}C_6H_4$ |
| 1.24. | $CH(CH_3)C_2H_5$ | $CH(CH_3)$ | $CH_3$ | $CH_2$ | $3\text{-}CH_3O\text{-}C_6H_4$ |
| 1.25. | $CH(CH_3)C_2H_5$ | $CH(CH_3)$ | $CH_3$ | $CH_2$ | $3\text{-}CN\text{-}C_6H_4$ |
| 1.26. | $CH(CH_3)C_2H_5$ | $CH(CH_3)$ | $CH_3$ | $CH_2$ | $2\text{-}Cl\text{-}C_6H_4$ |
| 1.27. | $CH(CH_3)C_2H_5$ | $CH(CH_3)$ | $CH_3$ | $CH_2$ | $2\text{-}CH_3\text{-}C_6H_4$ |

| Nr. | $R^1$ | A | Y | Z | Ar |
|---|---|---|---|---|---|
| 1.28. | $CH(CH_3)C_2H_5$ | $CH(CH_3)$ | $CH_3$ | $CH_2$ | $2\text{-}CH_3O\text{-}C_6H_4$ |
| 1.29. | $CH(CH_3)C_2H_5$ | $CH(CH_3)$ | $CH_3$ | $CH_2$ | $2\text{-}CN\text{-}C_6H_4$ |
| 1.30. | $CH(CH_3)C_2H_5$ | $CH(CH_3)$ | $CH_3$ | $CH_2$ | $3,4\text{-}(CH_3O)_2C_6H_3$ |
| 1.31. | $CH(CH_3)C_2H_5$ | $CH(CH_3)$ | $CH_3$ | $CH_2$ | $2,4\text{-}Cl_2\text{-}C_6H_3$ |
| 1.32. | $CH(CH_3)C_2H_5$ | $CH(CH_3)$ | $CH_3$ | $CH_2$ | $2,4\text{-}(CH_3)_2\text{-}C_6H_3$ |
| 1.33. | $CH(CH_3)C_2H_5$ | $CH(CH_3)$ | $CH_3$ | $CH_2$ | $3,4\text{-}(CH_3)_2C_6H_3$ |
| 1.34. | $CH(CH_3)C_2H_5$ | $CH(CH_3)$ | $CH_3$ | $CH_2$ | $4\text{-}NO_2\text{-}C_6H_4$ |
| 1.35. | $C(CH_3)_3$ | $CH(CH_3)$ | $CH_3$ | $CH_2$ | $4\text{-}Cl\text{-}C_6H_4$ |
| 1.36. | $C(CH_3)_3$ | $CH(CH_3)$ | $CH_3$ | $CH_2$ | $4\text{-}CH_3\text{-}C_6H_4$ |
| 1.37. | $C(CH_3)_3$ | $CH(CH_3)$ | $CH_3$ | $CH_2$ | $4\text{-}CH_3O\text{-}C_6H_4$ |
| 1.38. | $C(CH_3)_3$ | $CH(CH_3)$ | $CH_3$ | $CH_2$ | $4\text{-}CN\text{-}C_6H_4$ |
| 1.39. | $C(CH_3)_3$ | $CH(CH_3)$ | $CH_3$ | $CH_2$ | $3\text{-}Cl\text{-}C_6H_4$ |
| 1.40. | $C(CH_3)_3$ | $CH(CH_3)$ | $CH_3$ | $CH_2$ | $3\text{-}CH_3\text{-}C_6H_4$ |
| 1.41. | $C(CH_3)_3$ | $CH(CH_3)$ | $CH_3$ | $CH_2$ | $3\text{-}CH_3O\text{-}C_6H_4$ |
| 1.42. | $C(CH_3)_3$ | $CH(CH_3)$ | $CH_3$ | $CH_2$ | $3\text{-}CN\text{-}C_6H_4$ |
| 1.43. | $C(CH_3)_3$ | $CH(CH_3)$ | $CH_3$ | $CH_2$ | $2\text{-}Cl\text{-}C_6H_4$ |
| 1.44. | $C(CH_3)_3$ | $CH(CH_3)$ | $CH_3$ | $CH_2$ | $2\text{-}CH_3\text{-}C_6H_4$ |
| 1.45. | $C(CH_3)_3$ | $CH(CH_3)$ | $CH_3$ | $CH_2$ | $2\text{-}CH_3O\text{-}C_6H_4$ |
| 1.46. | $C(CH_3)_3$ | $CH(CH_3)$ | $CH_3$ | $CH_2$ | $2\text{-}CN\text{-}C_6H_4$ |
| 1.47. | $C(CH_3)_3$ | $CH(CH_3)$ | $CH_3$ | $CH_2$ | $3,4\text{-}(CH_3O)_2C_6H_3$ |
| 1.48. | $C(CH_3)_3$ | $CH(CH_3)$ | $CH_3$ | $CH_2$ | $2,4\text{-}Cl_2\text{-}C_6H_3$ |
| 1.49. | $C(CH_3)_3$ | $CH(CH_3)$ | $CH_3$ | $CH_2$ | $2,4\text{-}(CH_3)_2\text{-}C_6H_3$ |
| 1.50. | $C(CH_3)_3$ | $CH(CH_3)$ | $CH_3$ | $CH_2$ | $3,4\text{-}(CH_3)_2C_6H_3$ |
| 1.51. | $C(CH_3)_3$ | $CH(CH_3)$ | $CH_3$ | $CH_2$ | $4\text{-}NO_2\text{-}C_6H_4$ |
| 1.52. | $CH(CH_3)_2$ | $C(CH_3)_2\text{-}CH_2$ | $CH_3$ | $CH_2$ | $4\text{-}Cl\text{-}C_6H_4$ |
| 1.53. | $CH(CH_3)_2$ | $C(CH_3)_2\text{-}CH_2$ | $CH_3$ | $CH_2$ | $4\text{-}CH_3\text{-}C_6H_4$ |
| 1.54. | $CH(CH_3)_2$ | $C(CH_3)_2\text{-}CH_2$ | $CH_3$ | $CH_2$ | $4\text{-}CH_3O\text{-}C_6H_4$ |
| 1.55. | $CH(CH_3)_2$ | $C(CH_3)_2\text{-}CH_2$ | $CH_3$ | $CH_2$ | $4\text{-}CN\text{-}C_6H_4$ |
| 1.56. | $CH(CH_3)_2$ | $C(CH_3)_2\text{-}CH_2$ | $CH_3$ | $CH_2$ | $3\text{-}Cl\text{-}C_6H_4$ |
| 1.57. | $CH(CH_3)_2$ | $C(CH_3)_2\text{-}CH_2$ | $CH_3$ | $CH_2$ | $3\text{-}CH_3\text{-}C_6H_4$ |
| 1.58. | $CH(CH_3)_2$ | $C(CH_3)_2\text{-}CH_2$ | $CH_3$ | $CH_2$ | $3\text{-}CH_3O\text{-}C_6H_4$ |
| 1.59. | $CH(CH_3)_2$ | $C(CH_3)_2\text{-}CH_2$ | $CH_3$ | $CH_2$ | $3\text{-}CN\text{-}C_6H_4$ |
| 1.60. | $CH(CH_3)_2$ | $C(CH_3)_2\text{-}CH_2$ | $CH_3$ | $CH_2$ | $2\text{-}Cl\text{-}C_6H_4$ |
| 1.61. | $CH(CH_3)_2$ | $C(CH_3)_2\text{-}CH_2$ | $CH_3$ | $CH_2$ | $2\text{-}CH_3\text{-}C_6H_4$ |
| 1.62. | $CH(CH_3)_2$ | $C(CH_3)_2\text{-}CH_2$ | $CH_3$ | $CH_2$ | $2\text{-}CH_3O\text{-}C_6H_4$ |
| 1.63. | $CH(CH_3)_2$ | $C(CH_3)_2\text{-}CH_2$ | $CH_3$ | $CH_2$ | $2\text{-}CN\text{-}C_6H_4$ |
| 1.64. | $CH(CH_3)_2$ | $C(CH_3)_2\text{-}CH_2$ | $CH_3$ | $CH_2$ | $3,4\text{-}(CH_3O)_2C_6H_3$ |
| 1.65. | $CH(CH_3)_2$ | $C(CH_3)_2\text{-}CH_2$ | $CH_3$ | $CH_2$ | $2,4\text{-}Cl_2\text{-}C_6H_3$ |
| 1.66. | $CH(CH_3)_2$ | $C(CH_3)_2\text{-}CH_2$ | $CH_3$ | $CH_2$ | $2,4\text{-}(CH_3)_2\text{-}C_6H_3$ |

| Nr. | $R^1$ | A | Y | Z | Ar |
|---|---|---|---|---|---|
| 1.67. | $CH(CH_3)_2$ | $C(CH_3)_2-CH_2$ | $CH_3$ | $CH_2$ | $3,4-(CH_3)_2C_6H_3$ |
| 1.68. | $CH(CH_3)_2$ | $C(CH_3)_2-CH_2$ | $CH_3$ | $CH_2$ | $4-NO_2-C_6H_4$ |
| 1.69. | $CH(CH_3)C_2H_5$ | $C(CH_3)_2-CH_2$ | $CH_3$ | $CH_2$ | $4-Cl-C_6H_4$ |
| 1.70. | $CH(CH_3)C_2H_5$ | $C(CH_3)_2-CH_2$ | $CH_3$ | $CH_2$ | $4-CH_3-C_6H_4$ |
| 1.71. | $CH(CH_3)C_2H_5$ | $C(CH_3)_2-CH_2$ | $CH_3$ | $CH_2$ | $4-CH_3O-C_6H_4$ |
| 1.72. | $CH(CH_3)C_2H_5$ | $C(CH_3)_2-CH_2$ | $CH_3$ | $CH_2$ | $4-CN-C_6H_4$ |
| 1.73. | $CH(CH_3)C_2H_5$ | $C(CH_3)_2-CH_2$ | $CH_3$ | $CH_2$ | $3-Cl-C_6H_4$ |
| 1.74. | $CH(CH_3)C_2H_5$ | $C(CH_3)_2-CH_2$ | $CH_3$ | $CH_2$ | $3-CH_3-C_6H_4$ |
| 1.75. | $CH(CH_3)C_2H_5$ | $C(CH_3)_2-CH_2$ | $CH_3$ | $CH_2$ | $3-CH_3O-C_6H_4$ |
| 1.76. | $CH(CH_3)C_2H_5$ | $C(CH_3)_2-CH_2$ | $CH_3$ | $CH_2$ | $3-CN-C_6H_4$ |
| 1.77. | $CH(CH_3)C_2H_5$ | $C(CH_3)_2-CH_2$ | $CH_3$ | $CH_2$ | $2-Cl-C_6H_4$ |
| 1.78. | $CH(CH_3)C_2H_5$ | $C(CH_3)_2-CH_2$ | $CH_3$ | $CH_2$ | $2-CH_3-C_6H_4$ |
| 1.79. | $CH(CH_3)C_2H_5$ | $C(CH_3)_2-CH_2$ | $CH_3$ | $CH_2$ | $2-CH_3O-C_6H_4$ |
| 1.80. | $CH(CH_3)C_2H_5$ | $C(CH_3)_2-CH_2$ | $CH_3$ | $CH_2$ | $2-CN-C_6H_4$ |
| 1.81. | $CH(CH_3)C_2H_5$ | $C(CH_3)_2-CH_2$ | $CH_3$ | $CH_2$ | $3,4-(CH_3O)_2C_6H_3$ |
| 1.82. | $CH(CH_3)C_2H_5$ | $C(CH_3)_2-CH_2$ | $CH_3$ | $CH_2$ | $2,4-Cl_2-C_6H_3$ |
| 1.83. | $CH(CH_3)C_2H_5$ | $C(CH_3)_2-CH_2$ | $CH_3$ | $CH_2$ | $2,4-(CH_3)_2-C_6H_3$ |
| 1.84. | $CH(CH_3)C_2H_5$ | $C(CH_3)_2-CH_2$ | $CH_3$ | $CH_2$ | $3,4-(CH_3)_2C_6H_3$ |
| 1.85. | $CH(CH_3)C_2H_5$ | $C(CH_3)_2-CH_2$ | $CH_3$ | $CH_2$ | $4-NO_2-C_6H_4$ |
| 1.86. | $C(CH_3)_3$ | $C(CH_3)_2-CH_2$ | $CH_3$ | $CH_2$ | $4-Cl-C_6H_4$ |
| 1.87. | $C(CH_3)_3$ | $C(CH_3)_2-CH_2$ | $CH_3$ | $CH_2$ | $4-CH_3-C_6H_4$ |
| 1.88. | $C(CH_3)_3$ | $C(CH_3)_2-CH_2$ | $CH_3$ | $CH_2$ | $4-CH_3O-C_6H_4$ |
| 1.89. | $C(CH_3)_3$ | $C(CH_3)_2-CH_2$ | $CH_3$ | $CH_2$ | $4-CN-C_6H_4$ |
| 1.90. | $C(CH_3)_3$ | $C(CH_3)_2-CH_2$ | $CH_3$ | $CH_2$ | $3-Cl-C_6H_4$ |
| 1.91. | $C(CH_3)_3$ | $C(CH_3)_2-CH_2$ | $CH_3$ | $CH_2$ | $3-CH_3-C_6H_4$ |
| 1.92. | $C(CH_3)_3$ | $C(CH_3)_2-CH_2$ | $CH_3$ | $CH_2$ | $3-CH_3O-C_6H_4$ |
| 1.93. | $C(CH_3)_3$ | $C(CH_3)_2-CH_2$ | $CH_3$ | $CH_2$ | $3-CN-C_6H_4$ |
| 1.94. | $C(CH_3)_3$ | $C(CH_3)_2-CH_2$ | $CH_3$ | $CH_2$ | $2-Cl-C_6H_4$ |
| 1.95. | $C(CH_3)_3$ | $C(CH_3)_2-CH_2$ | $CH_3$ | $CH_2$ | $2-CH_3-C_6H_4$ |
| 1.96. | $C(CH_3)_3$ | $C(CH_3)_2-CH_2$ | $CH_3$ | $CH_2$ | $2-CH_3O-C_6H_4$ |
| 1.97. | $C(CH_3)_3$ | $C(CH_3)_2-CH_2$ | $CH_3$ | $CH_2$ | $2-CN-C_6H_4$ |
| 1.98. | $C(CH_3)_3$ | $C(CH_3)_2-CH_2$ | $CH_3$ | $CH_2$ | $3,4-(CH_3O)_2C_6H_3$ |
| 1.99. | $C(CH_3)_3$ | $C(CH_3)_2-CH_2$ | $CH_3$ | $CH_2$ | $2,4-Cl_2-C_6H_3$ |
| 1.100. | $C(CH_3)_3$ | $C(CH_3)_2-CH_2$ | $CH_3$ | $CH_2$ | $2,4-(CH_3)_2-C_6H_3$ |
| 1.101. | $C(CH_3)_3$ | $C(CH_3)_2-CH_2$ | $CH_3$ | $CH_2$ | $3,4-(CH_3)_2C_6H_3$ |
| 1.102. | $C(CH_3)_3$ | $C(CH_3)_2-CH_2$ | $CH_3$ | $CH_2$ | $4-NO_2-C_6H_4$ |
| 1.103. | $CH(CH_3)_2$ | $CH(CH_3)$ | H | $CH_2$ | $4-Cl-C_6H_4$ |
| 1.104. | $CH(CH_3)_2$ | $CH(CH_3)$ | H | $CH_2$ | $4-CH_3-C_6H_4$ |
| 1.105. | $CH(CH_3)_2$ | $CH(CH_3)$ | H | $CH_2$ | $4-CH_3O-C_6H_4$ |

| Nr. | $R^1$ | A | Y | Z | Ar |
|---|---|---|---|---|---|
| 1.106. | $CH(CH_3)_2$ | $CH(CH_3)$ | H | $CH_2$ | $4-CN-C_6H_4$ |
| 1.107. | $CH(CH_3)_2$ | $CH(CH_3)$ | H | $CH_2$ | $3-Cl-C_6H_4$ |
| 1.108. | $CH(CH_3)_2$ | $CH(CH_3)$ | H | $CH_2$ | $3-CH_3-C_6H_4$ |
| 1.109. | $CH(CH_3)_2$ | $CH(CH_3)$ | H | $CH_2$ | $3-CH_3O-C_6H_4$ |
| 1.110. | $CH(CH_3)_2$ | $CH(CH_3)$ | H | $CH_2$ | $3-CN-C_6H_4$ |
| 1.111. | $CH(CH_3)_2$ | $CH(CH_3)$ | H | $CH_2$ | $2-Cl-C_6H_4$ |
| 1.112. | $CH(CH_3)_2$ | $CH(CH_3)$ | H | $CH_2$ | $2-CH_3-C_6H_4$ |
| 1.113. | $CH(CH_3)_2$ | $CH(CH_3)$ | H | $CH_2$ | $2-CH_3O-C_6H_4$ |
| 1.114. | $CH(CH_3)_2$ | $CH(CH_3)$ | H | $CH_2$ | $2-CN-C_6H_4$ |
| 1.115. | $CH(CH_3)_2$ | $CH(CH_3)$ | H | $CH_2$ | $3,4-(CH_3O)_2C_6H_3$ |
| 1.116. | $CH(CH_3)_2$ | $CH(CH_3)$ | H | $CH_2$ | $2,4-Cl_2-C_6H_3$ |
| 1.117. | $CH(CH_3)_2$ | $CH(CH_3)$ | H | $CH_2$ | $2,4-(CH_3)_2-C_6H_3$ |
| 1.118. | $CH(CH_3)_2$ | $CH(CH_3)$ | H | $CH_2$ | $3,4-(CH_3)_2C_6H_3$ |
| 1.119. | $CH(CH_3)_2$ | $CH(CH_3)$ | H | $CH_2$ | $4-NO_2-C_6H_4$ |
| 1.120. | $CH(CH_3)C_2H_5$ | $CH(CH_3)$ | H | $CH_2$ | $4-Cl-C_6H_4$ |
| 1.121. | $CH(CH_3)C_2H_5$ | $CH(CH_3)$ | H | $CH_2$ | $4-CH_3-C_6H_4$ |
| 1.122. | $CH(CH_3)C_2H_5$ | $CH(CH_3)$ | H | $CH_2$ | $4-CH_3O-C_6H_4$ |
| 1.123. | $CH(CH_3)C_2H_5$ | $CH(CH_3)$ | H | $CH_2$ | $4-CN-C_6H_4$ |
| 1.124. | $CH(CH_3)C_2H_5$ | $CH(CH_3)$ | H | $CH_2$ | $3-Cl-C_6H_4$ |
| 1.125. | $CH(CH_3)C_2H_5$ | $CH(CH_3)$ | H | $CH_2$ | $3-CH_3-C_6H_4$ |
| 1.126. | $CH(CH_3)C_2H_5$ | $CH(CH_3)$ | H | $CH_2$ | $3-CH_3O-C_6H_4$ |
| 1.127. | $CH(CH_3)C_2H_5$ | $CH(CH_3)$ | H | $CH_2$ | $3-CN-C_6H_4$ |
| 1.128. | $CH(CH_3)C_2H_5$ | $CH(CH_3)$ | H | $CH_2$ | $2-Cl-C_6H_4$ |
| 1.129. | $CH(CH_3)C_2H_5$ | $CH(CH_3)$ | H | $CH_2$ | $2-CH_3-C_6H_4$ |
| 1.130. | $CH(CH_3)C_2H_5$ | $CH(CH_3)$ | H | $CH_2$ | $2-CH_3O-C_6H_4$ |
| 1.131. | $CH(CH_3)C_2H_5$ | $CH(CH_3)$ | H | $CH_2$ | $2-CN-C_6H_4$ |
| 1.132. | $CH(CH_3)C_2H_5$ | $CH(CH_3)$ | H | $CH_2$ | $3,4-(CH_3O)_2C_6H_3$ |
| 1.133. | $CH(CH_3)C_2H_5$ | $CH(CH_3)$ | H | $CH_2$ | $2,4-Cl_2-C_6H_3$ |
| 1.134. | $CH(CH_3)C_2H_5$ | $CH(CH_3)$ | H | $CH_2$ | $2,4-(CH_3)_2-C_6H_3$ |
| 1.135. | $CH(CH_3)C_2H_5$ | $CH(CH_3)$ | H | $CH_2$ | $3,4-(CH_3)_2C_6H_3$ |
| 1.136. | $CH(CH_3)C_2H_5$ | $CH(CH_3)$ | H | $CH_2$ | $4-NO_2-C_6H_4$ |
| 1.137. | $C(CH_3)_3$ | $CH(CH_3)$ | H | $CH_2$ | $4-Cl-C_6H_4$ |
| 1.138. | $C(CH_3)_3$ | $CH(CH_3)$ | H | $CH_2$ | $4-CH_3-C_6H_4$ |
| 1.139. | $C(CH_3)_3$ | $CH(CH_3)$ | H | $CH_2$ | $4-CH_3O-C_6H_4$ |
| 1.140. | $C(CH_3)_3$ | $CH(CH_3)$ | H | $CH_2$ | $4-CN-C_6H_4$ |
| 1.141. | $C(CH_3)_3$ | $CH(CH_3)$ | H | $CH_2$ | $3-Cl-C_6H_4$ |
| 1.142. | $C(CH_3)_3$ | $CH(CH_3)$ | H | $CH_2$ | $3-CH_3-C_6H_4$ |
| 1.143. | $C(CH_3)_3$ | $CH(CH_3)$ | H | $CH_2$ | $3-CH_3O-C_6H_4$ |
| 1.144. | $C(CH_3)_3$ | $CH(CH_3)$ | H | $CH_2$ | $3-CN-C_6H_4$ |

| Nr. | $R^1$ | A | Y | Z | Ar |
|---|---|---|---|---|---|
| 1.145. | $C(CH_3)_3$ | $CH(CH_3)$ | H | $CH_2$ | $2-Cl-C_6H_4$ |
| 1.146. | $C(CH_3)_3$ | $CH(CH_3)$ | H | $CH_2$ | $2-CH_3-C_6H_4$ |
| 1.147. | $C(CH_3)_3$ | $CH(CH_3)$ | H | $CH_2$ | $2-CH_3O-C_6H_4$ |
| 1.148. | $C(CH_3)_3$ | $CH(CH_3)$ | H | $CH_2$ | $2-CN-C_6H_4$ |
| 1.149. | $C(CH_3)_3$ | $CH(CH_3)$ | H | $CH_2$ | $3,4-(CH_3O)_2C_6H_3$ |
| 1.150. | $C(CH_3)_3$ | $CH(CH_3)$ | H | $CH_2$ | $2,4-Cl_2-C_6H_3$ |
| 1.151. | $C(CH_3)_3$ | $CH(CH_3)$ | H | $CH_2$ | $2,4-(CH_3)_2-C_6H_3$ |
| 1.152. | $C(CH_3)_3$ | $CH(CH_3)$ | H | $CH_2$ | $3,4-(CH_3)_2C_6H_3$ |
| 1.153. | $C(CH_3)_3$ | $CH(CH_3)$ | H | $CH_2$ | $4-NO_2-C_6H_4$ |
| 1.154. | $CH(CH_3)_2$ | $C(CH_3)_2-CH_2$ | H | $CH_2$ | $4-Cl-C_6H_4$ |
| 1.155. | $CH(CH_3)_2$ | $C(CH_3)_2-CH_2$ | H | $CH_2$ | $4-CH_3-C_6H_4$ |
| 1.156. | $CH(CH_3)_2$ | $C(CH_3)_2-CH_2$ | H | $CH_2$ | $4-CH_3O-C_6H_4$ |
| 1.157. | $CH(CH_3)_2$ | $C(CH_3)_2-CH_2$ | H | $CH_2$ | $4-CN-C_6H_4$ |
| 1.158. | $CH(CH_3)_2$ | $C(CH_3)_2-CH_2$ | H | $CH_2$ | $3-Cl-C_6H_4$ |
| 1.159. | $CH(CH_3)_2$ | $C(CH_3)_2-CH_2$ | H | $CH_2$ | $3-CH_3-C_6H_4$ |
| 1.160. | $CH(CH_3)_2$ | $C(CH_3)_2-CH_2$ | H | $CH_2$ | $3-CH_3O-C_6H_4$ |
| 1.161. | $CH(CH_3)_2$ | $C(CH_3)_2-CH_2$ | H | $CH_2$ | $3-CN-C_6H_4$ |
| 1.162. | $CH(CH_3)_2$ | $C(CH_3)_2-CH_2$ | H | $CH_2$ | $2-Cl-C_6H_4$ |
| 1.163. | $CH(CH_3)_2$ | $C(CH_3)_2-CH_2$ | H | $CH_2$ | $2-CH_3-C_6H_4$ |
| 1.164. | $CH(CH_3)_2$ | $C(CH_3)_2-CH_2$ | H | $CH_2$ | $2-CH_3O-C_6H_4$ |
| 1.165. | $CH(CH_3)_2$ | $C(CH_3)_2-CH_2$ | H | $CH_2$ | $2-CN-C_6H_4$ |
| 1.166. | $CH(CH_3)_2$ | $C(CH_3)_2-CH_2$ | H | $CH_2$ | $3,4-(CH_3O)_2C_6H_3$ |
| 1.167. | $CH(CH_3)_2$ | $C(CH_3)_2-CH_2$ | H | $CH_2$ | $2,4-Cl_2-C_6H_3$ |
| 1.168. | $CH(CH_3)_2$ | $C(CH_3)_2-CH_2$ | H | $CH_2$ | $2,4-(CH_3)_2-C_6H_3$ |
| 1.169. | $CH(CH_3)_2$ | $C(CH_3)_2-CH_2$ | H | $CH_2$ | $3,4-(CH_3)_2C_6H_3$ |
| 1.170. | $CH(CH_3)_2$ | $C(CH_3)_2-CH_2$ | H | $CH_2$ | $4-NO_2-C_6H_4$ |
| 1.171. | $CH(CH_3)C_2H_5$ | $C(CH_3)_2-CH_2$ | H | $CH_2$ | $4-Cl-C_6H_4$ |
| 1.172. | $CH(CH_3)C_2H_5$ | $C(CH_3)_2-CH_2$ | H | $CH_2$ | $4-CH_3-C_6H_4$ |
| 1.173. | $CH(CH_3)C_2H_5$ | $C(CH_3)_2-CH_2$ | H | $CH_2$ | $4-CH_3O-C_6H_4$ |
| 1.174. | $CH(CH_3)C_2H_5$ | $C(CH_3)_2-CH_2$ | H | $CH_2$ | $4-CN-C_6H_4$ |
| 1.175. | $CH(CH_3)C_2H_5$ | $C(CH_3)_2-CH_2$ | H | $CH_2$ | $3-Cl-C_6H_4$ |
| 1.176. | $CH(CH_3)C_2H_5$ | $C(CH_3)_2-CH_2$ | H | $CH_2$ | $3-CH_3-C_6H_4$ |
| 1.177. | $CH(CH_3)C_2H_5$ | $C(CH_3)_2-CH_2$ | H | $CH_2$ | $3-CH_3O-C_6H_4$ |
| 1.178. | $CH(CH_3)C_2H_5$ | $C(CH_3)_2-CH_2$ | H | $CH_2$ | $3-CN-C_6H_4$ |
| 1.179. | $CH(CH_3)C_2H_5$ | $C(CH_3)_2-CH_2$ | H | $CH_2$ | $2-Cl-C_6H_4$ |
| 1.180. | $CH(CH_3)C_2H_5$ | $C(CH_3)_2-CH_2$ | H | $CH_2$ | $2-CH_3-C_6H_4$ |
| 1.181. | $CH(CH_3)C_2H_5$ | $C(CH_3)_2-CH_2$ | H | $CH_2$ | $2-CH_3O-C_6H_4$ |
| 1.182. | $CH(CH_3)C_2H_5$ | $C(CH_3)_2-CH_2$ | H | $CH_2$ | $2-CN-C_6H_4$ |
| 1.183. | $CH(CH_3)C_2H_5$ | $C(CH_3)_2-CH_2$ | H | $CH_2$ | $3,4-(CH_3O)_2C_6H_3$ |

| Nr. | $R^1$ | A | Y | Z | Ar |
|---|---|---|---|---|---|
| 1.184. | $CH(CH_3)C_2H_5$ | $C(CH_3)_2-CH_2$ | H | $CH_2$ | $2,4-Cl_2-C_6H_3$ |
| 1.185. | $CH(CH_3)C_2H_5$ | $C(CH_3)_2-CH_2$ | H | $CH_2$ | $2,4-(CH_3)_2-C_6H_3$ |
| 1.186. | $CH(CH_3)C_2H_5$ | $C(CH_3)_2-CH_2$ | H | $CH_2$ | $3,4-(CH_3)_2C_6H_3$ |
| 1.187. | $CH(CH_3)C_2H_5$ | $C(CH_3)_2-CH_2$ | H | $CH_2$ | $4-NO_2-C_6H_4$ |
| 1.188. | $C(CH_3)_3$ | $C(CH_3)_2-CH_2$ | H | $CH_2$ | $4-Cl-C_6H_4$ |
| 1.189. | $C(CH_3)_3$ | $C(CH_3)_2-CH_2$ | H | $CH_2$ | $4-CH_3-C_6H_4$ |
| 1.190. | $C(CH_3)_3$ | $C(CH_3)_2-CH_2$ | H | $CH_2$ | $4-CH_3O-C_6H_4$ |
| 1.191. | $C(CH_3)_3$ | $C(CH_3)_2-CH_2$ | H | $CH_2$ | $4-CN-C_6H_4$ |
| 1.192. | $C(CH_3)_3$ | $C(CH_3)_2-CH_2$ | H | $CH_2$ | $3-Cl-C_6H_4$ |
| 1.193. | $C(CH_3)_3$ | $C(CH_3)_2-CH_2$ | H | $CH_2$ | $3-CH_3-C_6H_4$ |
| 1.194. | $C(CH_3)_3$ | $C(CH_3)_2-CH_2$ | H | $CH_2$ | $3-CH_3O-C_6H_4$ |
| 1.195. | $C(CH_3)_3$ | $C(CH_3)_2-CH_2$ | H | $CH_2$ | $3-CN-C_6H_4$ |
| 1.196. | $C(CH_3)_3$ | $C(CH_3)_2-CH_2$ | H | $CH_2$ | $2-Cl-C_6H_4$ |
| 1.197. | $C(CH_3)_3$ | $C(CH_3)_2-CH_2$ | H | $CH_2$ | $2-CH_3-C_6H_4$ |
| 1.198. | $C(CH_3)_3$ | $C(CH_3)_2-CH_2$ | H | $CH_2$ | $2-CH_3O-C_6H_4$ |
| 1.199. | $C(CH_3)_3$ | $C(CH_3)_2-CH_2$ | H | $CH_2$ | $2-CN-C_6H_4$ |
| 1.200. | $C(CH_3)_3$ | $C(CH_3)_2-CH_2$ | H | $CH_2$ | $3,4-(CH_3O)_2C_6H_3$ |
| 1.201. | $C(CH_3)_3$ | $C(CH_3)_2-CH_2$ | H | $CH_2$ | $2,4-Cl_2-C_6H_3$ |
| 1.202. | $C(CH_3)_3$ | $C(CH_3)_2-CH_2$ | H | $CH_2$ | $2,4-(CH_3)_2-C_6H_3$ |
| 1.203. | $C(CH_3)_3$ | $C(CH_3)_2-CH_2$ | H | $CH_2$ | $3,4-(CH_3)_2C_6H_3$ |
| 1.204. | $C(CH_3)_3$ | $C(CH_3)_2-CH_2$ | H | $CH_2$ | $4-NO_2-C_6H_4$ |
| 1.205. | $CH(CH_3)_2$ | $CH_2$ | $CH_3$ | $CH_2$ | $4-Cl-C_6H_4$ |
| 1.206. | $CH(CH_3)_2$ | $CH_2$ | $CH_3$ | $CH_2$ | $4-CH_3-C_6H_4$ |
| 1.207. | $CH(CH_3)_2$ | $CH_2$ | $CH_3$ | $CH_2$ | $4-CH_3O-C_6H_4$ |
| 1.208. | $CH(CH_3)_2$ | $CH_2$ | $CH_3$ | $CH_2$ | $4-CN-C_6H_4$ |
| 1.209. | $CH(CH_3)_2$ | $CH_2$ | $CH_3$ | $CH_2$ | $3-Cl-C_6H_4$ |
| 1.210. | $CH(CH_3)_2$ | $CH_2$ | $CH_3$ | $CH_2$ | $3-CH_3-C_6H_4$ |
| 1.211. | $CH(CH_3)_2$ | $CH_2$ | $CH_3$ | $CH_2$ | $3-CH_3O-C_6H_4$ |
| 1.212. | $CH(CH_3)_2$ | $CH_2$ | $CH_3$ | $CH_2$ | $3-CN-C_6H_4$ |
| 1.213. | $CH(CH_3)_2$ | $CH_2$ | $CH_3$ | $CH_2$ | $2-Cl-C_6H_4$ |
| 1.214. | $CH(CH_3)_2$ | $CH_2$ | $CH_3$ | $CH_2$ | $2-CH_3-C_6H_4$ |
| 1.215. | $CH(CH_3)_2$ | $CH_2$ | $CH_3$ | $CH_2$ | $2-CH_3O-C_6H_4$ |
| 1.216. | $CH(CH_3)_2$ | $CH_2$ | $CH_3$ | $CH_2$ | $2-CN-C_6H_4$ |
| 1.217. | $CH(CH_3)_2$ | $CH_2$ | $CH_3$ | $CH_2$ | $3,4-(CH_3O)_2C_6H_3$ |
| 1.218. | $CH(CH_3)_2$ | $CH_2$ | $CH_3$ | $CH_2$ | $2,4-Cl_2-C_6H_3$ |
| 1.219. | $CH(CH_3)_2$ | $CH_2$ | $CH_3$ | $CH_2$ | $2,4-(CH_3)_2-C_6H_3$ |
| 1.220. | $CH(CH_3)_2$ | $CH_2$ | $CH_3$ | $CH_2$ | $3,4-(CH_3)_2C_6H_3$ |
| 1.221. | $CH(CH_3)_2$ | $CH_2$ | $CH_3$ | $CH_2$ | $4-NO_2-C_6H_4$ |
| 1.222. | $CH(CH_3)C_2H_5$ | $CH_2$ | $CH_3$ | $CH_2$ | $4-Cl-C_6H_4$ |

| Nr. | $R^1$ | A | Y | Z | Ar |
|---|---|---|---|---|---|
| 1.223. | $CH(CH_3)C_2H_5$ | $CH_2$ | $CH_3$ | $CH_2$ | $4-CH_3-C_6H_4$ |
| 1.224. | $CH(CH_3)C_2H_5$ | $CH_2$ | $CH_3$ | $CH_2$ | $4-CH_3O-C_6H_4$ |
| 1.225. | $CH(CH_3)C_2H_5$ | $CH_2$ | $CH_3$ | $CH_2$ | $4-CN-C_6H_4$ |
| 1.226. | $CH(CH_3)C_2H_5$ | $CH_2$ | $CH_3$ | $CH_2$ | $3-Cl-C_6H_4$ |
| 1.227. | $CH(CH_3)C_2H_5$ | $CH_2$ | $CH_3$ | $CH_2$ | $3-CH_3-C_6H_4$ |
| 1.228. | $CH(CH_3)C_2H_5$ | $CH_2$ | $CH_3$ | $CH_2$ | $3-CH_3O-C_6H_4$ |
| 1.229. | $CH(CH_3)C_2H_5$ | $CH_2$ | $CH_3$ | $CH_2$ | $3-CN-C_6H_4$ |
| 1.230. | $CH(CH_3)C_2H_5$ | $CH_2$ | $CH_3$ | $CH_2$ | $2-Cl-C_6H_4$ |
| 1.231. | $CH(CH_3)C_2H_5$ | $CH_2$ | $CH_3$ | $CH_2$ | $2-CH_3-C_6H_4$ |
| 1.232. | $CH(CH_3)C_2H_5$ | $CH_2$ | $CH_3$ | $CH_2$ | $2-CH_3O-C_6H_4$ |
| 1.233. | $CH(CH_3)C_2H_5$ | $CH_2$ | $CH_3$ | $CH_2$ | $2-CN-C_6H_4$ |
| 1.234. | $CH(CH_3)C_2H_5$ | $CH_2$ | $CH_3$ | $CH_2$ | $3,4-(CH_3O)_2C_6H_3$ |
| 1.235. | $CH(CH_3)C_2H_5$ | $CH_2$ | $CH_3$ | $CH_2$ | $2,4-Cl_2-C_6H_3$ |
| 1.236. | $CH(CH_3)C_2H_5$ | $CH_2$ | $CH_3$ | $CH_2$ | $2,4-(CH_3)_2-C_6H_3$ |
| 1.237. | $CH(CH_3)C_2H_5$ | $CH_2$ | $CH_3$ | $CH_2$ | $3,4-(CH_3)_2C_6H_3$ |
| 1.238. | $CH(CH_3)C_2H_5$ | $CH_2$ | $CH_3$ | $CH_2$ | $4-NO_2-C_6H_4$ |
| 1.239. | $C(CH_3)_3$ | $CH_2$ | $CH_3$ | $CH_2$ | $4-Cl-C_6H_4$ |
| 1.240. | $C(CH_3)_3$ | $CH_2$ | $CH_3$ | $CH_2$ | $4-CH_3-C_6H_4$ |
| 1.241. | $C(CH_3)_3$ | $CH_2$ | $CH_3$ | $CH_2$ | $4-CH_3O-C_6H_4$ |
| 1.242. | $C(CH_3)_3$ | $CH_2$ | $CH_3$ | $CH_2$ | $4-CN-C_6H_4$ |
| 1.243. | $C(CH_3)_3$ | $CH_2$ | $CH_3$ | $CH_2$ | $3-Cl-C_6H_4$ |
| 1.244. | $C(CH_3)_3$ | $CH_2$ | $CH_3$ | $CH_2$ | $3-CH_3-C_6H_4$ |
| 1.245. | $C(CH_3)_3$ | $CH_2$ | $CH_3$ | $CH_2$ | $3-CH_3O-C_6H_4$ |
| 1.246. | $C(CH_3)_3$ | $CH_2$ | $CH_3$ | $CH_2$ | $3-CN-C_6H_4$ |
| 1.247. | $C(CH_3)_3$ | $CH_2$ | $CH_3$ | $CH_2$ | $2-Cl-C_6H_4$ |
| 1.248. | $C(CH_3)_3$ | $CH_2$ | $CH_3$ | $CH_2$ | $2-CH_3-C_6H_4$ |
| 1.249. | $C(CH_3)_3$ | $CH_2$ | $CH_3$ | $CH_2$ | $2-CH_3O-C_6H_4$ |
| 1.250. | $C(CH_3)_3$ | $CH_2$ | $CH_3$ | $CH_2$ | $2-CN-C_6H_4$ |
| 1.251. | $C(CH_3)_3$ | $CH_2$ | $CH_3$ | $CH_2$ | $3,4-(CH_3O)_2C_6H_3$ |
| 1.252. | $C(CH_3)_3$ | $CH_2$ | $CH_3$ | $CH_2$ | $2,4-Cl_2-C_6H_3$ |
| 1.253. | $C(CH_3)_3$ | $CH_2$ | $CH_3$ | $CH_2$ | $2,4-(CH_3)_2-C_6H_3$ |
| 1.254. | $C(CH_3)_3$ | $CH_2$ | $CH_3$ | $CH_2$ | $3,4-(CH_3)_2C_6H_3$ |
| 1.255. | $C(CH_3)_3$ | $CH_2$ | $CH_3$ | $CH_2$ | $4-NO_2-C_6H_4$ |
| 1.256. | $CH(CH_3)_2$ | $CH_2$ | H | $CH_2$ | $4-Cl-C_6H_4$ |
| 1.257. | $CH(CH_3)_2$ | $CH_2$ | H | $CH_2$ | $4-CH_3-C_6H_4$ |
| 1.258. | $CH(CH_3)_2$ | $CH_2$ | H | $CH_2$ | $4-CH_3O-C_6H_4$ |
| 1.259. | $CH(CH_3)_2$ | $CH_2$ | H | $CH_2$ | $4-CN-C_6H_4$ |
| 1.260. | $CH(CH_3)_2$ | $CH_2$ | H | $CH_2$ | $3-Cl-C_6H_4$ |
| 1.261. | $CH(CH_3)_2$ | $CH_2$ | H | $CH_2$ | $3-CH_3-C_6H_4$ |

| Nr. | $R^1$ | A | Y | Z | Ar |
|---|---|---|---|---|---|
| 1.262. | $CH(CH_3)_2$ | $CH_2$ | H | $CH_2$ | $3-CH_3O-C_6H_4$ |
| 1.263. | $CH(CH_3)_2$ | $CH_2$ | H | $CH_2$ | $3-CN-C_6H_4$ |
| 1.264. | $CH(CH_3)_2$ | $CH_2$ | H | $CH_2$ | $2-Cl-C_6H_4$ |
| 1.265. | $CH(CH_3)_2$ | $CH_2$ | H | $CH_2$ | $2-CH_3-C_6H_4$ |
| 1.266. | $CH(CH_3)_2$ | $CH_2$ | H | $CH_2$ | $2-CH_3O-C_6H_4$ |
| 1.267. | $CH(CH_3)_2$ | $CH_2$ | H | $CH_2$ | $2-CN-C_6H_4$ |
| 1.268. | $CH(CH_3)_2$ | $CH_2$ | H | $CH_2$ | $3,4-(CH_3O)_2C_6H_3$ |
| 1.269. | $CH(CH_3)_2$ | $CH_2$ | H | $CH_2$ | $2,4-Cl_2-C_6H_3$ |
| 1.270. | $CH(CH_3)_2$ | $CH_2$ | H | $CH_2$ | $2,4-(CH_3)_2-C_6H_3$ |
| 1.271. | $CH(CH_3)_2$ | $CH_2$ | H | $CH_2$ | $3,4-(CH_3)_2C_6H_3$ |
| 1.272. | $CH(CH_3)_2$ | $CH_2$ | H | $CH_2$ | $4-NO_2-C_6H_4$ |
| 1.273. | $CH(CH_3)C_2H_5$ | $CH_2$ | H | $CH_2$ | $4-Cl-C_6H_4$ |
| 1.274. | $CH(CH_3)C_2H_5$ | $CH_2$ | H | $CH_2$ | $4-CH_3-C_6H_4$ |
| 1.275. | $CH(CH_3)C_2H_5$ | $CH_2$ | H | $CH_2$ | $4-CH_3O-C_6H_4$ |
| 1.276. | $CH(CH_3)C_2H_5$ | $CH_2$ | H | $CH_2$ | $4-CN-C_6H_4$ |
| 1.277. | $CH(CH_3)C_2H_5$ | $CH_2$ | H | $CH_2$ | $3-Cl-C_6H_4$ |
| 1.278. | $CH(CH_3)C_2H_5$ | $CH_2$ | H | $CH_2$ | $3-CH_3-C_6H_4$ |
| 1.279. | $CH(CH_3)C_2H_5$ | $CH_2$ | H | $CH_2$ | $3-CH_3O-C_6H_4$ |
| 1.280. | $CH(CH_3)C_2H_5$ | $CH_2$ | H | $CH_2$ | $3-CN-C_6H_4$ |
| 1.281. | $CH(CH_3)C_2H_5$ | $CH_2$ | H | $CH_2$ | $2-Cl-C_6H_4$ |
| 1.282. | $CH(CH_3)C_2H_5$ | $CH_2$ | H | $CH_2$ | $2-CH_3-C_6H_4$ |
| 1.283. | $CH(CH_3)C_2H_5$ | $CH_2$ | H | $CH_2$ | $2-CH_3O-C_6H_4$ |
| 1.284. | $CH(CH_3)C_2H_5$ | $CH_2$ | H | $CH_2$ | $2-CN-C_6H_4$ |
| 1.285. | $CH(CH_3)C_2H_5$ | $CH_2$ | H | $CH_2$ | $3,4-(CH_3O)_2C_6H_3$ |
| 1.286. | $CH(CH_3)C_2H_5$ | $CH_2$ | H | $CH_2$ | $2,4-Cl_2-C_6H_3$ |
| 1.287. | $CH(CH_3)C_2H_5$ | $CH_2$ | H | $CH_2$ | $2,4-(CH_3)_2-C_6H_3$ |
| 1.288. | $CH(CH_3)C_2H_5$ | $CH_2$ | H | $CH_2$ | $3,4-(CH_3)_2C_6H_3$ |
| 1.289. | $CH(CH_3)C_2H_5$ | $CH_2$ | H | $CH_2$ | $4-NO_2-C_6H_4$ |
| 1.290. | $C(CH_3)_3$ | $CH_2$ | H | $CH_2$ | $4-Cl-C_6H_4$ |
| 1.291. | $C(CH_3)_3$ | $CH_2$ | H | $CH_2$ | $4-CH_3-C_6H_4$ |
| 1.292. | $C(CH_3)_3$ | $CH_2$ | H | $CH_2$ | $4-CH_3O-C_6H_4$ |
| 1.293. | $C(CH_3)_3$ | $CH_2$ | H | $CH_2$ | $4-CN-C_6H_4$ |
| 1.294. | $C(CH_3)_3$ | $CH_2$ | H | $CH_2$ | $3-Cl-C_6H_4$ |
| 1.295. | $C(CH_3)_3$ | $CH_2$ | H | $CH_2$ | $3-CH_3-C_6H_4$ |
| 1.296. | $C(CH_3)_3$ | $CH_2$ | H | $CH_2$ | $3-CH_3O-C_6H_4$ |
| 1.297. | $C(CH_3)_3$ | $CH_2$ | H | $CH_2$ | $3-CN-C_6H_4$ |
| 1.298. | $C(CH_3)_3$ | $CH_2$ | H | $CH_2$ | $2-Cl-C_6H_4$ |
| 1.299. | $C(CH_3)_3$ | $CH_2$ | H | $CH_2$ | $2-CH_3-C_6H_4$ |
| 1.300. | $C(CH_3)_3$ | $CH_2$ | H | $CH_2$ | $2-CH_3O-C_6H_4$ |

| Nr. | $R^1$ | A | Y | Z | Ar |
|---|---|---|---|---|---|
| 1.301. | $C(CH_3)_3$ | $CH_2$ | H | $CH_2$ | $2\text{-}CN\text{-}C_6H_4$ |
| 1.302. | $C(CH_3)_3$ | $CH_2$ | H | $CH_2$ | $3,4\text{-}(CH_3O)_2C_6H_3$ |
| 1.303. | $C(CH_3)_3$ | $CH_2$ | H | $CH_2$ | $2,4\text{-}Cl_2\text{-}C_6H_3$ |
| 1.304. | $C(CH_3)_3$ | $CH_2$ | H | $CH_2$ | $2,4\text{-}(CH_3)_2\text{-}C_6H_3$ |
| 1.305. | $C(CH_3)_3$ | $CH_2$ | H | $CH_2$ | $3,4\text{-}(CH_3)_2C_6H_3$ |
| 1.306. | $C(CH_3)_3$ | $CH_2$ | H | $CH_2$ | $4\text{-}NO_2\text{-}C_6H_4$ |
| 1.307. | $CH(CH_3)_2$ | $CH(CH_3)$ | $CH_3$ | $CH_2CH_2$ | $4\text{-}Cl\text{-}C_6H_4$ |
| 1.308. | $CH(CH_3)_2$ | $CH(CH_3)$ | $CH_3$ | $CH_2CH_2$ | $4\text{-}CH_3\text{-}C_6H_4$ |
| 1.309. | $CH(CH_3)_2$ | $CH(CH_3)$ | $CH_3$ | $CH_2CH_2$ | $4\text{-}CH_3O\text{-}C_6H_4$ |
| 1.310. | $CH(CH_3)_2$ | $CH(CH_3)$ | $CH_3$ | $CH_2CH_2$ | $4\text{-}CN\text{-}C_6H_4$ |
| 1.311. | $CH(CH_3)_2$ | $CH(CH_3)$ | $CH_3$ | $CH_2CH_2$ | $3\text{-}Cl\text{-}C_6H_4$ |
| 1.312. | $CH(CH_3)_2$ | $CH(CH_3)$ | $CH_3$ | $CH_2CH_2$ | $3\text{-}CH_3\text{-}C_6H_4$ |
| 1.313. | $CH(CH_3)_2$ | $CH(CH_3)$ | $CH_3$ | $CH_2CH_2$ | $3\text{-}CH_3O\text{-}C_6H_4$ |
| 1.314. | $CH(CH_3)_2$ | $CH(CH_3)$ | $CH_3$ | $CH_2CH_2$ | $3\text{-}CN\text{-}C_6H_4$ |
| 1.315. | $CH(CH_3)_2$ | $CH(CH_3)$ | $CH_3$ | $CH_2CH_2$ | $2\text{-}Cl\text{-}C_6H_4$ |
| 1.316. | $CH(CH_3)_2$ | $CH(CH_3)$ | $CH_3$ | $CH_2CH_2$ | $2\text{-}CH_3\text{-}C_6H_4$ |
| 1.317. | $CH(CH_3)_2$ | $CH(CH_3)$ | $CH_3$ | $CH_2CH_2$ | $2\text{-}CH_3O\text{-}C_6H_4$ |
| 1.318. | $CH(CH_3)_2$ | $CH(CH_3)$ | $CH_3$ | $CH_2CH_2$ | $2\text{-}CN\text{-}C_6H_4$ |
| 1.319. | $CH(CH_3)_2$ | $CH(CH_3)$ | $CH_3$ | $CH_2CH_2$ | $3,4\text{-}(CH_3O)_2C_6H_3$ |
| 1.320. | $CH(CH_3)_2$ | $CH(CH_3)$ | $CH_3$ | $CH_2CH_2$ | $2,4\text{-}Cl_2\text{-}C_6H_3$ |
| 1.321. | $CH(CH_3)_2$ | $CH(CH_3)$ | $CH_3$ | $CH_2CH_2$ | $2,4\text{-}(CH_3)_2\text{-}C_6H_3$ |
| 1.322. | $CH(CH_3)_2$ | $CH(CH_3)$ | $CH_3$ | $CH_2CH_2$ | $3,4\text{-}(CH_3)_2C_6H_3$ |
| 1.323. | $CH(CH_3)_2$ | $CH(CH_3)$ | $CH_3$ | $CH_2CH_2$ | $4\text{-}NO_2\text{-}C_6H_4$ |
| 1.324. | $CH(CH_3)C_2H_5$ | $CH(CH_3)$ | $CH_3$ | $CH_2CH_2$ | $4\text{-}Cl\text{-}C_6H_4$ |
| 1.325. | $CH(CH_3)C_2H_5$ | $CH(CH_3)$ | $CH_3$ | $CH_2CH_2$ | $4\text{-}CH_3\text{-}C_6H_4$ |
| 1.326. | $CH(CH_3)C_2H_5$ | $CH(CH_3)$ | $CH_3$ | $CH_2CH_2$ | $4\text{-}CH_3O\text{-}C_6H_4$ |
| 1.327. | $CH(CH_3)C_2H_5$ | $CH(CH_3)$ | $CH_3$ | $CH_2CH_2$ | $4\text{-}CN\text{-}C_6H_4$ |
| 1.328. | $CH(CH_3)C_2H_5$ | $CH(CH_3)$ | $CH_3$ | $CH_2CH_2$ | $3\text{-}Cl\text{-}C_6H_4$ |
| 1.329. | $CH(CH_3)C_2H_5$ | $CH(CH_3)$ | $CH_3$ | $CH_2CH_2$ | $3\text{-}CH_3\text{-}C_6H_4$ |
| 1.330. | $CH(CH_3)C_2H_5$ | $CH(CH_3)$ | $CH_3$ | $CH_2CH_2$ | $3\text{-}CH_3O\text{-}C_6H_4$ |
| 1.331. | $CH(CH_3)C_2H_5$ | $CH(CH_3)$ | $CH_3$ | $CH_2CH_2$ | $3\text{-}CN\text{-}C_6H_4$ |
| 1.332. | $CH(CH_3)C_2H_5$ | $CH(CH_3)$ | $CH_3$ | $CH_2CH_2$ | $2\text{-}Cl\text{-}C_6H_4$ |
| 1.333. | $CH(CH_3)C_2H_5$ | $CH(CH_3)$ | $CH_3$ | $CH_2CH_2$ | $2\text{-}CH_3\text{-}C_6H_4$ |
| 1.334. | $CH(CH_3)C_2H_5$ | $CH(CH_3)$ | $CH_3$ | $CH_2CH_2$ | $2\text{-}CH_3O\text{-}C_6H_4$ |
| 1.335. | $CH(CH_3)C_2H_5$ | $CH(CH_3)$ | $CH_3$ | $CH_2CH_2$ | $2\text{-}CN\text{-}C_6H_4$ |
| 1.336. | $CH(CH_3)C_2H_5$ | $CH(CH_3)$ | $CH_3$ | $CH_2CH_2$ | $3,4\text{-}(CH_3O)_2C_6H_3$ |
| 1.337. | $CH(CH_3)C_2H_5$ | $CH(CH_3)$ | $CH_3$ | $CH_2CH_2$ | $2,4\text{-}Cl_2\text{-}C_6H_3$ |
| 1.338. | $CH(CH_3)C_2H_5$ | $CH(CH_3)$ | $CH_3$ | $CH_2CH_2$ | $2,4\text{-}(CH_3)_2\text{-}C_6H_3$ |
| 1.339. | $CH(CH_3)C_2H_5$ | $CH(CH_3)$ | $CH_3$ | $CH_2CH_2$ | $3,4\text{-}(CH_3)_2C_6H_3$ |

| Nr. | $R^1$ | A | Y | Z | Ar |
|---|---|---|---|---|---|
| 1.340. | $CH(CH_3)C_2H_5$ | $CH(CH_3)$ | $CH_3$ | $CH_2CH_2$ | $4-NO_2-C_6H_4$ |
| 1.341. | $C(CH_3)_3$ | $CH(CH_3)$ | $CH_3$ | $CH_2CH_2$ | $4-Cl-C_6H_4$ |
| 1.342. | $C(CH_3)_3$ | $CH(CH_3)$ | $CH_3$ | $CH_2CH_2$ | $4-CH_3-C_6H_4$ |
| 1.343. | $C(CH_3)_3$ | $CH(CH_3)$ | $CH_3$ | $CH_2CH_2$ | $4-CH_3O-C_6H_4$ |
| 1.344. | $C(CH_3)_3$ | $CH(CH_3)$ | $CH_3$ | $CH_2CH_2$ | $4-CN-C_6H_4$ |
| 1.345. | $C(CH_3)_3$ | $CH(CH_3)$ | $CH_3$ | $CH_2CH_2$ | $3-Cl-C_6H_4$ |
| 1.346. | $C(CH_3)_3$ | $CH(CH_3)$ | $CH_3$ | $CH_2CH_2$ | $3-CH_3-C_6H_4$ |
| 1.347. | $C(CH_3)_3$ | $CH(CH_3)$ | $CH_3$ | $CH_2CH_2$ | $3-CH_3O-C_6H_4$ |
| 1.348. | $C(CH_3)_3$ | $CH(CH_3)$ | $CH_3$ | $CH_2CH_2$ | $3-CN-C_6H_4$ |
| 1.349. | $C(CH_3)_3$ | $CH(CH_3)$ | $CH_3$ | $CH_2CH_2$ | $2-Cl-C_6H_4$ |
| 1.350. | $C(CH_3)_3$ | $CH(CH_3)$ | $CH_3$ | $CH_2CH_2$ | $2-CH_3-C_6H_4$ |
| 1.351. | $C(CH_3)_3$ | $CH(CH_3)$ | $CH_3$ | $CH_2CH_2$ | $2-CH_3O-C_6H_4$ |
| 1.352. | $C(CH_3)_3$ | $CH(CH_3)$ | $CH_3$ | $CH_2CH_2$ | $2-CN-C_6H_4$ |
| 1.353. | $C(CH_3)_3$ | $CH(CH_3)$ | $CH_3$ | $CH_2CH_2$ | $3,4-(CH_3O)_2C_6H_3$ |
| 1.354. | $C(CH_3)_3$ | $CH(CH_3)$ | $CH_3$ | $CH_2CH_2$ | $2,4-Cl_2-C_6H_3$ |
| 1.355. | $C(CH_3)_3$ | $CH(CH_3)$ | $CH_3$ | $CH_2CH_2$ | $2,4-(CH_3)_2-C_6H_3$ |
| 1.356. | $C(CH_3)_3$ | $CH(CH_3)$ | $CH_3$ | $CH_2CH_2$ | $3,4-(CH_3)_2C_6H_3$ |
| 1.357. | $C(CH_3)_3$ | $CH(CH_3)$ | $CH_3$ | $CH_2CH_2$ | $4-NO_2-C_6H_4$ |
| 1.358. | $CH(CH_3)_2$ | $C(CH_3)_2-CH_2$ | $CH_3$ | $CH_2CH_2$ | $4-Cl-C_6H_4$ |
| 1.359. | $CH(CH_3)_2$ | $C(CH_3)_2-CH_2$ | $CH_3$ | $CH_2CH_2$ | $4-CH_3-C_6H_4$ |
| 1.360. | $CH(CH_3)_2$ | $C(CH_3)_2-CH_2$ | $CH_3$ | $CH_2CH_2$ | $4-CH_3O-C_6H_4$ |
| 1.361. | $CH(CH_3)_2$ | $C(CH_3)_2-CH_2$ | $CH_3$ | $CH_2CH_2$ | $4-CN-C_6H_4$ |
| 1.362. | $CH(CH_3)_2$ | $C(CH_3)_2-CH_2$ | $CH_3$ | $CH_2CH_2$ | $3-Cl-C_6H_4$ |
| 1.363. | $CH(CH_3)_2$ | $C(CH_3)_2-CH_2$ | $CH_3$ | $CH_2CH_2$ | $3-CH_3-C_6H_4$ |
| 1.364. | $CH(CH_3)_2$ | $C(CH_3)_2-CH_2$ | $CH_3$ | $CH_2CH_2$ | $3-CH_3O-C_6H_4$ |
| 1.365. | $CH(CH_3)_2$ | $C(CH_3)_2-CH_2$ | $CH_3$ | $CH_2CH_2$ | $3-CN-C_6H_4$ |
| 1.366. | $CH(CH_3)_2$ | $C(CH_3)_2-CH_2$ | $CH_3$ | $CH_2CH_2$ | $2-Cl-C_6H_4$ |
| 1.367. | $CH(CH_3)_2$ | $C(CH_3)_2-CH_2$ | $CH_3$ | $CH_2CH_2$ | $2-CH_3-C_6H_4$ |
| 1.368. | $CH(CH_3)_2$ | $C(CH_3)_2-CH_2$ | $CH_3$ | $CH_2CH_2$ | $2-CH_3O-C_6H_4$ |
| 1.369. | $CH(CH_3)_2$ | $C(CH_3)_2-CH_2$ | $CH_3$ | $CH_2CH_2$ | $2-CN-C_6H_4$ |
| 1.370. | $CH(CH_3)_2$ | $C(CH_3)_2-CH_2$ | $CH_3$ | $CH_2CH_2$ | $3,4-(CH_3O)_2C_6H_3$ |
| 1.371. | $CH(CH_3)_2$ | $C(CH_3)_2-CH_2$ | $CH_3$ | $CH_2CH_2$ | $2,4-Cl_2-C_6H_3$ |
| 1.372. | $CH(CH_3)_2$ | $C(CH_3)_2-CH_2$ | $CH_3$ | $CH_2CH_2$ | $2,4-(CH_3)_2-C_6H_3$ |
| 1.373. | $CH(CH_3)_2$ | $C(CH_3)_2-CH_2$ | $CH_3$ | $CH_2CH_2$ | $3,4-(CH_3)_2C_6H_3$ |
| 1.374. | $CH(CH_3)_2$ | $C(CH_3)_2-CH_2$ | $CH_3$ | $CH_2CH_2$ | $4-NO_2-C_6H_4$ |
| 1.375. | $CH(CH_3)C_2H_5$ | $C(CH_3)_2-CH_2$ | $CH_3$ | $CH_2CH_2$ | $4-Cl-C_6H_4$ |
| 1.376. | $CH(CH_3)C_2H_5$ | $C(CH_3)_2-CH_2$ | $CH_3$ | $CH_2CH_2$ | $4-CH_3-C_6H_4$ |
| 1.377. | $CH(CH_3)C_2H_5$ | $C(CH_3)_2-CH_2$ | $CH_3$ | $CH_2CH_2$ | $4-CH_3O-C_6H_4$ |
| 1.378. | $CH(CH_3)C_2H_5$ | $C(CH_3)_2-CH_2$ | $CH_3$ | $CH_2CH_2$ | $4-CN-C_6H_4$ |

| Nr. | $R^1$ | A | Y | Z | Ar |
|---|---|---|---|---|---|
| 1.379. | $CH(CH_3)C_2H_5$ | $C(CH_3)_2-CH_2$ | $CH_3$ | $CH_2CH_2$ | $3-Cl-C_6H_4$ |
| 1.380. | $CH(CH_3)C_2H_5$ | $C(CH_3)_2-CH_2$ | $CH_3$ | $CH_2CH_2$ | $3-CH_3-C_6H_4$ |
| 1.381. | $CH(CH_3)C_2H_5$ | $C(CH_3)_2-CH_2$ | $CH_3$ | $CH_2CH_2$ | $3-CH_3O-C_6H_4$ |
| 1.382. | $CH(CH_3)C_2H_5$ | $C(CH_3)_2-CH_2$ | $CH_3$ | $CH_2CH_2$ | $3-CN-C_6H_4$ |
| 1.383. | $CH(CH_3)C_2H_5$ | $C(CH_3)_2-CH_2$ | $CH_3$ | $CH_2CH_2$ | $2-Cl-C_6H_4$ |
| 1.384. | $CH(CH_3)C_2H_5$ | $C(CH_3)_2-CH_2$ | $CH_3$ | $CH_2CH_2$ | $2-CH_3-C_6H_4$ |
| 1.385. | $CH(CH_3)C_2H_5$ | $C(CH_3)_2-CH_2$ | $CH_3$ | $CH_2CH_2$ | $2-CH_3O-C_6H_4$ |
| 1.386. | $CH(CH_3)C_2H_5$ | $C(CH_3)_2-CH_2$ | $CH_3$ | $CH_2CH_2$ | $2-CN-C_6H_4$ |
| 1.387. | $CH(CH_3)C_2H_5$ | $C(CH_3)_2-CH_2$ | $CH_3$ | $CH_2CH_2$ | $3,4-(CH_3O)_2C_6H_3$ |
| 1.388. | $CH(CH_3)C_2H_5$ | $C(CH_3)_2-CH_2$ | $CH_3$ | $CH_2CH_2$ | $2,4-Cl_2-C_6H_3$ |
| 1.389. | $CH(CH_3)C_2H_5$ | $C(CH_3)_2-CH_2$ | $CH_3$ | $CH_2CH_2$ | $2,4-(CH_3)_2-C_6H_3$ |
| 1.390. | $CH(CH_3)C_2H_5$ | $C(CH_3)_2-CH_2$ | $CH_3$ | $CH_2CH_2$ | $3,4-(CH_3)_2C_6H_3$ |
| 1.391. | $CH(CH_3)C_2H_5$ | $C(CH_3)_2-CH_2$ | $CH_3$ | $CH_2CH_2$ | $4-NO_2-C_6H_4$ |
| 1.392. | $C(CH_3)_3$ | $C(CH_3)_2-CH_2$ | $CH_3$ | $CH_2CH_2$ | $4-Cl-C_6H_4$ |
| 1.393. | $C(CH_3)_3$ | $C(CH_3)_2-CH_2$ | $CH_3$ | $CH_2CH_2$ | $4-CH_3-C_6H_4$ |
| 1.394. | $C(CH_3)_3$ | $C(CH_3)_2-CH_2$ | $CH_3$ | $CH_2CH_2$ | $4-CH_3O-C_6H_4$ |
| 1.395. | $C(CH_3)_3$ | $C(CH_3)_2-CH_2$ | $CH_3$ | $CH_2CH_2$ | $4-CN-C_6H_4$ |
| 1.396. | $C(CH_3)_3$ | $C(CH_3)_2-CH_2$ | $CH_3$ | $CH_2CH_2$ | $3-Cl-C_6H_4$ |
| 1.397. | $C(CH_3)_3$ | $C(CH_3)_2-CH_2$ | $CH_3$ | $CH_2CH_2$ | $3-CH_3-C_6H_4$ |
| 1.398. | $C(CH_3)_3$ | $C(CH_3)_2-CH_2$ | $CH_3$ | $CH_2CH_2$ | $3-CH_3O-C_6H_4$ |
| 1.399. | $C(CH_3)_3$ | $C(CH_3)_2-CH_2$ | $CH_3$ | $CH_2CH_2$ | $3-CN-C_6H_4$ |
| 1.400. | $C(CH_3)_3$ | $C(CH_3)_2-CH_2$ | $CH_3$ | $CH_2CH_2$ | $2-Cl-C_6H_4$ |
| 1.401. | $C(CH_3)_3$ | $C(CH_3)_2-CH_2$ | $CH_3$ | $CH_2CH_2$ | $2-CH_3-C_6H_4$ |
| 1.402. | $C(CH_3)_3$ | $C(CH_3)_2-CH_2$ | $CH_3$ | $CH_2CH_2$ | $2-CH_3O-C_6H_4$ |
| 1.403. | $C(CH_3)_3$ | $C(CH_3)_2-CH_2$ | $CH_3$ | $CH_2CH_2$ | $2-CN-C_6H_4$ |
| 1.404. | $C(CH_3)_3$ | $C(CH_3)_2-CH_2$ | $CH_3$ | $CH_2CH_2$ | $3,4-(CH_3O)_2C_6H_3$ |
| 1.405. | $C(CH_3)_3$ | $C(CH_3)_2-CH_2$ | $CH_3$ | $CH_2CH_2$ | $2,4-Cl_2-C_6H_3$ |
| 1.406. | $C(CH_3)_3$ | $C(CH_3)_2-CH_2$ | $CH_3$ | $CH_2CH_2$ | $2,4-(CH_3)_2-C_6H_3$ |
| 1.407. | $C(CH_3)_3$ | $C(CH_3)_2-CH_2$ | $CH_3$ | $CH_2CH_2$ | $3,4-(CH_3)_2C_6H_3$ |
| 1.408. | $C(CH_3)_3$ | $C(CH_3)_2-CH_2$ | $CH_3$ | $CH_2CH_2$ | $4-NO_2-C_6H_4$ |
| 1.409. | $CH(CH_3)_2$ | $CH(CH_3)$ | H | $CH_2CH_2$ | $4-Cl-C_6H_4$ |
| 1.410. | $CH(CH_3)_2$ | $CH(CH_3)$ | H | $CH_2CH_2$ | $4-CH_3-C_6H_4$ |
| 1.411. | $CH(CH_3)_2$ | $CH(CH_3)$ | H | $CH_2CH_2$ | $4-CH_3O-C_6H_4$ |
| 1.412. | $CH(CH_3)_2$ | $CH(CH_3)$ | H | $CH_2CH_2$ | $4-CN-C_6H_4$ |
| 1.413. | $CH(CH_3)_2$ | $CH(CH_3)$ | H | $CH_2CH_2$ | $3-Cl-C_6H_4$ |
| 1.414. | $CH(CH_3)_2$ | $CH(CH_3)$ | H | $CH_2CH_2$ | $3-CH_3-C_6H_4$ |
| 1.415. | $CH(CH_3)_2$ | $CH(CH_3)$ | H | $CH_2CH_2$ | $3-CH_3O-C_6H_4$ |
| 1.416. | $CH(CH_3)_2$ | $CH(CH_3)$ | H | $CH_2CH_2$ | $3-CN-C_6H_4$ |
| 1.417. | $CH(CH_3)_2$ | $CH(CH_3)$ | H | $CH_2CH_2$ | $2-Cl-C_6H_4$ |

| Nr. | $R^1$ | A | Y | Z | Ar |
|---|---|---|---|---|---|
| 1.418. | $CH(CH_3)_2$ | $CH(CH_3)$ | H | $CH_2CH_2$ | $2-CH_3-C_6H_4$ |
| 1.419. | $CH(CH_3)_2$ | $CH(CH_3)$ | H | $CH_2CH_2$ | $2-CH_3O-C_6H_4$ |
| 1.420. | $CH(CH_3)_2$ | $CH(CH_3)$ | H | $CH_2CH_2$ | $2-CN-C_6H_4$ |
| 1.421. | $CH(CH_3)_2$ | $CH(CH_3)$ | H | $CH_2CH_2$ | $3,4-(CH_3O)_2C_6H_3$ |
| 1.422. | $CH(CH_3)_2$ | $CH(CH_3)$ | H | $CH_2CH_2$ | $2,4-Cl_2-C_6H_3$ |
| 1.423. | $CH(CH_3)_2$ | $CH(CH_3)$ | H | $CH_2CH_2$ | $2,4-(CH_3)_2-C_6H_3$ |
| 1.424. | $CH(CH_3)_2$ | $CH(CH_3)$ | H | $CH_2CH_2$ | $3,4-(CH_3)_2C_6H_3$ |
| 1.425. | $CH(CH_3)_2$ | $CH(CH_3)$ | H | $CH_2CH_2$ | $4-NO_2-C_6H_4$ |
| 1.426. | $CH(CH_3)C_2H_5$ | $CH(CH_3)$ | H | $CH_2CH_2$ | $4-Cl-C_6H_4$ |
| 1.427. | $CH(CH_3)C_2H_5$ | $CH(CH_3)$ | H | $CH_2CH_2$ | $4-CH_3-C_6H_4$ |
| 1.428. | $CH(CH_3)C_2H_5$ | $CH(CH_3)$ | H | $CH_2CH_2$ | $4-CH_3O-C_6H_4$ |
| 1.429. | $CH(CH_3)C_2H_5$ | $CH(CH_3)$ | H | $CH_2CH_2$ | $4-CN-C_6H_4$ |
| 1.430. | $CH(CH_3)C_2H_5$ | $CH(CH_3)$ | H | $CH_2CH_2$ | $3-Cl-C_6H_4$ |
| 1.431. | $CH(CH_3)C_2H_5$ | $CH(CH_3)$ | H | $CH_2CH_2$ | $3-CH_3-C_6H_4$ |
| 1.432. | $CH(CH_3)C_2H_5$ | $CH(CH_3)$ | H | $CH_2CH_2$ | $3-CH_3O-C_6H_4$ |
| 1.433. | $CH(CH_3)C_2H_5$ | $CH(CH_3)$ | H | $CH_2CH_2$ | $3-CN-C_6H_4$ |
| 1.434. | $CH(CH_3)C_2H_5$ | $CH(CH_3)$ | H | $CH_2CH_2$ | $2-Cl-C_6H_4$ |
| 1.435. | $CH(CH_3)C_2H_5$ | $CH(CH_3)$ | H | $CH_2CH_2$ | $2-CH_3-C_6H_4$ |
| 1.436. | $CH(CH_3)C_2H_5$ | $CH(CH_3)$ | H | $CH_2CH_2$ | $2-CH_3O-C_6H_4$ |
| 1.437. | $CH(CH_3)C_2H_5$ | $CH(CH_3)$ | H | $CH_2CH_2$ | $2-CN-C_6H_4$ |
| 1.438. | $CH(CH_3)C_2H_5$ | $CH(CH_3)$ | H | $CH_2CH_2$ | $3,4-(CH_3O)_2C_6H_3$ |
| 1.439. | $CH(CH_3)C_2H_5$ | $CH(CH_3)$ | H | $CH_2CH_2$ | $2,4-Cl_2-C_6H_3$ |
| 1.440. | $CH(CH_3)C_2H_5$ | $CH(CH_3)$ | H | $CH_2CH_2$ | $2,4-(CH_3)_2-C_6H_3$ |
| 1.441. | $CH(CH_3)C_2H_5$ | $CH(CH_3)$ | H | $CH_2CH_2$ | $3,4-(CH_3)_2C_6H_3$ |
| 1.442. | $CH(CH_3)C_2H_5$ | $CH(CH_3)$ | H | $CH_2CH_2$ | $4-NO_2-C_6H_4$ |
| 1.443. | $C(CH_3)_3$ | $CH(CH_3)$ | H | $CH_2CH_2$ | $4-Cl-C_6H_4$ |
| 1.444. | $C(CH_3)_3$ | $CH(CH_3)$ | H | $CH_2CH_2$ | $4-CH_3-C_6H_4$ |
| 1.445. | $C(CH_3)_3$ | $CH(CH_3)$ | H | $CH_2CH_2$ | $4-CH_3O-C_6H_4$ |
| 1.446. | $C(CH_3)_3$ | $CH(CH_3)$ | H | $CH_2CH_2$ | $4-CN-C_6H_4$ |
| 1.447. | $C(CH_3)_3$ | $CH(CH_3)$ | H | $CH_2CH_2$ | $3-Cl-C_6H_4$ |
| 1.448. | $C(CH_3)_3$ | $CH(CH_3)$ | H | $CH_2CH_2$ | $3-CH_3-C_6H_4$ |
| 1.449. | $C(CH_3)_3$ | $CH(CH_3)$ | H | $CH_2CH_2$ | $3-CH_3O-C_6H_4$ |
| 1.450. | $C(CH_3)_3$ | $CH(CH_3)$ | H | $CH_2CH_2$ | $3-CN-C_6H_4$ |
| 1.451. | $C(CH_3)_3$ | $CH(CH_3)$ | H | $CH_2CH_2$ | $2-Cl-C_6H_4$ |
| 1.452. | $C(CH_3)_3$ | $CH(CH_3)$ | H | $CH_2CH_2$ | $2-CH_3-C_6H_4$ |
| 1.453. | $C(CH_3)_3$ | $CH(CH_3)$ | H | $CH_2CH_2$ | $2-CH_3O-C_6H_4$ |
| 1.454. | $C(CH_3)_3$ | $CH(CH_3)$ | H | $CH_2CH_2$ | $2-CN-C_6H_4$ |
| 1.455. | $C(CH_3)_3$ | $CH(CH_3)$ | H | $CH_2CH_2$ | $3,4-(CH_3O)_2C_6H_3$ |
| 1.456. | $C(CH_3)_3$ | $CH(CH_3)$ | H | $CH_2CH_2$ | $2,4-Cl_2-C_6H_3$ |

| Nr. | $R^1$ | A | Y | Z | Ar |
|---|---|---|---|---|---|
| 1.457. | $C(CH_3)_3$ | $CH(CH_3)$ | H | $CH_2CH_2$ | $2,4-(CH_3)_2-C_6H_3$ |
| 1.458. | $C(CH_3)_3$ | $CH(CH_3)$ | H | $CH_2CH_2$ | $3,4-(CH_3)_2C_6H_3$ |
| 1.459. | $C(CH_3)_3$ | $CH(CH_3)$ | H | $CH_2CH_2$ | $4-NO_2-C_6H_4$ |
| 1.460. | $CH(CH_3)_2$ | $C(CH_3)_2-CH_2$ | H | $CH_2CH_2$ | $4-Cl-C_6H_4$ |
| 1.461. | $CH(CH_3)_2$ | $C(CH_3)_2-CH_2$ | H | $CH_2CH_2$ | $4-CH_3-C_6H_4$ |
| 1.462. | $CH(CH_3)_2$ | $C(CH_3)_2-CH_2$ | H | $CH_2CH_2$ | $4-CH_3O-C_6H_4$ |
| 1.463. | $CH(CH_3)_2$ | $C(CH_3)_2-CH_2$ | H | $CH_2CH_2$ | $4-CN-C_6H_4$ |
| 1.464. | $CH(CH_3)_2$ | $C(CH_3)_2-CH_2$ | H | $CH_2CH_2$ | $3-Cl-C_6H_4$ |
| 1.465. | $CH(CH_3)_2$ | $C(CH_3)_2-CH_2$ | H | $CH_2CH_2$ | $3-CH_3-C_6H_4$ |
| 1.466. | $CH(CH_3)_2$ | $C(CH_3)_2-CH_2$ | H | $CH_2CH_2$ | $3-CH_3O-C_6H_4$ |
| 1.467. | $CH(CH_3)_2$ | $C(CH_3)_2-CH_2$ | H | $CH_2CH_2$ | $3-CN-C_6H_4$ |
| 1.468. | $CH(CH_3)_2$ | $C(CH_3)_2-CH_2$ | H | $CH_2CH_2$ | $2-Cl-C_6H_4$ |
| 1.469. | $CH(CH_3)_2$ | $C(CH_3)_2-CH_2$ | H | $CH_2CH_2$ | $2-CH_3-C_6H_4$ |
| 1.470. | $CH(CH_3)_2$ | $C(CH_3)_2-CH_2$ | H | $CH_2CH_2$ | $2-CH_3O-C_6H_4$ |
| 1.471. | $CH(CH_3)_2$ | $C(CH_3)_2-CH_2$ | H | $CH_2CH_2$ | $2-CN-C_6H_4$ |
| 1.472. | $CH(CH_3)_2$ | $C(CH_3)_2-CH_2$ | H | $CH_2CH_2$ | $3,4-(CH_3O)_2C_6H_3$ |
| 1.473. | $CH(CH_3)_2$ | $C(CH_3)_2-CH_2$ | H | $CH_2CH_2$ | $2,4-Cl_2-C_6H_3$ |
| 1.474. | $CH(CH_3)_2$ | $C(CH_3)_2-CH_2$ | H | $CH_2CH_2$ | $2,4-(CH_3)_2-C_6H_3$ |
| 1.475. | $CH(CH_3)_2$ | $C(CH_3)_2-CH_2$ | H | $CH_2CH_2$ | $3,4-(CH_3)_2C_6H_3$ |
| 1.476. | $CH(CH_3)_2$ | $C(CH_3)_2-CH_2$ | H | $CH_2CH_2$ | $4-NO_2-C_6H_4$ |
| 1.477. | $CH(CH_3)C_2H_5$ | $C(CH_3)_2-CH_2$ | H | $CH_2CH_2$ | $4-Cl-C_6H_4$ |
| 1.478. | $CH(CH_3)C_2H_5$ | $C(CH_3)_2-CH_2$ | H | $CH_2CH_2$ | $4-CH_3-C_6H_4$ |
| 1.479. | $CH(CH_3)C_2H_5$ | $C(CH_3)_2-CH_2$ | H | $CH_2CH_2$ | $4-CH_3O-C_6H_4$ |
| 1.480. | $CH(CH_3)C_2H_5$ | $C(CH_3)_2-CH_2$ | H | $CH_2CH_2$ | $4-CN-C_6H_4$ |
| 1.481. | $CH(CH_3)C_2H_5$ | $C(CH_3)_2-CH_2$ | H | $CH_2CH_2$ | $3-Cl-C_6H_4$ |
| 1.482. | $CH(CH_3)C_2H_5$ | $C(CH_3)_2-CH_2$ | H | $CH_2CH_2$ | $3-CH_3-C_6H_4$ |
| 1.483. | $CH(CH_3)C_2H_5$ | $C(CH_3)_2-CH_2$ | H | $CH_2CH_2$ | $3-CH_3O-C_6H_4$ |
| 1.484. | $CH(CH_3)C_2H_5$ | $C(CH_3)_2-CH_2$ | H | $CH_2CH_2$ | $3-CN-C_6H_4$ |
| 1.485. | $CH(CH_3)C_2H_5$ | $C(CH_3)_2-CH_2$ | H | $CH_2CH_2$ | $2-Cl-C_6H_4$ |
| 1.486. | $CH(CH_3)C_2H_5$ | $C(CH_3)_2-CH_2$ | H | $CH_2CH_2$ | $2-CH_3-C_6H_4$ |
| 1.487. | $CH(CH_3)C_2H_5$ | $C(CH_3)_2-CH_2$ | H | $CH_2CH_2$ | $2-CH_3O-C_6H_4$ |
| 1.488. | $CH(CH_3)C_2H_5$ | $C(CH_3)_2-CH_2$ | H | $CH_2CH_2$ | $2-CN-C_6H_4$ |
| 1.489. | $CH(CH_3)C_2H_5$ | $C(CH_3)_2-CH_2$ | H | $CH_2CH_2$ | $3,4-(CH_3O)_2C_6H_3$ |
| 1.490. | $CH(CH_3)C_2H_5$ | $C(CH_3)_2-CH_2$ | H | $CH_2CH_2$ | $2,4-Cl_2-C_6H_3$ |
| 1.491. | $CH(CH_3)C_2H_5$ | $C(CH_3)_2-CH_2$ | H | $CH_2CH_2$ | $2,4-(CH_3)_2-C_6H_3$ |
| 1.492. | $CH(CH_3)C_2H_5$ | $C(CH_3)_2-CH_2$ | H | $CH_2CH_2$ | $3,4-(CH_3)_2C_6H_3$ |
| 1.493. | $CH(CH_3)C_2H_5$ | $C(CH_3)_2-CH_2$ | H | $CH_2CH_2$ | $4-NO_2-C_6H_4$ |
| 1.494. | $C(CH_3)_3$ | $C(CH_3)_2-CH_2$ | H | $CH_2CH_2$ | $4-Cl-C_6H_4$ |
| 1.495. | $C(CH_3)_3$ | $C(CH_3)_2-CH_2$ | H | $CH_2CH_2$ | $4-CH_3-C_6H_4$ |

| Nr. | $R^1$ | A | Y | Z | Ar |
|---|---|---|---|---|---|
| 1.496. | $C(CH_3)_3$ | $C(CH_3)_2-CH_2$ | H | $CH_2CH_2$ | $4-CH_3O-C_6H_4$ |
| 1.497. | $C(CH_3)_3$ | $C(CH_3)_2-CH_2$ | H | $CH_2CH_2$ | $4-CN-C_6H_4$ |
| 1.498. | $C(CH_3)_3$ | $C(CH_3)_2-CH_2$ | H | $CH_2CH_2$ | $3-Cl-C_6H_4$ |
| 1.499. | $C(CH_3)_3$ | $C(CH_3)_2-CH_2$ | H | $CH_2CH_2$ | $3-CH_3-C_6H_4$ |
| 1.500. | $C(CH_3)_3$ | $C(CH_3)_2-CH_2$ | H | $CH_2CH_2$ | $3-CH_3O-C_6H_4$ |
| 1.501. | $C(CH_3)_3$ | $C(CH_3)_2-CH_2$ | H | $CH_2CH_2$ | $3-CN-C_6H_4$ |
| 1.502. | $C(CH_3)_3$ | $C(CH_3)_2-CH_2$ | H | $CH_2CH_2$ | $2-Cl-C_6H_4$ |
| 1.503. | $C(CH_3)_3$ | $C(CH_3)_2-CH_2$ | H | $CH_2CH_2$ | $2-CH_3-C_6H_4$ |
| 1.504. | $C(CH_3)_3$ | $C(CH_3)_2-CH_2$ | H | $CH_2CH_2$ | $2-CH_3O-C_6H_4$ |
| 1.505. | $C(CH_3)_3$ | $C(CH_3)_2-CH_2$ | H | $CH_2CH_2$ | $2-CN-C_6H_4$ |
| 1.506. | $C(CH_3)_3$ | $C(CH_3)_2-CH_2$ | H | $CH_2CH_2$ | $3,4-(CH_3O)_2C_6H_3$ |
| 1.507. | $C(CH_3)_3$ | $C(CH_3)_2-CH_2$ | H | $CH_2CH_2$ | $2,4-Cl_2-C_6H_3$ |
| 1.508. | $C(CH_3)_3$ | $C(CH_3)_2-CH_2$ | H | $CH_2CH_2$ | $2,4-(CH_3)_2-C_6H_3$ |
| 1.509. | $C(CH_3)_3$ | $C(CH_3)_2-CH_2$ | H | $CH_2CH_2$ | $3,4-(CH_3)_2C_6H_3$ |
| 1.510. | $C(CH_3)_3$ | $C(CH_3)_2-CH_2$ | H | $CH_2CH_2$ | $4-NO_2-C_6H_4$ |
| 1.511. | $CH(CH_3)_2$ | $CH_2$ | $CH_3$ | $CH_2CH_2$ | $4-Cl-C_6H_4$ |
| 1.512. | $CH(CH_3)_2$ | $CH_2$ | $CH_3$ | $CH_2CH_2$ | $4-CH_3-C_6H_4$ |
| 1.513. | $CH(CH_3)_2$ | $CH_2$ | $CH_3$ | $CH_2CH_2$ | $4-CH_3O-C_6H_4$ |
| 1.514. | $CH(CH_3)_2$ | $CH_2$ | $CH_3$ | $CH_2CH_2$ | $4-CN-C_6H_4$ |
| 1.515. | $CH(CH_3)_2$ | $CH_2$ | $CH_3$ | $CH_2CH_2$ | $3-Cl-C_6H_4$ |
| 1.516. | $CH(CH_3)_2$ | $CH_2$ | $CH_3$ | $CH_2CH_2$ | $3-CH_3-C_6H_4$ |
| 1.517. | $CH(CH_3)_2$ | $CH_2$ | $CH_3$ | $CH_2CH_2$ | $3-CH_3O-C_6H_4$ |
| 1.518. | $CH(CH_3)_2$ | $CH_2$ | $CH_3$ | $CH_2CH_2$ | $3-CN-C_6H_4$ |
| 1.519. | $CH(CH_3)_2$ | $CH_2$ | $CH_3$ | $CH_2CH_2$ | $2-Cl-C_6H_4$ |
| 1.520. | $CH(CH_3)_2$ | $CH_2$ | $CH_3$ | $CH_2CH_2$ | $2-CH_3-C_6H_4$ |
| 1.521. | $CH(CH_3)_2$ | $CH_2$ | $CH_3$ | $CH_2CH_2$ | $2-CH_3O-C_6H_4$ |
| 1.522. | $CH(CH_3)_2$ | $CH_2$ | $CH_3$ | $CH_2CH_2$ | $2-CN-C_6H_4$ |
| 1.523. | $CH(CH_3)_2$ | $CH_2$ | $CH_3$ | $CH_2CH_2$ | $3,4-(CH_3O)_2C_6H_3$ |
| 1.524. | $CH(CH_3)_2$ | $CH_2$ | $CH_3$ | $CH_2CH_2$ | $2,4-Cl_2-C_6H_3$ |
| 1.525. | $CH(CH_3)_2$ | $CH_2$ | $CH_3$ | $CH_2CH_2$ | $2,4-(CH_3)_2-C_6H_3$ |
| 1.526. | $CH(CH_3)_2$ | $CH_2$ | $CH_3$ | $CH_2CH_2$ | $3,4-(CH_3)_2C_6H_3$ |
| 1.527. | $CH(CH_3)_2$ | $CH_2$ | $CH_3$ | $CH_2CH_2$ | $4-NO_2-C_6H_4$ |
| 1.528. | $CH(CH_3)C_2H_5$ | $CH_2$ | $CH_3$ | $CH_2CH_2$ | $4-Cl-C_6H_4$ |
| 1.529. | $CH(CH_3)C_2H_5$ | $CH_2$ | $CH_3$ | $CH_2CH_2$ | $4-CH_3-C_6H_4$ |
| 1.530. | $CH(CH_3)C_2H_5$ | $CH_2$ | $CH_3$ | $CH_2CH_2$ | $4-CH_3O-C_6H_4$ |
| 1.531. | $CH(CH_3)C_2H_5$ | $CH_2$ | $CH_3$ | $CH_2CH_2$ | $4-CN-C_6H_4$ |
| 1.532. | $CH(CH_3)C_2H_5$ | $CH_2$ | $CH_3$ | $CH_2CH_2$ | $3-Cl-C_6H_4$ |
| 1.533. | $CH(CH_3)C_2H_5$ | $CH_2$ | $CH_3$ | $CH_2CH_2$ | $3-CH_3-C_6H_4$ |
| 1.534. | $CH(CH_3)C_2H_5$ | $CH_2$ | $CH_3$ | $CH_2CH_2$ | $3-CH_3O-C_6H_4$ |

| Nr. | $R^1$ | A | Y | Z | Ar |
|---|---|---|---|---|---|
| 1.535. | $CH(CH_3)C_2H_5$ | $CH_2$ | $CH_3$ | $CH_2CH_2$ | $3-CN-C_6H_4$ |
| 1.536. | $CH(CH_3)C_2H_5$ | $CH_2$ | $CH_3$ | $CH_2CH_2$ | $2-Cl-C_6H_4$ |
| 1.537. | $CH(CH_3)C_2H_5$ | $CH_2$ | $CH_3$ | $CH_2CH_2$ | $2-CH_3-C_6H_4$ |
| 1.538. | $CH(CH_3)C_2H_5$ | $CH_2$ | $CH_3$ | $CH_2CH_2$ | $2-CH_3O-C_6H_4$ |
| 1.539. | $CH(CH_3)C_2H_5$ | $CH_2$ | $CH_3$ | $CH_2CH_2$ | $2-CN-C_6H_4$ |
| 1.540. | $CH(CH_3)C_2H_5$ | $CH_2$ | $CH_3$ | $CH_2CH_2$ | $3,4-(CH_3O)_2C_6H_3$ |
| 1.541. | $CH(CH_3)C_2H_5$ | $CH_2$ | $CH_3$ | $CH_2CH_2$ | $2,4-Cl_2-C_6H_3$ |
| 1.542. | $CH(CH_3)C_2H_5$ | $CH_2$ | $CH_3$ | $CH_2CH_2$ | $2,4-(CH_3)_2-C_6H_3$ |
| 1.543. | $CH(CH_3)C_2H_5$ | $CH_2$ | $CH_3$ | $CH_2CH_2$ | $3,4-(CH_3)_2C_6H_3$ |
| 1.544. | $CH(CH_3)C_2H_5$ | $CH_2$ | $CH_3$ | $CH_2CH_2$ | $4-NO_2-C_6H_4$ |
| 1.545. | $C(CH_3)_3$ | $CH_2$ | $CH_3$ | $CH_2CH_2$ | $4-Cl-C_6H_4$ |
| 1.546. | $C(CH_3)_3$ | $CH_2$ | $CH_3$ | $CH_2CH_2$ | $4-CH_3-C_6H_4$ |
| 1.547. | $C(CH_3)_3$ | $CH_2$ | $CH_3$ | $CH_2CH_2$ | $4-CH_3O-C_6H_4$ |
| 1.548. | $C(CH_3)_3$ | $CH_2$ | $CH_3$ | $CH_2CH_2$ | $4-CN-C_6H_4$ |
| 1.549. | $C(CH_3)_3$ | $CH_2$ | $CH_3$ | $CH_2CH_2$ | $3-Cl-C_6H_4$ |
| 1.550. | $C(CH_3)_3$ | $CH_2$ | $CH_3$ | $CH_2CH_2$ | $3-CH_3-C_6H_4$ |
| 1.551. | $C(CH_3)_3$ | $CH_2$ | $CH_3$ | $CH_2CH_2$ | $3-CH_3O-C_6H_4$ |
| 1.552. | $C(CH_3)_3$ | $CH_2$ | $CH_3$ | $CH_2CH_2$ | $3-CN-C_6H_4$ |
| 1.553. | $C(CH_3)_3$ | $CH_2$ | $CH_3$ | $CH_2CH_2$ | $2-Cl-C_6H_4$ |
| 1.554. | $C(CH_3)_3$ | $CH_2$ | $CH_3$ | $CH_2CH_2$ | $2-CH_3-C_6H_4$ |
| 1.555. | $C(CH_3)_3$ | $CH_2$ | $CH_3$ | $CH_2CH_2$ | $2-CH_3O-C_6H_4$ |
| 1.556. | $C(CH_3)_3$ | $CH_2$ | $CH_3$ | $CH_2CH_2$ | $2-CN-C_6H_4$ |
| 1.557. | $C(CH_3)_3$ | $CH_2$ | $CH_3$ | $CH_2CH_2$ | $3,4-(CH_3O)_2C_6H_3$ |
| 1.558. | $C(CH_3)_3$ | $CH_2$ | $CH_3$ | $CH_2CH_2$ | $2,4-Cl_2-C_6H_3$ |
| 1.559. | $C(CH_3)_3$ | $CH_2$ | $CH_3$ | $CH_2CH_2$ | $2,4-(CH_3)_2-C_6H_3$ |
| 1.560. | $C(CH_3)_3$ | $CH_2$ | $CH_3$ | $CH_2CH_2$ | $3,4-(CH_3)_2C_6H_3$ |
| 1.561. | $C(CH_3)_3$ | $CH_2$ | $CH_3$ | $CH_2CH_2$ | $4-NO_2-C_6H_4$ |
| 1.562. | $CH(CH_3)_2$ | $CH_2$ | H | $CH_2CH_2$ | $4-Cl-C_6H_4$ |
| 1.563. | $CH(CH_3)_2$ | $CH_2$ | H | $CH_2CH_2$ | $4-CH_3-C_6H_4$ |
| 1.564. | $CH(CH_3)_2$ | $CH_2$ | H | $CH_2CH_2$ | $4-CH_3O-C_6H_4$ |
| 1.565. | $CH(CH_3)_2$ | $CH_2$ | H | $CH_2CH_2$ | $4-CN-C_6H_4$ |
| 1.566. | $CH(CH_3)_2$ | $CH_2$ | H | $CH_2CH_2$ | $3-Cl-C_6H_4$ |
| 1.567. | $CH(CH_3)_2$ | $CH_2$ | H | $CH_2CH_2$ | $3-CH_3-C_6H_4$ |
| 1.568. | $CH(CH_3)_2$ | $CH_2$ | H | $CH_2CH_2$ | $3-CH_3O-C_6H_4$ |
| 1.569. | $CH(CH_3)_2$ | $CH_2$ | H | $CH_2CH_2$ | $3-CN-C_6H_4$ |
| 1.570. | $CH(CH_3)_2$ | $CH_2$ | H | $CH_2CH_2$ | $2-Cl-C_6H_4$ |
| 1.571. | $CH(CH_3)_2$ | $CH_2$ | H | $CH_2CH_2$ | $2-CH_3-C_6H_4$ |
| 1.572. | $CH(CH_3)_2$ | $CH_2$ | H | $CH_2CH_2$ | $2-CH_3O-C_6H_4$ |
| 1.573. | $CH(CH_3)_2$ | $CH_2$ | H | $CH_2CH_2$ | $2-CN-C_6H_4$ |

| Nr. | $R^1$ | A | Y | Z | Ar |
|---|---|---|---|---|---|
| 1.574. | $CH(CH_3)_2$ | $CH_2$ | H | $CH_2CH_2$ | $3,4-(CH_3O)_2C_6H_3$ |
| 1.575. | $CH(CH_3)_2$ | $CH_2$ | H | $CH_2CH_2$ | $2,4-Cl_2-C_6H_3$ |
| 1.576. | $CH(CH_3)_2$ | $CH_2$ | H | $CH_2CH_2$ | $2,4-(CH_3)_2-C_6H_3$ |
| 1.577. | $CH(CH_3)_2$ | $CH_2$ | H | $CH_2CH_2$ | $3,4-(CH_3)_2C_6H_3$ |
| 1.578. | $CH(CH_3)_2$ | $CH_2$ | H | $CH_2CH_2$ | $4-NO_2-C_6H_4$ |
| 1.579. | $CH(CH_3)C_2H_5$ | $CH_2$ | H | $CH_2CH_2$ | $4-Cl-C_6H_4$ |
| 1.580. | $CH(CH_3)C_2H_5$ | $CH_2$ | H | $CH_2CH_2$ | $4-CH_3-C_6H_4$ |
| 1.581. | $CH(CH_3)C_2H_5$ | $CH_2$ | H | $CH_2CH_2$ | $4-CH_3O-C_6H_4$ |
| 1.582. | $CH(CH_3)C_2H_5$ | $CH_2$ | H | $CH_2CH_2$ | $4-CN-C_6H_4$ |
| 1.583. | $CH(CH_3)C_2H_5$ | $CH_2$ | H | $CH_2CH_2$ | $3-Cl-C_6H_4$ |
| 1.584. | $CH(CH_3)C_2H_5$ | $CH_2$ | H | $CH_2CH_2$ | $3-CH_3-C_6H_4$ |
| 1.585. | $CH(CH_3)C_2H_5$ | $CH_2$ | H | $CH_2CH_2$ | $3-CH_3O-C_6H_4$ |
| 1.586. | $CH(CH_3)C_2H_5$ | $CH_2$ | H | $CH_2CH_2$ | $3-CN-C_6H_4$ |
| 1.587. | $CH(CH_3)C_2H_5$ | $CH_2$ | H | $CH_2CH_2$ | $2-Cl-C_6H_4$ |
| 1.588. | $CH(CH_3)C_2H_5$ | $CH_2$ | H | $CH_2CH_2$ | $2-CH_3-C_6H_4$ |
| 1.589. | $CH(CH_3)C_2H_5$ | $CH_2$ | H | $CH_2CH_2$ | $2-CH_3O-C_6H_4$ |
| 1.590. | $CH(CH_3)C_2H_5$ | $CH_2$ | H | $CH_2CH_2$ | $2-CN-C_6H_4$ |
| 1.591. | $CH(CH_3)C_2H_5$ | $CH_2$ | H | $CH_2CH_2$ | $3,4-(CH_3O)_2C_6H_3$ |
| 1.592. | $CH(CH_3)C_2H_5$ | $CH_2$ | H | $CH_2CH_2$ | $2,4-Cl_2-C_6H_3$ |
| 1.593. | $CH(CH_3)C_2H_5$ | $CH_2$ | H | $CH_2CH_2$ | $2,4-(CH_3)_2-C_6H_3$ |
| 1.594. | $CH(CH_3)C_2H_5$ | $CH_2$ | H | $CH_2CH_2$ | $3,4-(CH_3)_2C_6H_3$ |
| 1.595. | $CH(CH_3)C_2H_5$ | $CH_2$ | H | $CH_2CH_2$ | $4-NO_2-C_6H_4$ |
| 1.596. | $C(CH_3)_3$ | $CH_2$ | H | $CH_2CH_2$ | $4-Cl-C_6H_4$ |
| 1.597. | $C(CH_3)_3$ | $CH_2$ | H | $CH_2CH_2$ | $4-CH_3-C_6H_4$ |
| 1.598. | $C(CH_3)_3$ | $CH_2$ | H | $CH_2CH_2$ | $4-CH_3O-C_6H_4$ |
| 1.599. | $C(CH_3)_3$ | $CH_2$ | H | $CH_2CH_2$ | $4-CN-C_6H_4$ |
| 1.600. | $C(CH_3)_3$ | $CH_2$ | H | $CH_2CH_2$ | $3-Cl-C_6H_4$ |
| 1.601. | $C(CH_3)_3$ | $CH_2$ | H | $CH_2CH_2$ | $3-CH_3-C_6H_4$ |
| 1.602. | $C(CH_3)_3$ | $CH_2$ | H | $CH_2CH_2$ | $3-CH_3O-C_6H_4$ |
| 1.603. | $C(CH_3)_3$ | $CH_2$ | H | $CH_2CH_2$ | $3-CN-C_6H_4$ |
| 1.604. | $C(CH_3)_3$ | $CH_2$ | H | $CH_2CH_2$ | $2-Cl-C_6H_4$ |
| 1.605. | $C(CH_3)_3$ | $CH_2$ | H | $CH_2CH_2$ | $2-CH_3-C_6H_4$ |
| 1.606. | $C(CH_3)_3$ | $CH_2$ | H | $CH_2CH_2$ | $2-CH_3O-C_6H_4$ |
| 1.607. | $C(CH_3)_3$ | $CH_2$ | H | $CH_2CH_2$ | $2-CN-C_6H_4$ |
| 1.608. | $C(CH_3)_3$ | $CH_2$ | H | $CH_2CH_2$ | $3,4-(CH_3O)_2C_6H_3$ |
| 1.609. | $C(CH_3)_3$ | $CH_2$ | H | $CH_2CH_2$ | $2,4-Cl_2-C_6H_3$ |
| 1.610. | $C(CH_3)_3$ | $CH_2$ | H | $CH_2CH_2$ | $2,4-(CH_3)_2-C_6H_3$ |
| 1.611. | $C(CH_3)_3$ | $CH_2$ | H | $CH_2CH_2$ | $3,4-(CH_3)_2C_6H_3$ |
| 1.612. | $C(CH_3)_3$ | $CH_2$ | H | $CH_2CH_2$ | $4-NO_2-C_6H_4$ |

| Nr. | $R^1$ | A | Y | Z | Ar |
|---|---|---|---|---|---|
| 1.613. | $CH(CH_3)_2$ | $CH(CH_3)$ | $CH_3$ | $CH(CH_3)$ | $4-Cl-C_6H_4$ |
| 1.614. | $CH(CH_3)_2$ | $CH(CH_3)$ | $CH_3$ | $CH(CH_3)$ | $4-CH_3-C_6H_4$ |
| 1.615. | $CH(CH_3)_2$ | $CH(CH_3)$ | $CH_3$ | $CH(CH_3)$ | $4-CH_3O-C_6H_4$ |
| 1.616. | $CH(CH_3)_2$ | $CH(CH_3)$ | $CH_3$ | $CH(CH_3)$ | $4-CN-C_6H_4$ |
| 1.617. | $CH(CH_3)_2$ | $CH(CH_3)$ | $CH_3$ | $CH(CH_3)$ | $3-Cl-C_6H_4$ |
| 1.618. | $CH(CH_3)_2$ | $CH(CH_3)$ | $CH_3$ | $CH(CH_3)$ | $3-CH_3-C_6H_4$ |
| 1.619. | $CH(CH_3)_2$ | $CH(CH_3)$ | $CH_3$ | $CH(CH_3)$ | $3-CH_3O-C_6H_4$ |
| 1.620. | $CH(CH_3)_2$ | $CH(CH_3)$ | $CH_3$ | $CH(CH_3)$ | $3-CN-C_6H_4$ |
| 1.621. | $CH(CH_3)_2$ | $CH(CH_3)$ | $CH_3$ | $CH(CH_3)$ | $2-Cl-C_6H_4$ |
| 1.622. | $CH(CH_3)_2$ | $CH(CH_3)$ | $CH_3$ | $CH(CH_3)$ | $2-CH_3-C_6H_4$ |
| 1.623. | $CH(CH_3)_2$ | $CH(CH_3)$ | $CH_3$ | $CH(CH_3)$ | $2-CH_3O-C_6H_4$ |
| 1.624. | $CH(CH_3)_2$ | $CH(CH_3)$ | $CH_3$ | $CH(CH_3)$ | $2-CN-C_6H_4$ |
| 1.625. | $CH(CH_3)_2$ | $CH(CH_3)$ | $CH_3$ | $CH(CH_3)$ | $3,4-(CH_3O)_2C_6H_3$ |
| 1.626. | $CH(CH_3)_2$ | $CH(CH_3)$ | $CH_3$ | $CH(CH_3)$ | $2,4-Cl_2-C_6H_3$ |
| 1.627. | $CH(CH_3)_2$ | $CH(CH_3)$ | $CH_3$ | $CH(CH_3)$ | $2,4-(CH_3)_2-C_6H_3$ |
| 1.628. | $CH(CH_3)_2$ | $CH(CH_3)$ | $CH_3$ | $CH(CH_3)$ | $3,4-(CH_3)_2C_6H_3$ |
| 1.629. | $CH(CH_3)_2$ | $CH(CH_3)$ | $CH_3$ | $CH(CH_3)$ | $4-NO_2-C_6H_4$ |
| 1.630. | $CH(CH_3)C_2H_5$ | $CH(CH_3)$ | $CH_3$ | $CH(CH_3)$ | $4-Cl-C_6H_4$ |
| 1.631. | $CH(CH_3)C_2H_5$ | $CH(CH_3)$ | $CH_3$ | $CH(CH_3)$ | $4-CH_3-C_6H_4$ |
| 1.632. | $CH(CH_3)C_2H_5$ | $CH(CH_3)$ | $CH_3$ | $CH(CH_3)$ | $4-CH_3O-C_6H_4$ |
| 1.633. | $CH(CH_3)C_2H_5$ | $CH(CH_3)$ | $CH_3$ | $CH(CH_3)$ | $4-CN-C_6H_4$ |
| 1.634. | $CH(CH_3)C_2H_5$ | $CH(CH_3)$ | $CH_3$ | $CH(CH_3)$ | $3-Cl-C_6H_4$ |
| 1.635. | $CH(CH_3)C_2H_5$ | $CH(CH_3)$ | $CH_3$ | $CH(CH_3)$ | $3-CH_3-C_6H_4$ |
| 1.636. | $CH(CH_3)C_2H_5$ | $CH(CH_3)$ | $CH_3$ | $CH(CH_3)$ | $3-CH_3O-C_6H_4$ |
| 1.637. | $CH(CH_3)C_2H_5$ | $CH(CH_3)$ | $CH_3$ | $CH(CH_3)$ | $3-CN-C_6H_4$ |
| 1.638. | $CH(CH_3)C_2H_5$ | $CH(CH_3)$ | $CH_3$ | $CH(CH_3)$ | $2-Cl-C_6H_4$ |
| 1.639. | $CH(CH_3)C_2H_5$ | $CH(CH_3)$ | $CH_3$ | $CH(CH_3)$ | $2-CH_3-C_6H_4$ |
| 1.640. | $CH(CH_3)C_2H_5$ | $CH(CH_3)$ | $CH_3$ | $CH(CH_3)$ | $2-CH_3O-C_6H_4$ |
| 1.641. | $CH(CH_3)C_2H_5$ | $CH(CH_3)$ | $CH_3$ | $CH(CH_3)$ | $2-CN-C_6H_4$ |
| 1.642. | $CH(CH_3)C_2H_5$ | $CH(CH_3)$ | $CH_3$ | $CH(CH_3)$ | $3,4-(CH_3O)_2C_6H_3$ |
| 1.643. | $CH(CH_3)C_2H_5$ | $CH(CH_3)$ | $CH_3$ | $CH(CH_3)$ | $2,4-Cl_2-C_6H_3$ |
| 1.644. | $CH(CH_3)C_2H_5$ | $CH(CH_3)$ | $CH_3$ | $CH(CH_3)$ | $2,4-(CH_3)_2-C_6H_3$ |
| 1.645. | $CH(CH_3)C_2H_5$ | $CH(CH_3)$ | $CH_3$ | $CH(CH_3)$ | $3,4-(CH_3)_2C_6H_3$ |
| 1.646. | $CH(CH_3)C_2H_5$ | $CH(CH_3)$ | $CH_3$ | $CH(CH_3)$ | $4-NO_2-C_6H_4$ |
| 1.647. | $C(CH_3)_3$ | $CH(CH_3)$ | $CH_3$ | $CH(CH_3)$ | $4-Cl-C_6H_4$ |
| 1.648. | $C(CH_3)_3$ | $CH(CH_3)$ | $CH_3$ | $CH(CH_3)$ | $4-CH_3-C_6H_4$ |
| 1.649. | $C(CH_3)_3$ | $CH(CH_3)$ | $CH_3$ | $CH(CH_3)$ | $4-CH_3O-C_6H_4$ |
| 1.650. | $C(CH_3)_3$ | $CH(CH_3)$ | $CH_3$ | $CH(CH_3)$ | $4-CN-C_6H_4$ |
| 1.651. | $C(CH_3)_3$ | $CH(CH_3)$ | $CH_3$ | $CH(CH_3)$ | $3-Cl-C_6H_4$ |

| Nr. | $R^1$ | A | Y | Z | Ar |
|---|---|---|---|---|---|
| 1.652. | $C(CH_3)_3$ | $CH(CH_3)$ | $CH_3$ | $CH(CH_3)$ | $3-CH_3-C_6H_4$ |
| 1.653. | $C(CH_3)_3$ | $CH(CH_3)$ | $CH_3$ | $CH(CH_3)$ | $3-CH_3O-C_6H_4$ |
| 1.654. | $C(CH_3)_3$ | $CH(CH_3)$ | $CH_3$ | $CH(CH_3)$ | $3-CN-C_6H_4$ |
| 1.655. | $C(CH_3)_3$ | $CH(CH_3)$ | $CH_3$ | $CH(CH_3)$ | $2-Cl-C_6H_4$ |
| 1.656. | $C(CH_3)_3$ | $CH(CH_3)$ | $CH_3$ | $CH(CH_3)$ | $2-CH_3-C_6H_4$ |
| 1.657. | $C(CH_3)_3$ | $CH(CH_3)$ | $CH_3$ | $CH(CH_3)$ | $2-CH_3O-C_6H_4$ |
| 1.658. | $C(CH_3)_3$ | $CH(CH_3)$ | $CH_3$ | $CH(CH_3)$ | $2-CN-C_6H_4$ |
| 1.659. | $C(CH_3)_3$ | $CH(CH_3)$ | $CH_3$ | $CH(CH_3)$ | $3,4-(CH_3O)_2C_6H_3$ |
| 1.660. | $C(CH_3)_3$ | $CH(CH_3)$ | $CH_3$ | $CH(CH_3)$ | $2,4-Cl_2-C_6H_3$ |
| 1.661. | $C(CH_3)_3$ | $CH(CH_3)$ | $CH_3$ | $CH(CH_3)$ | $2,4-(CH_3)_2-C_6H_3$ |
| 1.662. | $C(CH_3)_3$ | $CH(CH_3)$ | $CH_3$ | $CH(CH_3)$ | $3,4-(CH_3)_2C_6H_3$ |
| 1.663. | $C(CH_3)_3$ | $CH(CH_3)$ | $CH_3$ | $CH(CH_3)$ | $4-NO_2-C_6H_4$ |
| 1.664. | $CH(CH_3)_2$ | $C(CH_3)_2-CH_2$ | $CH_3$ | $CH(CH_3)$ | $4-Cl-C_6H_4$ |
| 1.665. | $CH(CH_3)_2$ | $C(CH_3)_2-CH_2$ | $CH_3$ | $CH(CH_3)$ | $4-CH_3-C_6H_4$ |
| 1.666. | $CH(CH_3)_2$ | $C(CH_3)_2-CH_2$ | $CH_3$ | $CH(CH_3)$ | $4-CH_3O-C_6H_4$ |
| 1.667. | $CH(CH_3)_2$ | $C(CH_3)_2-CH_2$ | $CH_3$ | $CH(CH_3)$ | $4-CN-C_6H_4$ |
| 1.668. | $CH(CH_3)_2$ | $C(CH_3)_2-CH_2$ | $CH_3$ | $CH(CH_3)$ | $3-Cl-C_6H_4$ |
| 1.669. | $CH(CH_3)_2$ | $C(CH_3)_2-CH_2$ | $CH_3$ | $CH(CH_3)$ | $3-CH_3-C_6H_4$ |
| 1.670. | $CH(CH_3)_2$ | $C(CH_3)_2-CH_2$ | $CH_3$ | $CH(CH_3)$ | $3-CH_3O-C_6H_4$ |
| 1.671. | $CH(CH_3)_2$ | $C(CH_3)_2-CH_2$ | $CH_3$ | $CH(CH_3)$ | $3-CN-C_6H_4$ |
| 1.672. | $CH(CH_3)_2$ | $C(CH_3)_2-CH_2$ | $CH_3$ | $CH(CH_3)$ | $2-Cl-C_6H_4$ |
| 1.673. | $CH(CH_3)_2$ | $C(CH_3)_2-CH_2$ | $CH_3$ | $CH(CH_3)$ | $2-CH_3-C_6H_4$ |
| 1.674. | $CH(CH_3)_2$ | $C(CH_3)_2-CH_2$ | $CH_3$ | $CH(CH_3)$ | $2-CH_3O-C_6H_4$ |
| 1.675. | $CH(CH_3)_2$ | $C(CH_3)_2-CH_2$ | $CH_3$ | $CH(CH_3)$ | $2-CN-C_6H_4$ |
| 1.676. | $CH(CH_3)_2$ | $C(CH_3)_2-CH_2$ | $CH_3$ | $CH(CH_3)$ | $3,4-(CH_3O)_2C_6H_3$ |
| 1.677. | $CH(CH_3)_2$ | $C(CH_3)_2-CH_2$ | $CH_3$ | $CH(CH_3)$ | $2,4-Cl_2-C_6H_3$ |
| 1.678. | $CH(CH_3)_2$ | $C(CH_3)_2-CH_2$ | $CH_3$ | $CH(CH_3)$ | $2,4-(CH_3)_2-C_6H_3$ |
| 1.679. | $CH(CH_3)_2$ | $C(CH_3)_2-CH_2$ | $CH_3$ | $CH(CH_3)$ | $3,4-(CH_3)_2C_6H_3$ |
| 1.680. | $CH(CH_3)_2$ | $C(CH_3)_2-CH_2$ | $CH_3$ | $CH(CH_3)$ | $4-NO_2-C_6H_4$ |
| 1.681. | $CH(CH_3)C_2H_5$ | $C(CH_3)_2-CH_2$ | $CH_3$ | $CH(CH_3)$ | $4-Cl-C_6H_4$ |
| 1.682. | $CH(CH_3)C_2H_5$ | $C(CH_3)_2-CH_2$ | $CH_3$ | $CH(CH_3)$ | $4-CH_3-C_6H_4$ |
| 1.683. | $CH(CH_3)C_2H_5$ | $C(CH_3)_2-CH_2$ | $CH_3$ | $CH(CH_3)$ | $4-CH_3O-C_6H_4$ |
| 1.684. | $CH(CH_3)C_2H_5$ | $C(CH_3)_2-CH_2$ | $CH_3$ | $CH(CH_3)$ | $4-CN-C_6H_4$ |
| 1.685. | $CH(CH_3)C_2H_5$ | $C(CH_3)_2-CH_2$ | $CH_3$ | $CH(CH_3)$ | $3-Cl-C_6H_4$ |
| 1.686. | $CH(CH_3)C_2H_5$ | $C(CH_3)_2-CH_2$ | $CH_3$ | $CH(CH_3)$ | $3-CH_3-C_6H_4$ |
| 1.687. | $CH(CH_3)C_2H_5$ | $C(CH_3)_2-CH_2$ | $CH_3$ | $CH(CH_3)$ | $3-CH_3O-C_6H_4$ |
| 1.688. | $CH(CH_3)C_2H_5$ | $C(CH_3)_2-CH_2$ | $CH_3$ | $CH(CH_3)$ | $3-CN-C_6H_4$ |
| 1.689. | $CH(CH_3)C_2H_5$ | $C(CH_3)_2-CH_2$ | $CH_3$ | $CH(CH_3)$ | $2-Cl-C_6H_4$ |
| 1.690. | $CH(CH_3)C_2H_5$ | $C(CH_3)_2-CH_2$ | $CH_3$ | $CH(CH_3)$ | $2-CH_3-C_6H_4$ |

| Nr. | $R^1$ | A | Y | Z | Ar |
|---|---|---|---|---|---|
| 1.691. | $CH(CH_3)C_2H_5$ | $C(CH_3)_2-CH_2$ | $CH_3$ | $CH(CH_3)$ | $2-CH_3O-C_6H_4$ |
| 1.692. | $CH(CH_3)C_2H_5$ | $C(CH_3)_2-CH_2$ | $CH_3$ | $CH(CH_3)$ | $2-CN-C_6H_4$ |
| 1.693. | $CH(CH_3)C_2H_5$ | $C(CH_3)_2-CH_2$ | $CH_3$ | $CH(CH_3)$ | $3,4-(CH_3O)_2C_6H_3$ |
| 1.694. | $CH(CH_3)C_2H_5$ | $C(CH_3)_2-CH_2$ | $CH_3$ | $CH(CH_3)$ | $2,4-Cl_2-C_6H_3$ |
| 1.695. | $CH(CH_3)C_2H_5$ | $C(CH_3)_2-CH_2$ | $CH_3$ | $CH(CH_3)$ | $2,4-(CH_3)_2-C_6H_3$ |
| 1.696. | $CH(CH_3)C_2H_5$ | $C(CH_3)_2-CH_2$ | $CH_3$ | $CH(CH_3)$ | $3,4-(CH_3)_2C_6H_3$ |
| 1.697. | $CH(CH_3)C_2H_5$ | $C(CH_3)_2-CH_2$ | $CH_3$ | $CH(CH_3)$ | $4-NO_2-C_6H_4$ |
| 1.698. | $C(CH_3)_3$ | $C(CH_3)_2-CH_2$ | $CH_3$ | $CH(CH_3)$ | $4-Cl-C_6H_4$ |
| 1.699. | $C(CH_3)_3$ | $C(CH_3)_2-CH_2$ | $CH_3$ | $CH(CH_3)$ | $4-CH_3-C_6H_4$ |
| 1.700. | $C(CH_3)_3$ | $C(CH_3)_2-CH_2$ | $CH_3$ | $CH(CH_3)$ | $4-CH_3O-C_6H_4$ |
| 1.701. | $C(CH_3)_3$ | $C(CH_3)_2-CH_2$ | $CH_3$ | $CH(CH_3)$ | $4-CN-C_6H_4$ |
| 1.702. | $C(CH_3)_3$ | $C(CH_3)_2-CH_2$ | $CH_3$ | $CH(CH_3)$ | $3-Cl-C_6H_4$ |
| 1.703. | $C(CH_3)_3$ | $C(CH_3)_2-CH_2$ | $CH_3$ | $CH(CH_3)$ | $3-CH_3-C_6H_4$ |
| 1.704. | $C(CH_3)_3$ | $C(CH_3)_2-CH_2$ | $CH_3$ | $CH(CH_3)$ | $3-CH_3O-C_6H_4$ |
| 1.705. | $C(CH_3)_3$ | $C(CH_3)_2-CH_2$ | $CH_3$ | $CH(CH_3)$ | $3-CN-C_6H_4$ |
| 1.706. | $C(CH_3)_3$ | $C(CH_3)_2-CH_2$ | $CH_3$ | $CH(CH_3)$ | $2-Cl-C_6H_4$ |
| 1.707. | $C(CH_3)_3$ | $C(CH_3)_2-CH_2$ | $CH_3$ | $CH(CH_3)$ | $2-CH_3-C_6H_4$ |
| 1.708. | $C(CH_3)_3$ | $C(CH_3)_2-CH_2$ | $CH_3$ | $CH(CH_3)$ | $2-CH_3O-C_6H_4$ |
| 1.709. | $C(CH_3)_3$ | $C(CH_3)_2-CH_2$ | $CH_3$ | $CH(CH_3)$ | $2-CN-C_6H_4$ |
| 1.710. | $C(CH_3)_3$ | $C(CH_3)_2-CH_2$ | $CH_3$ | $CH(CH_3)$ | $3,4-(CH_3O)_2C_6H_3$ |
| 1.711. | $C(CH_3)_3$ | $C(CH_3)_2-CH_2$ | $CH_3$ | $CH(CH_3)$ | $2,4-Cl_2-C_6H_3$ |
| 1.712. | $C(CH_3)_3$ | $C(CH_3)_2-CH_2$ | $CH_3$ | $CH(CH_3)$ | $2,4-(CH_3)_2-C_6H_3$ |
| 1.713. | $C(CH_3)_3$ | $C(CH_3)_2-CH_2$ | $CH_3$ | $CH(CH_3)$ | $3,4-(CH_3)_2C_6H_3$ |
| 1.714. | $C(CH_3)_3$ | $C(CH_3)_2-CH_2$ | $CH_3$ | $CH(CH_3)$ | $4-NO_2-C_6H_4$ |
| 1.715. | $CH(CH_3)_2$ | $CH(CH_3)$ | H | $CH(CH_3)$ | $4-Cl-C_6H_4$ |
| 1.716. | $CH(CH_3)_2$ | $CH(CH_3)$ | H | $CH(CH_3)$ | $4-CH_3-C_6H_4$ |
| 1.717. | $CH(CH_3)_2$ | $CH(CH_3)$ | H | $CH(CH_3)$ | $4-CH_3O-C_6H_4$ |
| 1.718. | $CH(CH_3)_2$ | $CH(CH_3)$ | H | $CH(CH_3)$ | $4-CN-C_6H_4$ |
| 1.719. | $CH(CH_3)_2$ | $CH(CH_3)$ | H | $CH(CH_3)$ | $3-Cl-C_6H_4$ |
| 1.720. | $CH(CH_3)_2$ | $CH(CH_3)$ | H | $CH(CH_3)$ | $3-CH_3-C_6H_4$ |
| 1.721. | $CH(CH_3)_2$ | $CH(CH_3)$ | H | $CH(CH_3)$ | $3-CH_3O-C_6H_4$ |
| 1.722. | $CH(CH_3)_2$ | $CH(CH_3)$ | H | $CH(CH_3)$ | $3-CN-C_6H_4$ |
| 1.723. | $CH(CH_3)_2$ | $CH(CH_3)$ | H | $CH(CH_3)$ | $2-Cl-C_6H_4$ |
| 1.724. | $CH(CH_3)_2$ | $CH(CH_3)$ | H | $CH(CH_3)$ | $2-CH_3-C_6H_4$ |
| 1.725. | $CH(CH_3)_2$ | $CH(CH_3)$ | H | $CH(CH_3)$ | $2-CH_3O-C_6H_4$ |
| 1.726. | $CH(CH_3)_2$ | $CH(CH_3)$ | H | $CH(CH_3)$ | $2-CN-C_6H_4$ |
| 1.727. | $CH(CH_3)_2$ | $CH(CH_3)$ | H | $CH(CH_3)$ | $3,4-(CH_3O)_2C_6H_3$ |
| 1.728. | $CH(CH_3)_2$ | $CH(CH_3)$ | H | $CH(CH_3)$ | $2,4-Cl_2-C_6H_3$ |
| 1.729. | $CH(CH_3)_2$ | $CH(CH_3)$ | H | $CH(CH_3)$ | $2,4-(CH_3)_2-C_6H_3$ |

| Nr. | $R^1$ | A | Y | Z | Ar |
|---|---|---|---|---|---|
| 1.730. | $CH(CH_3)_2$ | $CH(CH_3)$ | H | $CH(CH_3)$ | $3,4-(CH_3)_2C_6H_3$ |
| 1.731. | $CH(CH_3)_2$ | $CH(CH_3)$ | H | $CH(CH_3)$ | $4-NO_2-C_6H_4$ |
| 1.732. | $CH(CH_3)C_2H_5$ | $CH(CH_3)$ | H | $CH(CH_3)$ | $4-Cl-C_6H_4$ |
| 1.733. | $CH(CH_3)C_2H_5$ | $CH(CH_3)$ | H | $CH(CH_3)$ | $4-CH_3-C_6H_4$ |
| 1.734. | $CH(CH_3)C_2H_5$ | $CH(CH_3)$ | H | $CH(CH_3)$ | $4-CH_3O-C_6H_4$ |
| 1.735. | $CH(CH_3)C_2H_5$ | $CH(CH_3)$ | H | $CH(CH_3)$ | $4-CN-C_6H_4$ |
| 1.736. | $CH(CH_3)C_2H_5$ | $CH(CH_3)$ | H | $CH(CH_3)$ | $3-Cl-C_6H_4$ |
| 1.737. | $CH(CH_3)C_2H_5$ | $CH(CH_3)$ | H | $CH(CH_3)$ | $3-CH_3-C_6H_4$ |
| 1.738. | $CH(CH_3)C_2H_5$ | $CH(CH_3)$ | H | $CH(CH_3)$ | $3-CH_3O-C_6H_4$ |
| 1.739. | $CH(CH_3)C_2H_5$ | $CH(CH_3)$ | H | $CH(CH_3)$ | $3-CN-C_6H_4$ |
| 1.740. | $CH(CH_3)C_2H_5$ | $CH(CH_3)$ | H | $CH(CH_3)$ | $2-Cl-C_6H_4$ |
| 1.741. | $CH(CH_3)C_2H_5$ | $CH(CH_3)$ | H | $CH(CH_3)$ | $2-CH_3-C_6H_4$ |
| 1.742. | $CH(CH_3)C_2H_5$ | $CH(CH_3)$ | H | $CH(CH_3)$ | $2-CH_3O-C_6H_4$ |
| 1.743. | $CH(CH_3)C_2H_5$ | $CH(CH_3)$ | H | $CH(CH_3)$ | $2-CN-C_6H_4$ |
| 1.744. | $CH(CH_3)C_2H_5$ | $CH(CH_3)$ | H | $CH(CH_3)$ | $3,4-(CH_3O)_2C_6H_3$ |
| 1.745. | $CH(CH_3)C_2H_5$ | $CH(CH_3)$ | H | $CH(CH_3)$ | $2,4-Cl_2-C_6H_3$ |
| 1.746. | $CH(CH_3)C_2H_5$ | $CH(CH_3)$ | H | $CH(CH_3)$ | $2,4-(CH_3)_2-C_6H_3$ |
| 1.747. | $CH(CH_3)C_2H_5$ | $CH(CH_3)$ | H | $CH(CH_3)$ | $3,4-(CH_3)_2C_6H_3$ |
| 1.748. | $CH(CH_3)C_2H_5$ | $CH(CH_3)$ | H | $CH(CH_3)$ | $4-NO_2-C_6H_4$ |
| 1.749. | $C(CH_3)_3$ | $CH(CH_3)$ | H | $CH(CH_3)$ | $4-Cl-C_6H_4$ |
| 1.750. | $C(CH_3)_3$ | $CH(CH_3)$ | H | $CH(CH_3)$ | $4-CH_3-C_6H_4$ |
| 1.751. | $C(CH_3)_3$ | $CH(CH_3)$ | H | $CH(CH_3)$ | $4-CH_3O-C_6H_4$ |
| 1.752. | $C(CH_3)_3$ | $CH(CH_3)$ | H | $CH(CH_3)$ | $4-CN-C_6H_4$ |
| 1.753. | $C(CH_3)_3$ | $CH(CH_3)$ | H | $CH(CH_3)$ | $3-Cl-C_6H_4$ |
| 1.754. | $C(CH_3)_3$ | $CH(CH_3)$ | H | $CH(CH_3)$ | $3-CH_3-C_6H_4$ |
| 1.755. | $C(CH_3)_3$ | $CH(CH_3)$ | H | $CH(CH_3)$ | $3-CH_3O-C_6H_4$ |
| 1.756. | $C(CH_3)_3$ | $CH(CH_3)$ | H | $CH(CH_3)$ | $3-CN-C_6H_4$ |
| 1.757. | $C(CH_3)_3$ | $CH(CH_3)$ | H | $CH(CH_3)$ | $2-Cl-C_6H_4$ |
| 1.758. | $C(CH_3)_3$ | $CH(CH_3)$ | H | $CH(CH_3)$ | $2-CH_3-C_6H_4$ |
| 1.759. | $C(CH_3)_3$ | $CH(CH_3)$ | H | $CH(CH_3)$ | $2-CH_3O-C_6H_4$ |
| 1.760. | $C(CH_3)_3$ | $CH(CH_3)$ | H | $CH(CH_3)$ | $2-CN-C_6H_4$ |
| 1.761. | $C(CH_3)_3$ | $CH(CH_3)$ | H | $CH(CH_3)$ | $3,4-(CH_3O)_2C_6H_3$ |
| 1.762. | $C(CH_3)_3$ | $CH(CH_3)$ | H | $CH(CH_3)$ | $2,4-Cl_2-C_6H_3$ |
| 1.763. | $C(CH_3)_3$ | $CH(CH_3)$ | H | $CH(CH_3)$ | $2,4-(CH_3)_2-C_6H_3$ |
| 1.764. | $C(CH_3)_3$ | $CH(CH_3)$ | H | $CH(CH_3)$ | $3,4-(CH_3)_2C_6H_3$ |
| 1.765. | $C(CH_3)_3$ | $CH(CH_3)$ | H | $CH(CH_3)$ | $4-NO_2-C_6H_4$ |
| 1.766. | $CH(CH_3)_2$ | $C(CH_3)_2-CH_2$ | H | $CH(CH_3)$ | $4-Cl-C_6H_4$ |
| 1.767. | $CH(CH_3)_2$ | $C(CH_3)_2-CH_2$ | H | $CH(CH_3)$ | $4-CH_3-C_6H_4$ |
| 1.768. | $CH(CH_3)_2$ | $C(CH_3)_2-CH_2$ | H | $CH(CH_3)$ | $4-CH_3O-C_6H_4$ |

| Nr. | $R^1$ | A | Y | Z | Ar |
|---|---|---|---|---|---|
| 1.769. | $CH(CH_3)_2$ | $C(CH_3)_2-CH_2$ | H | $CH(CH_3)$ | $4-CN-C_6H_4$ |
| 1.770. | $CH(CH_3)_2$ | $C(CH_3)_2-CH_2$ | H | $CH(CH_3)$ | $3-Cl-C_6H_4$ |
| 1.771. | $CH(CH_3)_2$ | $C(CH_3)_2-CH_2$ | H | $CH(CH_3)$ | $3-CH_3-C_6H_4$ |
| 1.772. | $CH(CH_3)_2$ | $C(CH_3)_2-CH_2$ | H | $CH(CH_3)$ | $3-CH_3O-C_6H_4$ |
| 1.773. | $CH(CH_3)_2$ | $C(CH_3)_2-CH_2$ | H | $CH(CH_3)$ | $3-CN-C_6H_4$ |
| 1.774. | $CH(CH_3)_2$ | $C(CH_3)_2-CH_2$ | H | $CH(CH_3)$ | $2-Cl-C_6H_4$ |
| 1.775. | $CH(CH_3)_2$ | $C(CH_3)_2-CH_2$ | H | $CH(CH_3)$ | $2-CH_3-C_6H_4$ |
| 1.776. | $CH(CH_3)_2$ | $C(CH_3)_2-CH_2$ | H | $CH(CH_3)$ | $2-CH_3O-C_6H_4$ |
| 1.777. | $CH(CH_3)_2$ | $C(CH_3)_2-CH_2$ | H | $CH(CH_3)$ | $2-CN-C_6H_4$ |
| 1.778. | $CH(CH_3)_2$ | $C(CH_3)_2-CH_2$ | H | $CH(CH_3)$ | $3,4-(CH_3O)_2C_6H_3$ |
| 1.779. | $CH(CH_3)_2$ | $C(CH_3)_2-CH_2$ | H | $CH(CH_3)$ | $2,4-Cl_2-C_6H_3$ |
| 1.780. | $CH(CH_3)_2$ | $C(CH_3)_2-CH_2$ | H | $CH(CH_3)$ | $2,4-(CH_3)_2-C_6H_3$ |
| 1.781. | $CH(CH_3)_2$ | $C(CH_3)_2-CH_2$ | H | $CH(CH_3)$ | $3,4-(CH_3)_2C_6H_3$ |
| 1.782. | $CH(CH_3)_2$ | $C(CH_3)_2-CH_2$ | H | $CH(CH_3)$ | $4-NO_2-C_6H_4$ |
| 1.783. | $CH(CH_3)C_2H_5$ | $C(CH_3)_2-CH_2$ | H | $CH(CH_3)$ | $4-Cl-C_6H_4$ |
| 1.784. | $CH(CH_3)C_2H_5$ | $C(CH_3)_2-CH_2$ | H | $CH(CH_3)$ | $4-CH_3-C_6H_4$ |
| 1.785. | $CH(CH_3)C_2H_5$ | $C(CH_3)_2-CH_2$ | H | $CH(CH_3)$ | $4-CH_3O-C_6H_4$ |
| 1.786. | $CH(CH_3)C_2H_5$ | $C(CH_3)_2-CH_2$ | H | $CH(CH_3)$ | $4-CN-C_6H_4$ |
| 1.787. | $CH(CH_3)C_2H_5$ | $C(CH_3)_2-CH_2$ | H | $CH(CH_3)$ | $3-Cl-C_6H_4$ |
| 1.788. | $CH(CH_3)C_2H_5$ | $C(CH_3)_2-CH_2$ | H | $CH(CH_3)$ | $3-CH_3-C_6H_4$ |
| 1.789. | $CH(CH_3)C_2H_5$ | $C(CH_3)_2-CH_2$ | H | $CH(CH_3)$ | $3-CH_3O-C_6H_4$ |
| 1.790. | $CH(CH_3)C_2H_5$ | $C(CH_3)_2-CH_2$ | H | $CH(CH_3)$ | $3-CN-C_6H_4$ |
| 1.791. | $CH(CH_3)C_2H_5$ | $C(CH_3)_2-CH_2$ | H | $CH(CH_3)$ | $2-Cl-C_6H_4$ |
| 1.792. | $CH(CH_3)C_2H_5$ | $C(CH_3)_2-CH_2$ | H | $CH(CH_3)$ | $2-CH_3-C_6H_4$ |
| 1.793. | $CH(CH_3)C_2H_5$ | $C(CH_3)_2-CH_2$ | H | $CH(CH_3)$ | $2-CH_3O-C_6H_4$ |
| 1.794. | $CH(CH_3)C_2H_5$ | $C(CH_3)_2-CH_2$ | H | $CH(CH_3)$ | $2-CN-C_6H_4$ |
| 1.795. | $CH(CH_3)C_2H_5$ | $C(CH_3)_2-CH_2$ | H | $CH(CH_3)$ | $3,4-(CH_3O)_2C_6H_3$ |
| 1.796. | $CH(CH_3)C_2H_5$ | $C(CH_3)_2-CH_2$ | H | $CH(CH_3)$ | $2,4-Cl_2-C_6H_3$ |
| 1.797. | $CH(CH_3)C_2H_5$ | $C(CH_3)_2-CH_2$ | H | $CH(CH_3)$ | $2,4-(CH_3)_2-C_6H_3$ |
| 1.798. | $CH(CH_3)C_2H_5$ | $C(CH_3)_2-CH_2$ | H | $CH(CH_3)$ | $3,4-(CH_3)_2C_6H_3$ |
| 1.799. | $CH(CH_3)C_2H_5$ | $C(CH_3)_2-CH_2$ | H | $CH(CH_3)$ | $4-NO_2-C_6H_4$ |
| 1.800. | $C(CH_3)_3$ | $C(CH_3)_2-CH_2$ | H | $CH(CH_3)$ | $4-Cl-C_6H_4$ |
| 1.801. | $C(CH_3)_3$ | $C(CH_3)_2-CH_2$ | H | $CH(CH_3)$ | $4-CH_3-C_6H_4$ |
| 1.802. | $C(CH_3)_3$ | $C(CH_3)_2-CH_2$ | H | $CH(CH_3)$ | $4-CH_3O-C_6H_4$ |
| 1.803. | $C(CH_3)_3$ | $C(CH_3)_2-CH_2$ | H | $CH(CH_3)$ | $4-CN-C_6H_4$ |
| 1.804. | $C(CH_3)_3$ | $C(CH_3)_2-CH_2$ | H | $CH(CH_3)$ | $3-Cl-C_6H_4$ |
| 1.805. | $C(CH_3)_3$ | $C(CH_3)_2-CH_2$ | H | $CH(CH_3)$ | $3-CH_3-C_6H_4$ |
| 1.806. | $C(CH_3)_3$ | $C(CH_3)_2-CH_2$ | H | $CH(CH_3)$ | $3-CH_3O-C_6H_4$ |
| 1.807. | $C(CH_3)_3$ | $C(CH_3)_2-CH_2$ | H | $CH(CH_3)$ | $3-CN-C_6H_4$ |

| Nr. | $R^1$ | A | Y | Z | Ar |
|---|---|---|---|---|---|
| 1.808. | $C(CH_3)_3$ | $C(CH_3)_2-CH_2$ | H | $CH(CH_3)$ | $2-Cl-C_6H_4$ |
| 1.809. | $C(CH_3)_3$ | $C(CH_3)_2-CH_2$ | H | $CH(CH_3)$ | $2-CH_3-C_6H_4$ |
| 1.810. | $C(CH_3)_3$ | $C(CH_3)_2-CH_2$ | H | $CH(CH_3)$ | $2-CH_3O-C_6H_4$ |
| 1.811. | $C(CH_3)_3$ | $C(CH_3)_2-CH_2$ | H | $CH(CH_3)$ | $2-CN-C_6H_4$ |
| 1.812. | $C(CH_3)_3$ | $C(CH_3)_2-CH_2$ | H | $CH(CH_3)$ | $3,4-(CH_3O)_2C_6H_3$ |
| 1.813. | $C(CH_3)_3$ | $C(CH_3)_2-CH_2$ | H | $CH(CH_3)$ | $2,4-Cl_2-C_6H_3$ |
| 1.814. | $C(CH_3)_3$ | $C(CH_3)_2-CH_2$ | H | $CH(CH_3)$ | $2,4-(CH_3)_2-C_6H_3$ |
| 1.815. | $C(CH_3)_3$ | $C(CH_3)_2-CH_2$ | H | $CH(CH_3)$ | $3,4-(CH_3)_2C_6H_3$ |
| 1.816. | $C(CH_3)_3$ | $C(CH_3)_2-CH_2$ | H | $CH(CH_3)$ | $4-NO_2-C_6H_4$ |
| 1.817. | $CH(CH_3)_2$ | $CH_2$ | $CH_3$ | $CH(CH_3)$ | $4-Cl-C_6H_4$ |
| 1.818. | $CH(CH_3)_2$ | $CH_2$ | $CH_3$ | $CH(CH_3)$ | $4-CH_3-C_6H_4$ |
| 1.819. | $CH(CH_3)_2$ | $CH_2$ | $CH_3$ | $CH(CH_3)$ | $4-CH_3O-C_6H_4$ |
| 1.820. | $CH(CH_3)_2$ | $CH_2$ | $CH_3$ | $CH(CH_3)$ | $4-CN-C_6H_4$ |
| 1.821. | $CH(CH_3)_2$ | $CH_2$ | $CH_3$ | $CH(CH_3)$ | $3-Cl-C_6H_4$ |
| 1.822. | $CH(CH_3)_2$ | $CH_2$ | $CH_3$ | $CH(CH_3)$ | $3-CH_3-C_6H_4$ |
| 1.823. | $CH(CH_3)_2$ | $CH_2$ | $CH_3$ | $CH(CH_3)$ | $3-CH_3O-C_6H_4$ |
| 1.824. | $CH(CH_3)_2$ | $CH_2$ | $CH_3$ | $CH(CH_3)$ | $3-CN-C_6H_4$ |
| 1.825. | $CH(CH_3)_2$ | $CH_2$ | $CH_3$ | $CH(CH_3)$ | $2-Cl-C_6H_4$ |
| 1.826. | $CH(CH_3)_2$ | $CH_2$ | $CH_3$ | $CH(CH_3)$ | $2-CH_3-C_6H_4$ |
| 1.827. | $CH(CH_3)_2$ | $CH_2$ | $CH_3$ | $CH(CH_3)$ | $2-CH_3O-C_6H_4$ |
| 1.828. | $CH(CH_3)_2$ | $CH_2$ | $CH_3$ | $CH(CH_3)$ | $2-CN-C_6H_4$ |
| 1.829. | $CH(CH_3)_2$ | $CH_2$ | $CH_3$ | $CH(CH_3)$ | $3,4-(CH_3O)_2C_6H_3$ |
| 1.830. | $CH(CH_3)_2$ | $CH_2$ | $CH_3$ | $CH(CH_3)$ | $2,4-Cl_2-C_6H_3$ |
| 1.831. | $CH(CH_3)_2$ | $CH_2$ | $CH_3$ | $CH(CH_3)$ | $2,4-(CH_3)_2-C_6H_3$ |
| 1.832. | $CH(CH_3)_2$ | $CH_2$ | $CH_3$ | $CH(CH_3)$ | $3,4-(CH_3)_2C_6H_3$ |
| 1.833. | $CH(CH_3)_2$ | $CH_2$ | $CH_3$ | $CH(CH_3)$ | $4-NO_2-C_6H_4$ |
| 1.834. | $CH(CH_3)C_2H_5$ | $CH_2$ | $CH_3$ | $CH(CH_3)$ | $4-Cl-C_6H_4$ |
| 1.835. | $CH(CH_3)C_2H_5$ | $CH_2$ | $CH_3$ | $CH(CH_3)$ | $4-CH_3-C_6H_4$ |
| 1.836. | $CH(CH_3)C_2H_5$ | $CH_2$ | $CH_3$ | $CH(CH_3)$ | $4-CH_3O-C_6H_4$ |
| 1.837. | $CH(CH_3)C_2H_5$ | $CH_2$ | $CH_3$ | $CH(CH_3)$ | $4-CN-C_6H_4$ |
| 1.838. | $CH(CH_3)C_2H_5$ | $CH_2$ | $CH_3$ | $CH(CH_3)$ | $3-Cl-C_6H_4$ |
| 1.839. | $CH(CH_3)C_2H_5$ | $CH_2$ | $CH_3$ | $CH(CH_3)$ | $3-CH_3-C_6H_4$ |
| 1.840. | $CH(CH_3)C_2H_5$ | $CH_2$ | $CH_3$ | $CH(CH_3)$ | $3-CH_3O-C_6H_4$ |
| 1.841. | $CH(CH_3)C_2H_5$ | $CH_2$ | $CH_3$ | $CH(CH_3)$ | $3-CN-C_6H_4$ |
| 1.842. | $CH(CH_3)C_2H_5$ | $CH_2$ | $CH_3$ | $CH(CH_3)$ | $2-Cl-C_6H_4$ |
| 1.843. | $CH(CH_3)C_2H_5$ | $CH_2$ | $CH_3$ | $CH(CH_3)$ | $2-CH_3-C_6H_4$ |
| 1.844. | $CH(CH_3)C_2H_5$ | $CH_2$ | $CH_3$ | $CH(CH_3)$ | $2-CH_3O-C_6H_4$ |
| 1.845. | $CH(CH_3)C_2H_5$ | $CH_2$ | $CH_3$ | $CH(CH_3)$ | $2-CN-C_6H_4$ |
| 1.846. | $CH(CH_3)C_2H_5$ | $CH_2$ | $CH_3$ | $CH(CH_3)$ | $3,4-(CH_3O)_2C_6H_3$ |

| Nr. | $R^1$ | A | Y | Z | Ar |
|---|---|---|---|---|---|
| 1.847. | $CH(CH_3)C_2H_5$ | $CH_2$ | $CH_3$ | $CH(CH_3)$ | $2,4-Cl_2-C_6H_3$ |
| 1.848. | $CH(CH_3)C_2H_5$ | $CH_2$ | $CH_3$ | $CH(CH_3)$ | $2,4-(CH_3)_2-C_6H_3$ |
| 1.849. | $CH(CH_3)C_2H_5$ | $CH_2$ | $CH_3$ | $CH(CH_3)$ | $3,4-(CH_3)_2C_6H_3$ |
| 1.850. | $CH(CH_3)C_2H_5$ | $CH_2$ | $CH_3$ | $CH(CH_3)$ | $4-NO_2-C_6H_4$ |
| 1.851. | $C(CH_3)_3$ | $CH_2$ | $CH_3$ | $CH(CH_3)$ | $4-Cl-C_6H_4$ |
| 1.852. | $C(CH_3)_3$ | $CH_2$ | $CH_3$ | $CH(CH_3)$ | $4-CH_3-C_6H_4$ |
| 1.853. | $C(CH_3)_3$ | $CH_2$ | $CH_3$ | $CH(CH_3)$ | $4-CH_3O-C_6H_4$ |
| 1.854. | $C(CH_3)_3$ | $CH_2$ | $CH_3$ | $CH(CH_3)$ | $4-CN-C_6H_4$ |
| 1.855. | $C(CH_3)_3$ | $CH_2$ | $CH_3$ | $CH(CH_3)$ | $3-Cl-C_6H_4$ |
| 1.856. | $C(CH_3)_3$ | $CH_2$ | $CH_3$ | $CH(CH_3)$ | $3-CH_3-C_6H_4$ |
| 1.857. | $C(CH_3)_3$ | $CH_2$ | $CH_3$ | $CH(CH_3)$ | $3-CH_3O-C_6H_4$ |
| 1.858. | $C(CH_3)_3$ | $CH_2$ | $CH_3$ | $CH(CH_3)$ | $3-CN-C_6H_4$ |
| 1.859. | $C(CH_3)_3$ | $CH_2$ | $CH_3$ | $CH(CH_3)$ | $2-Cl-C_6H_4$ |
| 1.860. | $C(CH_3)_3$ | $CH_2$ | $CH_3$ | $CH(CH_3)$ | $2-CH_3-C_6H_4$ |
| 1.861. | $C(CH_3)_3$ | $CH_2$ | $CH_3$ | $CH(CH_3)$ | $2-CH_3O-C_6H_4$ |
| 1.862. | $C(CH_3)_3$ | $CH_2$ | $CH_3$ | $CH(CH_3)$ | $2-CN-C_6H_4$ |
| 1.863. | $C(CH_3)_3$ | $CH_2$ | $CH_3$ | $CH(CH_3)$ | $3,4-(CH_3O)_2C_6H_3$ |
| 1.864. | $C(CH_3)_3$ | $CH_2$ | $CH_3$ | $CH(CH_3)$ | $2,4-Cl_2-C_6H_3$ |
| 1.865. | $C(CH_3)_3$ | $CH_2$ | $CH_3$ | $CH(CH_3)$ | $2,4-(CH_3)_2-C_6H_3$ |
| 1.866. | $C(CH_3)_3$ | $CH_2$ | $CH_3$ | $CH(CH_3)$ | $3,4-(CH_3)_2C_6H_3$ |
| 1.867. | $C(CH_3)_3$ | $CH_2$ | $CH_3$ | $CH(CH_3)$ | $4-NO_2-C_6H_4$ |
| 1.868. | $CH(CH_3)_2$ | $CH_2$ | H | $CH(CH_3)$ | $4-Cl-C_6H_4$ |
| 1.869. | $CH(CH_3)_2$ | $CH_2$ | H | $CH(CH_3)$ | $4-CH_3-C_6H_4$ |
| 1.870. | $CH(CH_3)_2$ | $CH_2$ | H | $CH(CH_3)$ | $4-CH_3O-C_6H_4$ |
| 1.871. | $CH(CH_3)_2$ | $CH_2$ | H | $CH(CH_3)$ | $4-CN-C_6H_4$ |
| 1.872. | $CH(CH_3)_2$ | $CH_2$ | H | $CH(CH_3)$ | $3-Cl-C_6H_4$ |
| 1.873. | $CH(CH_3)_2$ | $CH_2$ | H | $CH(CH_3)$ | $3-CH_3-C_6H_4$ |
| 1.874. | $CH(CH_3)_2$ | $CH_2$ | H | $CH(CH_3)$ | $3-CH_3O-C_6H_4$ |
| 1.875. | $CH(CH_3)_2$ | $CH_2$ | H | $CH(CH_3)$ | $3-CN-C_6H_4$ |
| 1.876. | $CH(CH_3)_2$ | $CH_2$ | H | $CH(CH_3)$ | $2-Cl-C_6H_4$ |
| 1.877. | $CH(CH_3)_2$ | $CH_2$ | H | $CH(CH_3)$ | $2-CH_3-C_6H_4$ |
| 1.878. | $CH(CH_3)_2$ | $CH_2$ | H | $CH(CH_3)$ | $2-CH_3O-C_6H_4$ |
| 1.879. | $CH(CH_3)_2$ | $CH_2$ | H | $CH(CH_3)$ | $2-CN-C_6H_4$ |
| 1.880. | $CH(CH_3)_2$ | $CH_2$ | H | $CH(CH_3)$ | $3,4-(CH_3O)_2C_6H_3$ |
| 1.881. | $CH(CH_3)_2$ | $CH_2$ | H | $CH(CH_3)$ | $2,4-Cl_2-C_6H_3$ |
| 1.882. | $CH(CH_3)_2$ | $CH_2$ | H | $CH(CH_3)$ | $2,4-(CH_3)_2-C_6H_3$ |
| 1.883. | $CH(CH_3)_2$ | $CH_2$ | H | $CH(CH_3)$ | $3,4-(CH_3)_2C_6H_3$ |
| 1.884. | $CH(CH_3)_2$ | $CH_2$ | H | $CH(CH_3)$ | $4-NO_2-C_6H_4$ |
| 1.885. | $CH(CH_3)C_2H_5$ | $CH_2$ | H | $CH(CH_3)$ | $4-Cl-C_6H_4$ |

| Nr. | $R^1$ | A | Y | Z | Ar |
|---|---|---|---|---|---|
| 1.886. | $CH(CH_3)C_2H_5$ | $CH_2$ | H | $CH(CH_3)$ | $4-CH_3-C_6H_4$ |
| 1.887. | $CH(CH_3)C_2H_5$ | $CH_2$ | H | $CH(CH_3)$ | $4-CH_3O-C_6H_4$ |
| 1.888. | $CH(CH_3)C_2H_5$ | $CH_2$ | H | $CH(CH_3)$ | $4-CN-C_6H_4$ |
| 1.889. | $CH(CH_3)C_2H_5$ | $CH_2$ | H | $CH(CH_3)$ | $3-Cl-C_6H_4$ |
| 1.890. | $CH(CH_3)C_2H_5$ | $CH_2$ | H | $CH(CH_3)$ | $3-CH_3-C_6H_4$ |
| 1.891. | $CH(CH_3)C_2H_5$ | $CH_2$ | H | $CH(CH_3)$ | $3-CH_3O-C_6H_4$ |
| 1.892. | $CH(CH_3)C_2H_5$ | $CH_2$ | H | $CH(CH_3)$ | $3-CN-C_6H_4$ |
| 1.893. | $CH(CH_3)C_2H_5$ | $CH_2$ | H | $CH(CH_3)$ | $2-Cl-C_6H_4$ |
| 1.894. | $CH(CH_3)C_2H_5$ | $CH_2$ | H | $CH(CH_3)$ | $2-CH_3-C_6H_4$ |
| 1.895. | $CH(CH_3)C_2H_5$ | $CH_2$ | H | $CH(CH_3)$ | $2-CH_3O-C_6H_4$ |
| 1.896. | $CH(CH_3)C_2H_5$ | $CH_2$ | H | $CH(CH_3)$ | $2-CN-C_6H_4$ |
| 1.897. | $CH(CH_3)C_2H_5$ | $CH_2$ | H | $CH(CH_3)$ | $3,4-(CH_3O)_2C_6H_3$ |
| 1.898. | $CH(CH_3)C_2H_5$ | $CH_2$ | H | $CH(CH_3)$ | $2,4-Cl_2-C_6H_3$ |
| 1.899. | $CH(CH_3)C_2H_5$ | $CH_2$ | H | $CH(CH_3)$ | $2,4-(CH_3)_2-C_6H_3$ |
| 1.900. | $CH(CH_3)C_2H_5$ | $CH_2$ | H | $CH(CH_3)$ | $3,4-(CH_3)_2C_6H_3$ |
| 1.901. | $CH(CH_3)C_2H_5$ | $CH_2$ | H | $CH(CH_3)$ | $4-NO_2-C_6H_4$ |
| 1.902. | $C(CH_3)_3$ | $CH_2$ | H | $CH(CH_3)$ | $4-Cl-C_6H_4$ |
| 1.903. | $C(CH_3)_3$ | $CH_2$ | H | $CH(CH_3)$ | $4-CH_3-C_6H_4$ |
| 1.904. | $C(CH_3)_3$ | $CH_2$ | H | $CH(CH_3)$ | $4-CH_3O-C_6H_4$ |
| 1.905. | $C(CH_3)_3$ | $CH_2$ | H | $CH(CH_3)$ | $4-CN-C_6H_4$ |
| 1.906. | $C(CH_3)_3$ | $CH_2$ | H | $CH(CH_3)$ | $3-Cl-C_6H_4$ |
| 1.907. | $C(CH_3)_3$ | $CH_2$ | H | $CH(CH_3)$ | $3-CH_3-C_6H_4$ |
| 1.908. | $C(CH_3)_3$ | $CH_2$ | H | $CH(CH_3)$ | $3-CH_3O-C_6H_4$ |
| 1.909. | $C(CH_3)_3$ | $CH_2$ | H | $CH(CH_3)$ | $3-CN-C_6H_4$ |
| 1.910. | $C(CH_3)_3$ | $CH_2$ | H | $CH(CH_3)$ | $2-Cl-C_6H_4$ |
| 1.911. | $C(CH_3)_3$ | $CH_2$ | H | $CH(CH_3)$ | $2-CH_3-C_6H_4$ |
| 1.912. | $C(CH_3)_3$ | $CH_2$ | H | $CH(CH_3)$ | $2-CH_3O-C_6H_4$ |
| 1.913. | $C(CH_3)_3$ | $CH_2$ | H | $CH(CH_3)$ | $2-CN-C_6H_4$ |
| 1.914. | $C(CH_3)_3$ | $CH_2$ | H | $CH(CH_3)$ | $3,4-(CH_3O)_2C_6H_3$ |
| 1.915. | $C(CH_3)_3$ | $CH_2$ | H | $CH(CH_3)$ | $2,4-Cl_2-C_6H_3$ |
| 1.916. | $C(CH_3)_3$ | $CH_2$ | H | $CH(CH_3)$ | $2,4-(CH_3)_2-C_6H_3$ |
| 1.917. | $C(CH_3)_3$ | $CH_2$ | H | $CH(CH_3)$ | $3,4-(CH_3)_2C_6H_3$ |
| 1.918. | $C(CH_3)_3$ | $CH_2$ | H | $CH(CH_3)$ | $4-NO_2-C_6H_4$ |
| 1.919. | $CH(CH_3)_2$ | $CH(CH_3)$ | $CH_3$ | $CH(CH_3)CH_2$ | $4-Cl-C_6H_4$ |
| 1.920. | $CH(CH_3)_2$ | $CH(CH_3)$ | $CH_3$ | $CH(CH_3)CH_2$ | $4-CH_3-C_6H_4$ |
| 1.921. | $CH(CH_3)_2$ | $CH(CH_3)$ | $CH_3$ | $CH(CH_3)CH_2$ | $4-CH_3O-C_6H_4$ |
| 1.922. | $CH(CH_3)_2$ | $CH(CH_3)$ | $CH_3$ | $CH(CH_3)CH_2$ | $4-CN-C_6H_4$ |
| 1.923. | $CH(CH_3)_2$ | $CH(CH_3)$ | $CH_3$ | $CH(CH_3)CH_2$ | $3-Cl-C_6H_4$ |
| 1.924. | $CH(CH_3)_2$ | $CH(CH_3)$ | $CH_3$ | $CH(CH_3)CH_2$ | $3-CH_3-C_6H_4$ |

| Nr. | $R^1$ | A | Y | Z | Ar |
|---|---|---|---|---|---|
| 1.925. | $CH(CH_3)_2$ | $CH(CH_3)$ | $CH_3$ | $CH(CH_3)CH_2$ | $3-CH_3O-C_6H_4$ |
| 1.926. | $CH(CH_3)_2$ | $CH(CH_3)$ | $CH_3$ | $CH(CH_3)CH_2$ | $3-CN-C_6H_4$ |
| 1.927. | $CH(CH_3)_2$ | $CH(CH_3)$ | $CH_3$ | $CH(CH_3)CH_2$ | $2-Cl-C_6H_4$ |
| 1.928. | $CH(CH_3)_2$ | $CH(CH_3)$ | $CH_3$ | $CH(CH_3)CH_2$ | $2-CH_3-C_6H_4$ |
| 1.929. | $CH(CH_3)_2$ | $CH(CH_3)$ | $CH_3$ | $CH(CH_3)CH_2$ | $2-CH_3O-C_6H_4$ |
| 1.930. | $CH(CH_3)_2$ | $CH(CH_3)$ | $CH_3$ | $CH(CH_3)CH_2$ | $2-CN-C_6H_4$ |
| 1.931. | $CH(CH_3)_2$ | $CH(CH_3)$ | $CH_3$ | $CH(CH_3)CH_2$ | $3,4-(CH_3O)_2C_6H_3$ |
| 1.932. | $CH(CH_3)_2$ | $CH(CH_3)$ | $CH_3$ | $CH(CH_3)CH_2$ | $2,4-Cl_2-C_6H_3$ |
| 1.933. | $CH(CH_3)_2$ | $CH(CH_3)$ | $CH_3$ | $CH(CH_3)CH_2$ | $2,4-(CH_3)_2-C_6H_3$ |
| 1.934. | $CH(CH_3)_2$ | $CH(CH_3)$ | $CH_3$ | $CH(CH_3)CH_2$ | $3,4-(CH_3)_2C_6H_3$ |
| 1.935. | $CH(CH_3)_2$ | $CH(CH_3)$ | $CH_3$ | $CH(CH_3)CH_2$ | $4-NO_2-C_6H_4$ |
| 1.936. | $CH(CH_3)C_2H_5$ | $CH(CH_3)$ | $CH_3$ | $CH(CH_3)CH_2$ | $4-Cl-C_6H_4$ |
| 1.937. | $CH(CH_3)C_2H_5$ | $CH(CH_3)$ | $CH_3$ | $CH(CH_3)CH_2$ | $4-CH_3-C_6H_4$ |
| 1.938. | $CH(CH_3)C_2H_5$ | $CH(CH_3)$ | $CH_3$ | $CH(CH_3)CH_2$ | $4-CH_3O-C_6H_4$ |
| 1.939. | $CH(CH_3)C_2H_5$ | $CH(CH_3)$ | $CH_3$ | $CH(CH_3)CH_2$ | $4-CN-C_6H_4$ |
| 1.940. | $CH(CH_3)C_2H_5$ | $CH(CH_3)$ | $CH_3$ | $CH(CH_3)CH_2$ | $3-Cl-C_6H_4$ |
| 1.941. | $CH(CH_3)C_2H_5$ | $CH(CH_3)$ | $CH_3$ | $CH(CH_3)CH_2$ | $3-CH_3-C_6H_4$ |
| 1.942. | $CH(CH_3)C_2H_5$ | $CH(CH_3)$ | $CH_3$ | $CH(CH_3)CH_2$ | $3-CH_3O-C_6H_4$ |
| 1.943. | $CH(CH_3)C_2H_5$ | $CH(CH_3)$ | $CH_3$ | $CH(CH_3)CH_2$ | $3-CN-C_6H_4$ |
| 1.944. | $CH(CH_3)C_2H_5$ | $CH(CH_3)$ | $CH_3$ | $CH(CH_3)CH_2$ | $2-Cl-C_6H_4$ |
| 1.945. | $CH(CH_3)C_2H_5$ | $CH(CH_3)$ | $CH_3$ | $CH(CH_3)CH_2$ | $2-CH_3-C_6H_4$ |
| 1.946. | $CH(CH_3)C_2H_5$ | $CH(CH_3)$ | $CH_3$ | $CH(CH_3)CH_2$ | $2-CH_3O-C_6H_4$ |
| 1.947. | $CH(CH_3)C_2H_5$ | $CH(CH_3)$ | $CH_3$ | $CH(CH_3)CH_2$ | $2-CN-C_6H_4$ |
| 1.948. | $CH(CH_3)C_2H_5$ | $CH(CH_3)$ | $CH_3$ | $CH(CH_3)CH_2$ | $3,4-(CH_3O)_2C_6H_3$ |
| 1.949. | $CH(CH_3)C_2H_5$ | $CH(CH_3)$ | $CH_3$ | $CH(CH_3)CH_2$ | $2,4-Cl_2-C_6H_3$ |
| 1.950. | $CH(CH_3)C_2H_5$ | $CH(CH_3)$ | $CH_3$ | $CH(CH_3)CH_2$ | $2,4-(CH_3)_2-C_6H_3$ |
| 1.951. | $CH(CH_3)C_2H_5$ | $CH(CH_3)$ | $CH_3$ | $CH(CH_3)CH_2$ | $3,4-(CH_3)_2C_6H_3$ |
| 1.952. | $CH(CH_3)C_2H_5$ | $CH(CH_3)$ | $CH_3$ | $CH(CH_3)CH_2$ | $4-NO_2-C_6H_4$ |
| 1.953. | $C(CH_3)_3$ | $CH(CH_3)$ | $CH_3$ | $CH(CH_3)CH_2$ | $4-Cl-C_6H_4$ |
| 1.954. | $C(CH_3)_3$ | $CH(CH_3)$ | $CH_3$ | $CH(CH_3)CH_2$ | $4-CH_3-C_6H_4$ |
| 1.955. | $C(CH_3)_3$ | $CH(CH_3)$ | $CH_3$ | $CH(CH_3)CH_2$ | $4-CH_3O-C_6H_4$ |
| 1.956. | $C(CH_3)_3$ | $CH(CH_3)$ | $CH_3$ | $CH(CH_3)CH_2$ | $4-CN-C_6H_4$ |
| 1.957. | $C(CH_3)_3$ | $CH(CH_3)$ | $CH_3$ | $CH(CH_3)CH_2$ | $3-Cl-C_6H_4$ |
| 1.958. | $C(CH_3)_3$ | $CH(CH_3)$ | $CH_3$ | $CH(CH_3)CH_2$ | $3-CH_3-C_6H_4$ |
| 1.959. | $C(CH_3)_3$ | $CH(CH_3)$ | $CH_3$ | $CH(CH_3)CH_2$ | $3-CH_3O-C_6H_4$ |
| 1.960. | $C(CH_3)_3$ | $CH(CH_3)$ | $CH_3$ | $CH(CH_3)CH_2$ | $3-CN-C_6H_4$ |
| 1.961. | $C(CH_3)_3$ | $CH(CH_3)$ | $CH_3$ | $CH(CH_3)CH_2$ | $2-Cl-C_6H_4$ |
| 1.962. | $C(CH_3)_3$ | $CH(CH_3)$ | $CH_3$ | $CH(CH_3)CH_2$ | $2-CH_3-C_6H_4$ |
| 1.963. | $C(CH_3)_3$ | $CH(CH_3)$ | $CH_3$ | $CH(CH_3)CH_2$ | $2-CH_3O-C_6H_4$ |

| Nr. | $R^1$ | A | Y | Z | Ar |
|---|---|---|---|---|---|
| 1.964. | $C(CH_3)_3$ | $CH(CH_3)$ | $CH_3$ | $CH(CH_3)CH_2$ | $2-CN-C_6H_4$ |
| 1.965. | $C(CH_3)_3$ | $CH(CH_3)$ | $CH_3$ | $CH(CH_3)CH_2$ | $3,4-(CH_3O)_2C_6H_3$ |
| 1.966. | $C(CH_3)_3$ | $CH(CH_3)$ | $CH_3$ | $CH(CH_3)CH_2$ | $2,4-Cl_2-C_6H_3$ |
| 1.967. | $C(CH_3)_3$ | $CH(CH_3)$ | $CH_3$ | $CH(CH_3)CH_2$ | $2,4-(CH_3)_2-C_6H_3$ |
| 1.968. | $C(CH_3)_3$ | $CH(CH_3)$ | $CH_3$ | $CH(CH_3)CH_2$ | $3,4-(CH_3)_2C_6H_3$ |
| 1.969. | $C(CH_3)_3$ | $CH(CH_3)$ | $CH_3$ | $CH(CH_3)CH_2$ | $4-NO_2-C_6H_4$ |
| 1.970. | $CH(CH_3)_2$ | $C(CH_3)_2-CH_2$ | $CH_3$ | $CH(CH_3)CH_2$ | $4-Cl-C_6H_4$ |
| 1.971. | $CH(CH_3)_2$ | $C(CH_3)_2-CH_2$ | $CH_3$ | $CH(CH_3)CH_2$ | $4-CH_3-C_6H_4$ |
| 1.972. | $CH(CH_3)_2$ | $C(CH_3)_2-CH_2$ | $CH_3$ | $CH(CH_3)CH_2$ | $4-CH_3O-C_6H_4$ |
| 1.973. | $CH(CH_3)_2$ | $C(CH_3)_2-CH_2$ | $CH_3$ | $CH(CH_3)CH_2$ | $4-CN-C_6H_4$ |
| 1.974. | $CH(CH_3)_2$ | $C(CH_3)_2-CH_2$ | $CH_3$ | $CH(CH_3)CH_2$ | $3-Cl-C_6H_4$ |
| 1.975. | $CH(CH_3)_2$ | $C(CH_3)_2-CH_2$ | $CH_3$ | $CH(CH_3)CH_2$ | $3-CH_3-C_6H_4$ |
| 1.976. | $CH(CH_3)_2$ | $C(CH_3)_2-CH_2$ | $CH_3$ | $CH(CH_3)CH_2$ | $3-CH_3O-C_6H_4$ |
| 1.977. | $CH(CH_3)_2$ | $C(CH_3)_2-CH_2$ | $CH_3$ | $CH(CH_3)CH_2$ | $3-CN-C_6H_4$ |
| 1.978. | $CH(CH_3)_2$ | $C(CH_3)_2-CH_2$ | $CH_3$ | $CH(CH_3)CH_2$ | $2-Cl-C_6H_4$ |
| 1.979. | $CH(CH_3)_2$ | $C(CH_3)_2-CH_2$ | $CH_3$ | $CH(CH_3)CH_2$ | $2-CH_3-C_6H_4$ |
| 1.980. | $CH(CH_3)_2$ | $C(CH_3)_2-CH_2$ | $CH_3$ | $CH(CH_3)CH_2$ | $2-CH_3O-C_6H_4$ |
| 1.981. | $CH(CH_3)_2$ | $C(CH_3)_2-CH_2$ | $CH_3$ | $CH(CH_3)CH_2$ | $2-CN-C_6H_4$ |
| 1.982. | $CH(CH_3)_2$ | $C(CH_3)_2-CH_2$ | $CH_3$ | $CH(CH_3)CH_2$ | $3,4-(CH_3O)_2C_6H_3$ |
| 1.983. | $CH(CH_3)_2$ | $C(CH_3)_2-CH_2$ | $CH_3$ | $CH(CH_3)CH_2$ | $2,4-Cl_2-C_6H_3$ |
| 1.984. | $CH(CH_3)_2$ | $C(CH_3)_2-CH_2$ | $CH_3$ | $CH(CH_3)CH_2$ | $2,4-(CH_3)_2-C_6H_3$ |
| 1.985. | $CH(CH_3)_2$ | $C(CH_3)_2-CH_2$ | $CH_3$ | $CH(CH_3)CH_2$ | $3,4-(CH_3)_2C_6H_3$ |
| 1.986. | $CH(CH_3)_2$ | $C(CH_3)_2-CH_2$ | $CH_3$ | $CH(CH_3)CH_2$ | $4-NO_2-C_6H_4$ |
| 1.987. | $CH(CH_3)C_2H_5$ | $C(CH_3)_2-CH_2$ | $CH_3$ | $CH(CH_3)CH_2$ | $4-Cl-C_6H_4$ |
| 1.988. | $CH(CH_3)C_2H_5$ | $C(CH_3)_2-CH_2$ | $CH_3$ | $CH(CH_3)CH_2$ | $4-CH_3-C_6H_4$ |
| 1.989. | $CH(CH_3)C_2H_5$ | $C(CH_3)_2-CH_2$ | $CH_3$ | $CH(CH_3)CH_2$ | $4-CH_3O-C_6H_4$ |
| 1.990. | $CH(CH_3)C_2H_5$ | $C(CH_3)_2-CH_2$ | $CH_3$ | $CH(CH_3)CH_2$ | $4-CN-C_6H_4$ |
| 1.991. | $CH(CH_3)C_2H_5$ | $C(CH_3)_2-CH_2$ | $CH_3$ | $CH(CH_3)CH_2$ | $3-Cl-C_6H_4$ |
| 1.992. | $CH(CH_3)C_2H_5$ | $C(CH_3)_2-CH_2$ | $CH_3$ | $CH(CH_3)CH_2$ | $3-CH_3-C_6H_4$ |
| 1.993. | $CH(CH_3)C_2H_5$ | $C(CH_3)_2-CH_2$ | $CH_3$ | $CH(CH_3)CH_2$ | $3-CH_3O-C_6H_4$ |
| 1.994. | $CH(CH_3)C_2H_5$ | $C(CH_3)_2-CH_2$ | $CH_3$ | $CH(CH_3)CH_2$ | $3-CN-C_6H_4$ |
| 1.995. | $CH(CH_3)C_2H_5$ | $C(CH_3)_2-CH_2$ | $CH_3$ | $CH(CH_3)CH_2$ | $2-Cl-C_6H_4$ |
| 1.996. | $CH(CH_3)C_2H_5$ | $C(CH_3)_2-CH_2$ | $CH_3$ | $CH(CH_3)CH_2$ | $2-CH_3-C_6H_4$ |
| 1.997. | $CH(CH_3)C_2H_5$ | $C(CH_3)_2-CH_2$ | $CH_3$ | $CH(CH_3)CH_2$ | $2-CH_3O-C_6H_4$ |
| 1.998. | $CH(CH_3)C_2H_5$ | $C(CH_3)_2-CH_2$ | $CH_3$ | $CH(CH_3)CH_2$ | $2-CN-C_6H_4$ |
| 1.999. | $CH(CH_3)C_2H_5$ | $C(CH_3)_2-CH_2$ | $CH_3$ | $CH(CH_3)CH_2$ | $3,4-(CH_3O)_2C_6H_3$ |
| 1.1000. | $CH(CH_3)C_2H_5$ | $C(CH_3)_2-CH_2$ | $CH_3$ | $CH(CH_3)CH_2$ | $2,4-Cl_2-C_6H_3$ |
| 1.1001. | $CH(CH_3)C_2H_5$ | $C(CH_3)_2-CH_2$ | $CH_3$ | $CH(CH_3)CH_2$ | $2,4-(CH_3)_2-C_6H_3$ |
| 1.1002. | $CH(CH_3)C_2H_5$ | $C(CH_3)_2-CH_2$ | $CH_3$ | $CH(CH_3)CH_2$ | $3,4-(CH_3)_2C_6H_3$ |

| Nr. | $R^1$ | A | Y | Z | Ar |
|---|---|---|---|---|---|
| 1.1003. | $CH(CH_3)C_2H_5$ | $C(CH_3)_2-CH_2$ | $CH_3$ | $CH(CH_3)CH_2$ | $4-NO_2-C_6H_4$ |
| 1.1004. | $C(CH_3)_3$ | $C(CH_3)_2-CH_2$ | $CH_3$ | $CH(CH_3)CH_2$ | $4-Cl-C_6H_4$ |
| 1.1005. | $C(CH_3)_3$ | $C(CH_3)_2-CH_2$ | $CH_3$ | $CH(CH_3)CH_2$ | $4-CH_3-C_6H_4$ |
| 1.1006. | $C(CH_3)_3$ | $C(CH_3)_2-CH_2$ | $CH_3$ | $CH(CH_3)CH_2$ | $4-CH_3O-C_6H_4$ |
| 1.1007. | $C(CH_3)_3$ | $C(CH_3)_2-CH_2$ | $CH_3$ | $CH(CH_3)CH_2$ | $4-CN-C_6H_4$ |
| 1.1008. | $C(CH_3)_3$ | $C(CH_3)_2-CH_2$ | $CH_3$ | $CH(CH_3)CH_2$ | $3-Cl-C_6H_4$ |
| 1.1009. | $C(CH_3)_3$ | $C(CH_3)_2-CH_2$ | $CH_3$ | $CH(CH_3)CH_2$ | $3-CH_3-C_6H_4$ |
| 1.1010. | $C(CH_3)_3$ | $C(CH_3)_2-CH_2$ | $CH_3$ | $CH(CH_3)CH_2$ | $3-CH_3O-C_6H_4$ |
| 1.1011. | $C(CH_3)_3$ | $C(CH_3)_2-CH_2$ | $CH_3$ | $CH(CH_3)CH_2$ | $3-CN-C_6H_4$ |
| 1.1012. | $C(CH_3)_3$ | $C(CH_3)_2-CH_2$ | $CH_3$ | $CH(CH_3)CH_2$ | $2-Cl-C_6H_4$ |
| 1.1013. | $C(CH_3)_3$ | $C(CH_3)_2-CH_2$ | $CH_3$ | $CH(CH_3)CH_2$ | $2-CH_3-C_6H_4$ |
| 1.1014. | $C(CH_3)_3$ | $C(CH_3)_2-CH_2$ | $CH_3$ | $CH(CH_3)CH_2$ | $2-CH_3O-C_6H_4$ |
| 1.1015. | $C(CH_3)_3$ | $C(CH_3)_2-CH_2$ | $CH_3$ | $CH(CH_3)CH_2$ | $2-CN-C_6H_4$ |
| 1.1016. | $C(CH_3)_3$ | $C(CH_3)_2-CH_2$ | $CH_3$ | $CH(CH_3)CH_2$ | $3,4-(CH_3O)_2C_6H_3$ |
| 1.1017. | $C(CH_3)_3$ | $C(CH_3)_2-CH_2$ | $CH_3$ | $CH(CH_3)CH_2$ | $2,4-Cl_2-C_6H_3$ |
| 1.1018. | $C(CH_3)_3$ | $C(CH_3)_2-CH_2$ | $CH_3$ | $CH(CH_3)CH_2$ | $2,4-(CH_3)_2-C_6H_3$ |
| 1.1019. | $C(CH_3)_3$ | $C(CH_3)_2-CH_2$ | $CH_3$ | $CH(CH_3)CH_2$ | $3,4-(CH_3)_2C_6H_3$ |
| 1.1020. | $C(CH_3)_3$ | $C(CH_3)_2-CH_2$ | $CH_3$ | $CH(CH_3)CH_2$ | $4-NO_2-C_6H_4$ |
| 1.1021. | $CH(CH_3)_2$ | $CH(CH_3)$ | H | $CH(CH_3)CH_2$ | $4-Cl-C_6H_4$ |
| 1.1022. | $CH(CH_3)_2$ | $CH(CH_3)$ | H | $CH(CH_3)CH_2$ | $4-CH_3-C_6H_4$ |
| 1.1023. | $CH(CH_3)_2$ | $CH(CH_3)$ | H | $CH(CH_3)CH_2$ | $4-CH_3O-C_6H_4$ |
| 1.1024. | $CH(CH_3)_2$ | $CH(CH_3)$ | H | $CH(CH_3)CH_2$ | $4-CN-C_6H_4$ |
| 1.1025. | $CH(CH_3)_2$ | $CH(CH_3)$ | H | $CH(CH_3)CH_2$ | $3-Cl-C_6H_4$ |
| 1.1026. | $CH(CH_3)_2$ | $CH(CH_3)$ | H | $CH(CH_3)CH_2$ | $3-CH_3-C_6H_4$ |
| 1.1027. | $CH(CH_3)_2$ | $CH(CH_3)$ | H | $CH(CH_3)CH_2$ | $3-CH_3O-C_6H_4$ |
| 1.1028. | $CH(CH_3)_2$ | $CH(CH_3)$ | H | $CH(CH_3)CH_2$ | $3-CN-C_6H_4$ |
| 1.1029. | $CH(CH_3)_2$ | $CH(CH_3)$ | H | $CH(CH_3)CH_2$ | $2-Cl-C_6H_4$ |
| 1.1030. | $CH(CH_3)_2$ | $CH(CH_3)$ | H | $CH(CH_3)CH_2$ | $2-CH_3-C_6H_4$ |
| 1.1031. | $CH(CH_3)_2$ | $CH(CH_3)$ | H | $CH(CH_3)CH_2$ | $2-CH_3O-C_6H_4$ |
| 1.1032. | $CH(CH_3)_2$ | $CH(CH_3)$ | H | $CH(CH_3)CH_2$ | $2-CN-C_6H_4$ |
| 1.1033. | $CH(CH_3)_2$ | $CH(CH_3)$ | H | $CH(CH_3)CH_2$ | $3,4-(CH_3O)_2C_6H_3$ |
| 1.1034. | $CH(CH_3)_2$ | $CH(CH_3)$ | H | $CH(CH_3)CH_2$ | $2,4-Cl_2-C_6H_3$ |
| 1.1035. | $CH(CH_3)_2$ | $CH(CH_3)$ | H | $CH(CH_3)CH_2$ | $2,4-(CH_3)_2-C_6H_3$ |
| 1.1036. | $CH(CH_3)_2$ | $CH(CH_3)$ | H | $CH(CH_3)CH_2$ | $3,4-(CH_3)_2C_6H_3$ |
| 1.1037. | $CH(CH_3)_2$ | $CH(CH_3)$ | H | $CH(CH_3)CH_2$ | $4-NO_2-C_6H_4$ |
| 1.1038. | $CH(CH_3)C_2H_5$ | $CH(CH_3)$ | H | $CH(CH_3)CH_2$ | $4-Cl-C_6H_4$ |
| 1.1039. | $CH(CH_3)C_2H_5$ | $CH(CH_3)$ | H | $CH(CH_3)CH_2$ | $4-CH_3-C_6H_4$ |
| 1.1040. | $CH(CH_3)C_2H_5$ | $CH(CH_3)$ | H | $CH(CH_3)CH_2$ | $4-CH_3O-C_6H_4$ |
| 1.1041. | $CH(CH_3)C_2H_5$ | $CH(CH_3)$ | H | $CH(CH_3)CH_2$ | $4-CN-C_6H_4$ |

| Nr. | $R^1$ | A | Y | Z | Ar |
|---|---|---|---|---|---|
| 1.1042. | $CH(CH_3)C_2H_5$ | $CH(CH_3)$ | H | $CH(CH_3)CH_2$ | $3-Cl-C_6H_4$ |
| 1.1043. | $CH(CH_3)C_2H_5$ | $CH(CH_3)$ | H | $CH(CH_3)CH_2$ | $3-CH_3-C_6H_4$ |
| 1.1044. | $CH(CH_3)C_2H_5$ | $CH(CH_3)$ | H | $CH(CH_3)CH_2$ | $3-CH_3O-C_6H_4$ |
| 1.1045. | $CH(CH_3)C_2H_5$ | $CH(CH_3)$ | H | $CH(CH_3)CH_2$ | $3-CN-C_6H_4$ |
| 1.1046. | $CH(CH_3)C_2H_5$ | $CH(CH_3)$ | H | $CH(CH_3)CH_2$ | $2-Cl-C_6H_4$ |
| 1.1047. | $CH(CH_3)C_2H_5$ | $CH(CH_3)$ | H | $CH(CH_3)CH_2$ | $2-CH_3-C_6H_4$ |
| 1.1048. | $CH(CH_3)C_2H_5$ | $CH(CH_3)$ | H | $CH(CH_3)CH_2$ | $2-CH_3O-C_6H_4$ |
| 1.1049. | $CH(CH_3)C_2H_5$ | $CH(CH_3)$ | H | $CH(CH_3)CH_2$ | $2-CN-C_6H_4$ |
| 1.1050. | $CH(CH_3)C_2H_5$ | $CH(CH_3)$ | H | $CH(CH_3)CH_2$ | $3,4-(CH_3O)_2C_6H_3$ |
| 1.1051. | $CH(CH_3)C_2H_5$ | $CH(CH_3)$ | H | $CH(CH_3)CH_2$ | $2,4-Cl_2-C_6H_3$ |
| 1.1052. | $CH(CH_3)C_2H_5$ | $CH(CH_3)$ | H | $CH(CH_3)CH_2$ | $2,4-(CH_3)_2-C_6H_3$ |
| 1.1053. | $CH(CH_3)C_2H_5$ | $CH(CH_3)$ | H | $CH(CH_3)CH_2$ | $3,4-(CH_3)_2C_6H_3$ |
| 1.1054. | $CH(CH_3)C_2H_5$ | $CH(CH_3)$ | H | $CH(CH_3)CH_2$ | $4-NO_2-C_6H_4$ |
| 1.1055. | $C(CH_3)_3$ | $CH(CH_3)$ | H | $CH(CH_3)CH_2$ | $4-Cl-C_6H_4$ |
| 1.1056. | $C(CH_3)_3$ | $CH(CH_3)$ | H | $CH(CH_3)CH_2$ | $4-CH_3-C_6H_4$ |
| 1.1057. | $C(CH_3)_3$ | $CH(CH_3)$ | H | $CH(CH_3)CH_2$ | $4-CH_3O-C_6H_4$ |
| 1.1058. | $C(CH_3)_3$ | $CH(CH_3)$ | H | $CH(CH_3)CH_2$ | $4-CN-C_6H_4$ |
| 1.1059. | $C(CH_3)_3$ | $CH(CH_3)$ | H | $CH(CH_3)CH_2$ | $3-Cl-C_6H_4$ |
| 1.1060. | $C(CH_3)_3$ | $CH(CH_3)$ | H | $CH(CH_3)CH_2$ | $3-CH_3-C_6H_4$ |
| 1.1061. | $C(CH_3)_3$ | $CH(CH_3)$ | H | $CH(CH_3)CH_2$ | $3-CH_3O-C_6H_4$ |
| 1.1062. | $C(CH_3)_3$ | $CH(CH_3)$ | H | $CH(CH_3)CH_2$ | $3-CN-C_6H_4$ |
| 1.1063. | $C(CH_3)_3$ | $CH(CH_3)$ | H | $CH(CH_3)CH_2$ | $2-Cl-C_6H_4$ |
| 1.1064. | $C(CH_3)_3$ | $CH(CH_3)$ | H | $CH(CH_3)CH_2$ | $2-CH_3-C_6H_4$ |
| 1.1065. | $C(CH_3)_3$ | $CH(CH_3)$ | H | $CH(CH_3)CH_2$ | $2-CH_3O-C_6H_4$ |
| 1.1066. | $C(CH_3)_3$ | $CH(CH_3)$ | H | $CH(CH_3)CH_2$ | $2-CN-C_6H_4$ |
| 1.1067. | $C(CH_3)_3$ | $CH(CH_3)$ | H | $CH(CH_3)CH_2$ | $3,4-(CH_3O)_2C_6H_3$ |
| 1.1068. | $C(CH_3)_3$ | $CH(CH_3)$ | H | $CH(CH_3)CH_2$ | $2,4-Cl_2-C_6H_3$ |
| 1.1069. | $C(CH_3)_3$ | $CH(CH_3)$ | H | $CH(CH_3)CH_2$ | $2,4-(CH_3)_2-C_6H_3$ |
| 1.1070. | $C(CH_3)_3$ | $CH(CH_3)$ | H | $CH(CH_3)CH_2$ | $3,4-(CH_3)_2C_6H_3$ |
| 1.1071. | $C(CH_3)_3$ | $CH(CH_3)$ | H | $CH(CH_3)CH_2$ | $4-NO_2-C_6H_4$ |
| 1.1072. | $CH(CH_3)_2$ | $C(CH_3)_2-CH_2$ | H | $CH(CH_3)CH_2$ | $4-Cl-C_6H_4$ |
| 1.1073. | $CH(CH_3)_2$ | $C(CH_3)_2-CH_2$ | H | $CH(CH_3)CH_2$ | $4-CH_3-C_6H_4$ |
| 1.1074. | $CH(CH_3)_2$ | $C(CH_3)_2-CH_2$ | H | $CH(CH_3)CH_2$ | $4-CH_3O-C_6H_4$ |
| 1.1075. | $CH(CH_3)_2$ | $C(CH_3)_2-CH_2$ | H | $CH(CH_3)CH_2$ | $4-CN-C_6H_4$ |
| 1.1076. | $CH(CH_3)_2$ | $C(CH_3)_2-CH_2$ | H | $CH(CH_3)CH_2$ | $3-Cl-C_6H_4$ |
| 1.1077. | $CH(CH_3)_2$ | $C(CH_3)_2-CH_2$ | H | $CH(CH_3)CH_2$ | $3-CH_3-C_6H_4$ |
| 1.1078. | $CH(CH_3)_2$ | $C(CH_3)_2-CH_2$ | H | $CH(CH_3)CH_2$ | $3-CH_3O-C_6H_4$ |
| 1.1079. | $CH(CH_3)_2$ | $C(CH_3)_2-CH_2$ | H | $CH(CH_3)CH_2$ | $3-CN-C_6H_4$ |
| 1.1080. | $CH(CH_3)_2$ | $C(CH_3)_2-CH_2$ | H | $CH(CH_3)CH_2$ | $2-Cl-C_6H_4$ |

| Nr. | $R^1$ | A | Y | Z | Ar |
|---|---|---|---|---|---|
| 1.1081. | $CH(CH_3)_2$ | $C(CH_3)_2-CH_2$ | H | $CH(CH_3)CH_2$ | $2-CH_3-C_6H_4$ |
| 1.1082. | $CH(CH_3)_2$ | $C(CH_3)_2-CH_2$ | H | $CH(CH_3)CH_2$ | $2-CH_3O-C_6H_4$ |
| 1.1083. | $CH(CH_3)_2$ | $C(CH_3)_2-CH_2$ | H | $CH(CH_3)CH_2$ | $2-CN-C_6H_4$ |
| 1.1084. | $CH(CH_3)_2$ | $C(CH_3)_2-CH_2$ | H | $CH(CH_3)CH_2$ | $3,4-(CH_3O)_2C_6H_3$ |
| 1.1085. | $CH(CH_3)_2$ | $C(CH_3)_2-CH_2$ | H | $CH(CH_3)CH_2$ | $2,4-Cl_2-C_6H_3$ |
| 1.1086. | $CH(CH_3)_2$ | $C(CH_3)_2-CH_2$ | H | $CH(CH_3)CH_2$ | $2,4-(CH_3)_2-C_6H_3$ |
| 1.1087. | $CH(CH_3)_2$ | $C(CH_3)_2-CH_2$ | H | $CH(CH_3)CH_2$ | $3,4-(CH_3)_2C_6H_3$ |
| 1.1088. | $CH(CH_3)_2$ | $C(CH_3)_2-CH_2$ | H | $CH(CH_3)CH_2$ | $4-NO_2-C_6H_4$ |
| 1.1089. | $CH(CH_3)C_2H_5$ | $C(CH_3)_2-CH_2$ | H | $CH(CH_3)CH_2$ | $4-Cl-C_6H_4$ |
| 1.1090. | $CH(CH_3)C_2H_5$ | $C(CH_3)_2-CH_2$ | H | $CH(CH_3)CH_2$ | $4-CH_3-C_6H_4$ |
| 1.1091. | $CH(CH_3)C_2H_5$ | $C(CH_3)_2-CH_2$ | H | $CH(CH_3)CH_2$ | $4-CH_3O-C_6H_4$ |
| 1.1092. | $CH(CH_3)C_2H_5$ | $C(CH_3)_2-CH_2$ | H | $CH(CH_3)CH_2$ | $4-CN-C_6H_4$ |
| 1.1093. | $CH(CH_3)C_2H_5$ | $C(CH_3)_2-CH_2$ | H | $CH(CH_3)CH_2$ | $3-Cl-C_6H_4$ |
| 1.1094. | $CH(CH_3)C_2H_5$ | $C(CH_3)_2-CH_2$ | H | $CH(CH_3)CH_2$ | $3-CH_3-C_6H_4$ |
| 1.1095. | $CH(CH_3)C_2H_5$ | $C(CH_3)_2-CH_2$ | H | $CH(CH_3)CH_2$ | $3-CH_3O-C_6H_4$ |
| 1.1096. | $CH(CH_3)C_2H_5$ | $C(CH_3)_2-CH_2$ | H | $CH(CH_3)CH_2$ | $3-CN-C_6H_4$ |
| 1.1097. | $CH(CH_3)C_2H_5$ | $C(CH_3)_2-CH_2$ | H | $CH(CH_3)CH_2$ | $2-Cl-C_6H_4$ |
| 1.1098. | $CH(CH_3)C_2H_5$ | $C(CH_3)_2-CH_2$ | H | $CH(CH_3)CH_2$ | $2-CH_3-C_6H_4$ |
| 1.1099. | $CH(CH_3)C_2H_5$ | $C(CH_3)_2-CH_2$ | H | $CH(CH_3)CH_2$ | $2-CH_3O-C_6H_4$ |
| 1.1100. | $CH(CH_3)C_2H_5$ | $C(CH_3)_2-CH_2$ | H | $CH(CH_3)CH_2$ | $2-CN-C_6H_4$ |
| 1.1101. | $CH(CH_3)C_2H_5$ | $C(CH_3)_2-CH_2$ | H | $CH(CH_3)CH_2$ | $3,4-(CH_3O)_2C_6H_3$ |
| 1.1102. | $CH(CH_3)C_2H_5$ | $C(CH_3)_2-CH_2$ | H | $CH(CH_3)CH_2$ | $2,4-Cl_2-C_6H_3$ |
| 1.1103. | $CH(CH_3)C_2H_5$ | $C(CH_3)_2-CH_2$ | H | $CH(CH_3)CH_2$ | $2,4-(CH_3)_2-C_6H_3$ |
| 1.1104. | $CH(CH_3)C_2H_5$ | $C(CH_3)_2-CH_2$ | H | $CH(CH_3)CH_2$ | $3,4-(CH_3)_2C_6H_3$ |
| 1.1105. | $CH(CH_3)C_2H_5$ | $C(CH_3)_2-CH_2$ | H | $CH(CH_3)CH_2$ | $4-NO_2-C_6H_4$ |
| 1.1106. | $C(CH_3)_3$ | $C(CH_3)_2-CH_2$ | H | $CH(CH_3)CH_2$ | $4-Cl-C_6H_4$ |
| 1.1107. | $C(CH_3)_3$ | $C(CH_3)_2-CH_2$ | H | $CH(CH_3)CH_2$ | $4-CH_3-C_6H_4$ |
| 1.1108. | $C(CH_3)_3$ | $C(CH_3)_2-CH_2$ | H | $CH(CH_3)CH_2$ | $4-CH_3O-C_6H_4$ |
| 1.1109. | $C(CH_3)_3$ | $C(CH_3)_2-CH_2$ | H | $CH(CH_3)CH_2$ | $4-CN-C_6H_4$ |
| 1.1110. | $C(CH_3)_3$ | $C(CH_3)_2-CH_2$ | H | $CH(CH_3)CH_2$ | $3-Cl-C_6H_4$ |
| 1.1111. | $C(CH_3)_3$ | $C(CH_3)_2-CH_2$ | H | $CH(CH_3)CH_2$ | $3-CH_3-C_6H_4$ |
| 1.1112. | $C(CH_3)_3$ | $C(CH_3)_2-CH_2$ | H | $CH(CH_3)CH_2$ | $3-CH_3O-C_6H_4$ |
| 1.1113. | $C(CH_3)_3$ | $C(CH_3)_2-CH_2$ | H | $CH(CH_3)CH_2$ | $3-CN-C_6H_4$ |
| 1.1114. | $C(CH_3)_3$ | $C(CH_3)_2-CH_2$ | H | $CH(CH_3)CH_2$ | $2-Cl-C_6H_4$ |
| 1.1115. | $C(CH_3)_3$ | $C(CH_3)_2-CH_2$ | H | $CH(CH_3)CH_2$ | $2-CH_3-C_6H_4$ |
| 1.1116. | $C(CH_3)_3$ | $C(CH_3)_2-CH_2$ | H | $CH(CH_3)CH_2$ | $2-CH_3O-C_6H_4$ |
| 1.1117. | $C(CH_3)_3$ | $C(CH_3)_2-CH_2$ | H | $CH(CH_3)CH_2$ | $2-CN-C_6H_4$ |
| 1.1118. | $C(CH_3)_3$ | $C(CH_3)_2-CH_2$ | H | $CH(CH_3)CH_2$ | $3,4-(CH_3O)_2C_6H_3$ |
| 1.1119. | $C(CH_3)_3$ | $C(CH_3)_2-CH_2$ | H | $CH(CH_3)CH_2$ | $2,4-Cl_2-C_6H_3$ |

| Nr. | $R^1$ | A | Y | Z | Ar |
|---|---|---|---|---|---|
| 1.1120. | $C(CH_3)_3$ | $C(CH_3)_2-CH_2$ | H | $CH(CH_3)CH_2$ | $2,4-(CH_3)_2-C_6H_3$ |
| 1.1121. | $C(CH_3)_3$ | $C(CH_3)_2-CH_2$ | H | $CH(CH_3)CH_2$ | $3,4-(CH_3)_2C_6H_3$ |
| 1.1122. | $C(CH_3)_3$ | $C(CH_3)_2-CH_2$ | H | $CH(CH_3)CH_2$ | $4-NO_2-C_6H_4$ |
| 1.1123. | $CH(CH_3)_2$ | $CH_2$ | $CH_3$ | $CH(CH_3)CH_2$ | $4-Cl-C_6H_4$ |
| 1.1124. | $CH(CH_3)_2$ | $CH_2$ | $CH_3$ | $CH(CH_3)CH_2$ | $4-CH_3-C_6H_4$ |
| 1.1125. | $CH(CH_3)_2$ | $CH_2$ | $CH_3$ | $CH(CH_3)CH_2$ | $4-CH_3O-C_6H_4$ |
| 1.1126. | $CH(CH_3)_2$ | $CH_2$ | $CH_3$ | $CH(CH_3)CH_2$ | $4-CN-C_6H_4$ |
| 1.1127. | $CH(CH_3)_2$ | $CH_2$ | $CH_3$ | $CH(CH_3)CH_2$ | $3-Cl-C_6H_4$ |
| 1.1128. | $CH(CH_3)_2$ | $CH_2$ | $CH_3$ | $CH(CH_3)CH_2$ | $3-CH_3-C_6H_4$ |
| 1.1129. | $CH(CH_3)_2$ | $CH_2$ | $CH_3$ | $CH(CH_3)CH_2$ | $3-CH_3O-C_6H_4$ |
| 1.1130. | $CH(CH_3)_2$ | $CH_2$ | $CH_3$ | $CH(CH_3)CH_2$ | $3-CN-C_6H_4$ |
| 1.1131. | $CH(CH_3)_2$ | $CH_2$ | $CH_3$ | $CH(CH_3)CH_2$ | $2-Cl-C_6H_4$ |
| 1.1132. | $CH(CH_3)_2$ | $CH_2$ | $CH_3$ | $CH(CH_3)CH_2$ | $2-CH_3-C_6H_4$ |
| 1.1133. | $CH(CH_3)_2$ | $CH_2$ | $CH_3$ | $CH(CH_3)CH_2$ | $2-CH_3O-C_6H_4$ |
| 1.1134. | $CH(CH_3)_2$ | $CH_2$ | $CH_3$ | $CH(CH_3)CH_2$ | $2-CN-C_6H_4$ |
| 1.1135. | $CH(CH_3)_2$ | $CH_2$ | $CH_3$ | $CH(CH_3)CH_2$ | $3,4-(CH_3O)_2C_6H_3$ |
| 1.1136. | $CH(CH_3)_2$ | $CH_2$ | $CH_3$ | $CH(CH_3)CH_2$ | $2,4-Cl_2-C_6H_3$ |
| 1.1137. | $CH(CH_3)_2$ | $CH_2$ | $CH_3$ | $CH(CH_3)CH_2$ | $2,4-(CH_3)_2-C_6H_3$ |
| 1.1138. | $CH(CH_3)_2$ | $CH_2$ | $CH_3$ | $CH(CH_3)CH_2$ | $3,4-(CH_3)_2C_6H_3$ |
| 1.1139. | $CH(CH_3)_2$ | $CH_2$ | $CH_3$ | $CH(CH_3)CH_2$ | $4-NO_2-C_6H_4$ |
| 1.1140. | $CH(CH_3)C_2H_5$ | $CH_2$ | $CH_3$ | $CH(CH_3)CH_2$ | $4-Cl-C_6H_4$ |
| 1.1141. | $CH(CH_3)C_2H_5$ | $CH_2$ | $CH_3$ | $CH(CH_3)CH_2$ | $4-CH_3-C_6H_4$ |
| 1.1142. | $CH(CH_3)C_2H_5$ | $CH_2$ | $CH_3$ | $CH(CH_3)CH_2$ | $4-CH_3O-C_6H_4$ |
| 1.1143. | $CH(CH_3)C_2H_5$ | $CH_2$ | $CH_3$ | $CH(CH_3)CH_2$ | $4-CN-C_6H_4$ |
| 1.1144. | $CH(CH_3)C_2H_5$ | $CH_2$ | $CH_3$ | $CH(CH_3)CH_2$ | $3-Cl-C_6H_4$ |
| 1.1145. | $CH(CH_3)C_2H_5$ | $CH_2$ | $CH_3$ | $CH(CH_3)CH_2$ | $3-CH_3-C_6H_4$ |
| 1.1146. | $CH(CH_3)C_2H_5$ | $CH_2$ | $CH_3$ | $CH(CH_3)CH_2$ | $3-CH_3O-C_6H_4$ |
| 1.1147. | $CH(CH_3)C_2H_5$ | $CH_2$ | $CH_3$ | $CH(CH_3)CH_2$ | $3-CN-C_6H_4$ |
| 1.1148. | $CH(CH_3)C_2H_5$ | $CH_2$ | $CH_3$ | $CH(CH_3)CH_2$ | $2-Cl-C_6H_4$ |
| 1.1149. | $CH(CH_3)C_2H_5$ | $CH_2$ | $CH_3$ | $CH(CH_3)CH_2$ | $2-CH_3-C_6H_4$ |
| 1.1150. | $CH(CH_3)C_2H_5$ | $CH_2$ | $CH_3$ | $CH(CH_3)CH_2$ | $2-CH_3O-C_6H_4$ |
| 1.1151. | $CH(CH_3)C_2H_5$ | $CH_2$ | $CH_3$ | $CH(CH_3)CH_2$ | $2-CN-C_6H_4$ |
| 1.1152. | $CH(CH_3)C_2H_5$ | $CH_2$ | $CH_3$ | $CH(CH_3)CH_2$ | $3,4-(CH_3O)_2C_6H_3$ |
| 1.1153. | $CH(CH_3)C_2H_5$ | $CH_2$ | $CH_3$ | $CH(CH_3)CH_2$ | $2,4-Cl_2-C_6H_3$ |
| 1.1154. | $CH(CH_3)C_2H_5$ | $CH_2$ | $CH_3$ | $CH(CH_3)CH_2$ | $2,4-(CH_3)_2-C_6H_3$ |
| 1.1155. | $CH(CH_3)C_2H_5$ | $CH_2$ | $CH_3$ | $CH(CH_3)CH_2$ | $3,4-(CH_3)_2C_6H_3$ |
| 1.1156. | $CH(CH_3)C_2H_5$ | $CH_2$ | $CH_3$ | $CH(CH_3)CH_2$ | $4-NO_2-C_6H_4$ |
| 1.1157. | $C(CH_3)_3$ | $CH_2$ | $CH_3$ | $CH(CH_3)CH_2$ | $4-Cl-C_6H_4$ |
| 1.1158. | $C(CH_3)_3$ | $CH_2$ | $CH_3$ | $CH(CH_3)CH_2$ | $4-CH_3-C_6H_4$ |

| Nr. | $R^1$ | A | Y | Z | Ar |
|---|---|---|---|---|---|
| 1.1159. | $C(CH_3)_3$ | $CH_2$ | $CH_3$ | $CH(CH_3)CH_2$ | $4-CH_3O-C_6H_4$ |
| 1.1160. | $C(CH_3)_3$ | $CH_2$ | $CH_3$ | $CH(CH_3)CH_2$ | $4-CN-C_6H_4$ |
| 1.1161. | $C(CH_3)_3$ | $CH_2$ | $CH_3$ | $CH(CH_3)CH_2$ | $3-Cl-C_6H_4$ |
| 1.1162. | $C(CH_3)_3$ | $CH_2$ | $CH_3$ | $CH(CH_3)CH_2$ | $3-CH_3-C_6H_4$ |
| 1.1163. | $C(CH_3)_3$ | $CH_2$ | $CH_3$ | $CH(CH_3)CH_2$ | $3-CH_3O-C_6H_4$ |
| 1.1164. | $C(CH_3)_3$ | $CH_2$ | $CH_3$ | $CH(CH_3)CH_2$ | $3-CN-C_6H_4$ |
| 1.1165. | $C(CH_3)_3$ | $CH_2$ | $CH_3$ | $CH(CH_3)CH_2$ | $2-Cl-C_6H_4$ |
| 1.1166. | $C(CH_3)_3$ | $CH_2$ | $CH_3$ | $CH(CH_3)CH_2$ | $2-CH_3-C_6H_4$ |
| 1.1167. | $C(CH_3)_3$ | $CH_2$ | $CH_3$ | $CH(CH_3)CH_2$ | $2-CH_3O-C_6H_4$ |
| 1.1168. | $C(CH_3)_3$ | $CH_2$ | $CH_3$ | $CH(CH_3)CH_2$ | $2-CN-C_6H_4$ |
| 1.1169. | $C(CH_3)_3$ | $CH_2$ | $CH_3$ | $CH(CH_3)CH_2$ | $3,4-(CH_3O)_2C_6H_3$ |
| 1.1170. | $C(CH_3)_3$ | $CH_2$ | $CH_3$ | $CH(CH_3)CH_2$ | $2,4-Cl_2-C_6H_3$ |
| 1.1171. | $C(CH_3)_3$ | $CH_2$ | $CH_3$ | $CH(CH_3)CH_2$ | $2,4-(CH_3)_2-C_6H_3$ |
| 1.1172. | $C(CH_3)_3$ | $CH_2$ | $CH_3$ | $CH(CH_3)CH_2$ | $3,4-(CH_3)_2C_6H_3$ |
| 1.1173. | $C(CH_3)_3$ | $CH_2$ | $CH_3$ | $CH(CH_3)CH_2$ | $4-NO_2-C_6H_4$ |
| 1.1174. | $CH(CH_3)_2$ | $CH_2$ | H | $CH(CH_3)CH_2$ | $4-Cl-C_6H_4$ |
| 1.1175. | $CH(CH_3)_2$ | $CH_2$ | H | $CH(CH_3)CH_2$ | $4-CH_3-C_6H_4$ |
| 1.1176. | $CH(CH_3)_2$ | $CH_2$ | H | $CH(CH_3)CH_2$ | $4-CH_3O-C_6H_4$ |
| 1.1177. | $CH(CH_3)_2$ | $CH_2$ | H | $CH(CH_3)CH_2$ | $4-CN-C_6H_4$ |
| 1.1178. | $CH(CH_3)_2$ | $CH_2$ | H | $CH(CH_3)CH_2$ | $3-Cl-C_6H_4$ |
| 1.1179. | $CH(CH_3)_2$ | $CH_2$ | H | $CH(CH_3)CH_2$ | $3-CH_3-C_6H_4$ |
| 1.1180. | $CH(CH_3)_2$ | $CH_2$ | H | $CH(CH_3)CH_2$ | $3-CH_3O-C_6H_4$ |
| 1.1181. | $CH(CH_3)_2$ | $CH_2$ | H | $CH(CH_3)CH_2$ | $3-CN-C_6H_4$ |
| 1.1182. | $CH(CH_3)_2$ | $CH_2$ | H | $CH(CH_3)CH_2$ | $2-Cl-C_6H_4$ |
| 1.1183. | $CH(CH_3)_2$ | $CH_2$ | H | $CH(CH_3)CH_2$ | $2-CH_3-C_6H_4$ |
| 1.1184. | $CH(CH_3)_2$ | $CH_2$ | H | $CH(CH_3)CH_2$ | $2-CH_3O-C_6H_4$ |
| 1.1185. | $CH(CH_3)_2$ | $CH_2$ | H | $CH(CH_3)CH_2$ | $2-CN-C_6H_4$ |
| 1.1186. | $CH(CH_3)_2$ | $CH_2$ | H | $CH(CH_3)CH_2$ | $3,4-(CH_3O)_2C_6H_3$ |
| 1.1187. | $CH(CH_3)_2$ | $CH_2$ | H | $CH(CH_3)CH_2$ | $2,4-Cl_2-C_6H_3$ |
| 1.1188. | $CH(CH_3)_2$ | $CH_2$ | H | $CH(CH_3)CH_2$ | $2,4-(CH_3)_2-C_6H_3$ |
| 1.1189. | $CH(CH_3)_2$ | $CH_2$ | H | $CH(CH_3)CH_2$ | $3,4-(CH_3)_2C_6H_3$ |
| 1.1190. | $CH(CH_3)_2$ | $CH_2$ | H | $CH(CH_3)CH_2$ | $4-NO_2-C_6H_4$ |
| 1.1191. | $CH(CH_3)C_2H_5$ | $CH_2$ | H | $CH(CH_3)CH_2$ | $4-Cl-C_6H_4$ |
| 1.1192. | $CH(CH_3)C_2H_5$ | $CH_2$ | H | $CH(CH_3)CH_2$ | $4-CH_3-C_6H_4$ |
| 1.1193. | $CH(CH_3)C_2H_5$ | $CH_2$ | H | $CH(CH_3)CH_2$ | $4-CH_3O-C_6H_4$ |
| 1.1194. | $CH(CH_3)C_2H_5$ | $CH_2$ | H | $CH(CH_3)CH_2$ | $4-CN-C_6H_4$ |
| 1.1195. | $CH(CH_3)C_2H_5$ | $CH_2$ | H | $CH(CH_3)CH_2$ | $3-Cl-C_6H_4$ |
| 1.1196. | $CH(CH_3)C_2H_5$ | $CH_2$ | H | $CH(CH_3)CH_2$ | $3-CH_3-C_6H_4$ |
| 1.1197. | $CH(CH_3)C_2H_5$ | $CH_2$ | H | $CH(CH_3)CH_2$ | $3-CH_3O-C_6H_4$ |

| Nr. | $R^1$ | A | Y | Z | Ar |
|---|---|---|---|---|---|
| 1.1198. | $CH(CH_3)C_2H_5$ | $CH_2$ | H | $CH(CH_3)CH_2$ | $3-CN-C_6H_4$ |
| 1.1199. | $CH(CH_3)C_2H_5$ | $CH_2$ | H | $CH(CH_3)CH_2$ | $2-Cl-C_6H_4$ |
| 1.1200. | $CH(CH_3)C_2H_5$ | $CH_2$ | H | $CH(CH_3)CH_2$ | $2-CH_3-C_6H_4$ |
| 1.1201. | $CH(CH_3)C_2H_5$ | $CH_2$ | H | $CH(CH_3)CH_2$ | $2-CH_3O-C_6H_4$ |
| 1.1202. | $CH(CH_3)C_2H_5$ | $CH_2$ | H | $CH(CH_3)CH_2$ | $2-CN-C_6H_4$ |
| 1.1203. | $CH(CH_3)C_2H_5$ | $CH_2$ | H | $CH(CH_3)CH_2$ | $3,4-(CH_3O)_2C_6H_3$ |
| 1.1204. | $CH(CH_3)C_2H_5$ | $CH_2$ | H | $CH(CH_3)CH_2$ | $2,4-Cl_2-C_6H_3$ |
| 1.1205. | $CH(CH_3)C_2H_5$ | $CH_2$ | H | $CH(CH_3)CH_2$ | $2,4-(CH_3)_2-C_6H_3$ |
| 1.1206. | $CH(CH_3)C_2H_5$ | $CH_2$ | H | $CH(CH_3)CH_2$ | $3,4-(CH_3)_2C_6H_3$ |
| 1.1207. | $CH(CH_3)C_2H_5$ | $CH_2$ | H | $CH(CH_3)CH_2$ | $4-NO_2-C_6H_4$ |
| 1.1208. | $C(CH_3)_3$ | $CH_2$ | H | $CH(CH_3)CH_2$ | $4-Cl-C_6H_4$ |
| 1.1209. | $C(CH_3)_3$ | $CH_2$ | H | $CH(CH_3)CH_2$ | $4-CH_3-C_6H_4$ |
| 1.1210. | $C(CH_3)_3$ | $CH_2$ | H | $CH(CH_3)CH_2$ | $4-CH_3O-C_6H_4$ |
| 1.1211. | $C(CH_3)_3$ | $CH_2$ | H | $CH(CH_3)CH_2$ | $4-CN-C_6H_4$ |
| 1.1212. | $C(CH_3)_3$ | $CH_2$ | H | $CH(CH_3)CH_2$ | $3-Cl-C_6H_4$ |
| 1.1213. | $C(CH_3)_3$ | $CH_2$ | H | $CH(CH_3)CH_2$ | $3-CH_3-C_6H_4$ |
| 1.1214. | $C(CH_3)_3$ | $CH_2$ | H | $CH(CH_3)CH_2$ | $3-CH_3O-C_6H_4$ |
| 1.1215. | $C(CH_3)_3$ | $CH_2$ | H | $CH(CH_3)CH_2$ | $3-CN-C_6H_4$ |
| 1.1216. | $C(CH_3)_3$ | $CH_2$ | H | $CH(CH_3)CH_2$ | $2-Cl-C_6H_4$ |
| 1.1217. | $C(CH_3)_3$ | $CH_2$ | H | $CH(CH_3)CH_2$ | $2-CH_3-C_6H_4$ |
| 1.1218. | $C(CH_3)_3$ | $CH_2$ | H | $CH(CH_3)CH_2$ | $2-CH_3O-C_6H_4$ |
| 1.1219. | $C(CH_3)_3$ | $CH_2$ | H | $CH(CH_3)CH_2$ | $2-CN-C_6H_4$ |
| 1.1220. | $C(CH_3)_3$ | $CH_2$ | H | $CH(CH_3)CH_2$ | $3,4-(CH_3O)_2C_6H_3$ |
| 1.1221. | $C(CH_3)_3$ | $CH_2$ | H | $CH(CH_3)CH_2$ | $2,4-Cl_2-C_6H_3$ |
| 1.1222. | $C(CH_3)_3$ | $CH_2$ | H | $CH(CH_3)CH_2$ | $2,4-(CH_3)_2-C_6H_3$ |
| 1.1223. | $C(CH_3)_3$ | $CH_2$ | H | $CH(CH_3)CH_2$ | $3,4-(CH_3)_2C_6H_3$ |
| 1.1224. | $C(CH_3)_3$ | $CH_2$ | H | $CH(CH_3)CH_2$ | $4-NO_2-C_6H_4$ |

Die Verbindungen I eignen sich zur Bekämpfung von Schadpilzen.

Sie können in Abhängigkeit von ihren chemischen und physikalischen Eigenschaften mit üblichen, also dem Fachmann geläufigen, Formulierungshilfsmitteln formuliert werden. Die Produkte dieses Vorgangs werden als "Mittel" bezeichnet.

Geeignete Formulierungshilfsmittel sind z.B. feste oder flüssige Trägerstoffe, oberflächenaktive Mittel und Haftmittel.

Unter flüssigen Trägerstoffen werden flüssige Lösungsmittel wie Wasser und organische Lösungsmittel verstanden, wobei letztere vor allem bei Verwendung von Wasser als Lösungsmittel die Funktion eines Hilfslösungsmittels haben. Als organische Lösungsmittel können verwendet werden: Aromaten wie Xylol, Toluol und Alkylnaphthaline, chlorierte Aromaten oder chlorierte aliphatische Kohlenwasserstoffe wie Chlorbenzole, Chlorethylene und Methylenchlorid, aliphatische Kohlenwasserstoffe wie Cyclohexan und Paraffine, z.B. Mineralölfraktionen, Alkohole wie Butanol, iso-Butanol, Cyclohexanol und Glykol sowie die zugehörigen Ether und Ester, Ketone wie Aceton, Methylethylketon, Methyl-iso-butylketon und Cyclohexanon, aprotisch dipolare Lösungsmittel wie Dimethylformamid, N-Methyl-2-pyrrolidon und Dimethylsulfoxid.

Als feste Trägerstoffe kommen beispielsweise in Betracht: Natürliche Gesteinsmehle und Mineralerden wie Kieselsäuren, Silicate, Kaoline, Tonerden, Bolus, Löß, Talkum, Kreide, Kalkstein, Kalk, Dolomit, Magnesiumoxid, Quarz, Attapulgit, Montmorillonit und Diatomeenerde; synthetische Gesteinsmehle wie hochdisperse Kieselsäure oder Mehle von synthetischem Aluminiumoxid und von synthetischen Silikaten. Insbesondere für Granulate geeignete feste Trägerstoffe sind beispielsweise: Gebrochene und fraktionierte natürliche Gesteine wie Calcit, Marmor, Bims, Sepiolith; synthetische Granulate aus anorganischen und organischen Mehlen; Granulate aus organischem Material wie Sägemehl, Kokosnußschalen, Maiskolben oder Tabakstengel.

Geeignete oberflächenaktive Mittel sind nichtionogene und anionische Emulgiermittel/schaumerzeugende Mittel und Dispergiermittel:

- Fettsäure-Polyoxyethylenester wie Laurylalkohol-Polyoxyethylenetheracetat,
- Alkyl-Polyoxyethylen- oder -Polyoxypropylenether etwa von iso-Tridecylalkohol und Fettalkohol-Polyoxyethylenether,
- Alkylarylalkohol-Polyoxyethylenether wie Octylphenol-Polyoxyethylenether,
- Tributylphenol-Polyoxyethylenether,
- ethoxyliertes iso-Octyl-, Octyl- oder Nonylphenol oder Ricinusöl,
- Sorbitester,
- Arylsulfonsäuren, Alkylsulfonsäuren, Alkylschwefelsäuren,
- Alkali-, Erdalkali- und Ammoniumsalze von Arylsulfonsäuren, z.B. Lignin-, Phenol-, Naphthalin- und Dibutylnaphthalinsulfonsäure, Alkylsulfonsäuren, Alkylarylsulfonsäuren, Alkyl-, Laurylether- und Fettalkoholschwefelsäuren, Fettsäuren, sulfatierten Hexa-, Hepta- und Octadecanolen und Fettalkoholglykolethern,
- Kondensationsprodukte von sulfoniertem Naphthalin und seiner Derivate mit Formaldehyd,
- Kondensationsprodukte von Naphthalinsulfonsäuren mit Phenol und Formaldehyd,
- Eiweißhydrolysate und
- insbesondere als Dispergiermittel: Lignin-Sulfitablaugen und Methylcellulose.

Als Haftmittel eignen sich beispielsweise: Carboxymethylcellulose; natürliche und synthetische pulverige, körnige oder latexförmige Polymere wie Gummiarabicum, Polyvinylalkohol, Polyvinylacetat, natürliche Phospholipide wie Kephaline und Lecithine, synthetische Phospholipide.

Weiterhin können die Mittel einen oder mehrere Vertreter der folgenden Stoffgruppen enthalten: Farbstoffe, andere bekannte Wirkstoffe, Spurennährstoffe und weitere Additive.

Als Farbstoffe kommen z.B. anorganische Pigmente wie Eisenoxid, Titanoxid, Ferrocyanblau, ferner organische Pigmente wie Alizarin-, Azo- und Metallphthalocyaninfarbstoffe in Betracht. Unter anderen bekannten Wirkstoffen sind etwa andere Fungizide sowie Insektizide, Akarizide, Herbizide und Wachstumsregulatoren zu verstehen. Spurennährstoffe sind beispielsweise Salze von Eisen, Mangan, Bor, Kupfer, Kobalt, Molybdän und Zink. Als weitere Additive sind etwa mineralische und vegetabile Öle geeignet.

Die Mittel können darüberhinaus mit sonstigen, praktisch bedeutsamen Mischungspartnern wie Düngemittel oder sonstige fertige wirkstoffhaltige Mittel vermischt sein.

Die Herstellung der Mittel erfolgt in an sich bekannter Weise, nämlich in Abhängigkeit von den chemischen und physikalischen Eigenschaften der eingesetzten Stoffe z.B. durch Mischen, gemeinsames Vermahlen, Aufsprühen, Extrudieren, Granulieren oder Auflösen in Wasser, letzteres ggf. unter Zuhilfenahme eines organischen Lösungsmittels. Pulver, Streu- und Stäubemittel sind z.B. durch Mischen oder gemeinsames Vermahlen der Verbindungen I mit einem festen Trägerstoff erhältlich.

Bei den Mitteln handelt es sich in Abhängigkeit von den eingesetzten Stoffen z.B. um Lösungen, Emulsionen, Suspensionen, Pulver, Schäume, Pasten, Granulate, Aerosole oder Feinstverkapselungen in polymeren Stoffen oder in Saatgut-Hüllmassen.

Zur Anwendung werden die für den Handel in der Regel als Konzentrate vorliegenden Mittel gegebenenfalls wie üblich aufgelöst, verdünnt usw., bei Spritzpulvern, wasserdispergierbaren Granulaten, emulgierbaren Konzentraten, Dispersionen und teilweise auch bei Mikrogranulaten normalerweise unter Verwendung von Wasser. Staubförmige und granulierte Zubereitungen sowie versprühbare Lösungen werden vor der Anwendung meist nicht mehr mit weiteren inerten Stoffen verdünnt.

Die Ausbringung der Mittel erfolgt in an sich bekannter Weise, etwa durch Versprühen, Vernebeln, Verstäuben, Verstreuen oder Gießen. Die Pflanzen werden in der Regel mit den Mitteln besprüht oder bestäubt. Alternativ oder zusätzlich behandelt man die Samen der Pflanzen in an sich bekannter Weise.

Beispiele für solche Zubereitungen sind:

I. eine Lösung aus 90 Gew.-Teilen einer erfindungsgemäßen Verbindung I und 10 Gew.-Teilen N-Methyl-2-pyrrolidon, die zur Anwendung in Form kleinster Tropfen geeignet ist;

II. eine Mischung aus 20 Gew.-Teilen einer erfindungsgemäßen Verbindung I, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen des Calciumsalzes der Dodecylbenzolsulfonsäure, 5 Gew.-Teilen des Anlagerungsproduktes von 40 Mol Ethylenoxid an 1 Mol Ricinusöl: durch feines Verteilen der Lösung in Wasser erhält man eine Dispersion;

III. eine wäßrige Dispersion aus 20 Gew.-Teilen einer erfindungsgemäßen Verbindung I, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen einer erfindungsgemäßen Verbindung I, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen einer erfindungsgemäßen Verbindung I, 3 Gew.-Teilen des Natriumsalzes der Diisobutylnaphtalin-1-sulfonsäure, 10 Gew.-Teilen des Natriumsalzes einer Ligninsulfonsäure aus einer Sulfitablauge und 7 Gew.-Teilen pulverförmigem Kieselsäuregel: durch feines Verteilen der Mischung in Wasser erhält man eine Spritzbrühe;

VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;

VII. eine innige Mischung aus 30 Gew.-Teilen einer erfindungsgemäßen Verbindung I, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen einer erfindungsgemäßen Verbindung I, 10 Gew.-Teilen des Natriumsalzes eines Phenolsulfonsäure-Harnstoff-Formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-Harnstoff-Formaldehyd-Kondensates und 68 Gew.-Teilen eines paraffinischen Mineralöls.

Werden die Verbindungen I als solche appliziert, so kommt es vor allem auf deren feine Verteilung an.
Die Verbindungen I und die erfindungsgemäßen Mittel zeichnen sich durch eine hervorragende Wirksamkeit gegen ein breites Spektrum von Schadpilzen (pflanzenpathogene Pilze), insbesondere aus der Klasse der

- Ascomyceten, - Deuteromyceten und
- Basidiomyceten, - Phycomyceten

aus. Sie sind zum Teil systemisch wirksam und können als Blattund Bodenfungizide eingesetzt werden.
Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen sowie an den Samen dieser Pflanzen.
Die Verbindungen I, ihre Salze und N-Oxide sowie die erfindungsgemäßen Mittel werden angewendet, indem man die Schadpilze, deren Lebensraum oder die vor Pilzbefall zu schützenden Saatgüter, Pflanzen, Flächen, Materialien oder Räume mit einer fungizid wirksamen Menge der Mittel oder der Verbindungen I behandelt. Die Anwendung kann vor oder nach dem Befall durch die Pilze erfolgen.
Speziell eignen sich die erfindungsgemäßen Mittel und die Verbindungen I zur Bekämpfung folgender Pflanzenkrankheiten:
Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Pseudoperonospora-Arten in Hopfen

und Gurken, Alternaria-Arten an Gemüse und Obst.

Die fungiziden Mittel enthalten im allgemeinen zwischen 0,1 und 95, vorzugsweise zwischen 0,5 und 90 Gew. % Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:

Schwefel, Dithiocarbamate und deren Derivate wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)disulfid;

Nitroderivate wie Dinitro-(1-methylheptyl)phenylcrotonat, 2-sec.-Butyl-4,6-dinitrophenyl-3,3-dimethylacrylat, 2-sec.-Butyl-4,6-dinitrophenyl-iso-propylcarbonat, 5-Nitro-iso-phthalsäure-di-isopropylester;

heterocyclische Substanzen wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)phosphinyl]-3-phenyl-1,2,4-triazol, 2,3-Dicyano-1,4-dithioanthrachinon, 2-Thio-1,3-dithiolo-[4,5-b]-chinoxalin, 1-(Butylcarbamoyl)-2-benzimidazol-carbaminsäuremethylester, 2-Methoxycarbonylaminobenzimidazol, 2-(Furyl-(2))benzimidazol, 2-(Thiazolyl-(4))benzimidazol, N-(1,1,2,2-Tetrachlorethylthio)tetrahydrophthalimid, N-Trichlormethylthio-tetrahydrophthalimid, N-Trichlor-methylthiophthalimid,

N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäureanilid, N-Formyl-N-morpholin-2,2,2-trichlor-ethylacetal, Piperazin-1,4-diylbis-(1-(2,2,2-trichlor-ethyl)formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol,1-[2-(2,4-Dichlorphenyl)-4-n-propyl-1,3-dioxolan-2-yl-ethyl]-1H-1,2,4-triazol,N-(n-Propyl)-N-(2,4,6-trichlor-phenoxyethyl)-N'-imidazol-yl-harnstoff, 1-(4-Chlorphenoxy)-3,3-dimethyl-1-(1H-1,2,4-triazol-1-yl)-2-butanon, (2-Chlorphenyl)-(4-chlorphenyl)-5-pyrimidin-methanol, 5-Butyl-2-dimethylamino-4-hydroxy-6-methylpyrimidin, Bis-(p-chlorphenyl)-3-pyridinmethanol, 1,2-Bis-(3-ethoxycarbonyl-2-thioureido)benzol, 1,2-Bis-3-methoxycarbonyl-2-thioureido)benzol, [2-(4-Chlorphenyl)ethyl]-(1,1-dimethylethyl)-1H-1,2,4-triazol-1-ethanol sowie

verschiedene Fungizide wie Dodecylguanidinacetat, 3-[3-(3,5-Dimethyl-2-oxycyclohexyl)-2-hydroxyethyl)]glutarimid, Hexachlorbenzol, DL-Methyl-N-(2,6-dimethyl-phenyl)-N-furoyl(2)alaninat, DL-N-(2,6-Dimethyl-phenyl)-N-(2'-methoxyacetyl)alanin-methylester, N-(2,6-Dimethylphenyl)-N-chloracetyl-D,L-2-aminobutyrolacton, DL-N-(2,6-Dimethylphenyl)-N-(phenylacetyl)alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl] -1,3-oxazolidin-2,4-dion, 3-(3,5-Dichlorhenyl)-1-iso-propylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)pentyl]-1H-1,2,4-triazol, 2,4-Difluor-a-(1H-1,2,4-triazolyl-1-methyl)benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)methylsilyl)-methyl)-1H-1,2,4-triazol.

Strobilurine wie Methyl-E-methoximino-[a-(o-tolyloxy)-o-tolyl]-acetat, Methyl-E-2-{2-[6-(2-cyanophenoxy)pyrimidin-4-yloxy]-phenyl}-3-methoxyacrylat, Methyl-E-methoximino-[a-(2-phenoxyphenyl)]acetamid, Methyl-E-methoximino-[a-(2,5-dimethylphenoxy)-o-tolyl]acetamid.

Anilinopyrimidine wie N-(4,6-Dimethylpyrimidin-2-yl)anilin, N-[4-Methyl-6-(1-propinyl)pyrimidin-2-yl]anilin, N-(4-Methyl-6-cyclopropyl-pyrimidin-2-yl)anilin.

Phenylpyrrole wie 4-(2,2-difluor-1,3-benzodioxol-4-yl)pyrrol-3-carbonitril.
Zimtsäureamide wie 3-(4-Chlorphenyl)-3-(3,4-dimethoxyphenyl)-acrylsäuremorpholid.
(2RS,3SR)-1- [3-(2-Chlorphenyl)-2-[4-fluorphenyl]oxiran-2-ylmethyl]-1H-1,2,4-triazol.

Synthesebeispiele

Die in den nachstehenden Synthesebeispielen wiedergegebenen Vorschriften können unter Abwandlung der Ausgangsverbindungen zur Gewinnung weiterer Vertreter der Verbindungen I benutzt werden. Die physikalischen Daten der demgemäß hergestellten Produkte sind in den jeweils anschließenden Tabellen wiedergegeben.
Die chemischen Verschiebungen (in ppm) der $^1$H-NMR-Spektren wurde gemessen gegen Tetramethylsilan.
N-(iso-Propyloxycarbonyl)-L-valin-2-(3-p-chlorbenzyloxyimino)butylamid (Verbindung 2.4 in Tabelle S1)

1. N-(iso-Propyloxycarbonyl)-L-valin-2-(3-hydroxy)butylamid

Zu der Lösung von 20,9 g (96 mmol) N-(iso-Propyloxycarbonyl)-L-valin und 8,6 g (96 mmol) 2-Amino-3-hydroxybutan in 200 ml Toluol wurden 10,1 g (0,1 mol) Triethylamin zugegeben. Sodann wurden 16,8 g 93 %-iger (96 mmol) Cyanphosphonsäurediethylester bei 10°C zugetropft und über Nacht bei Raumtemperatur gerührt. Nach Zugabe von 150 ml Essigester wurde mit jeweils 300 ml 2n Natronlauge und 10 %-iger Salzsäure gewaschen. Die organische Phase wurde getrocknet, eingeengt und mit den festen Bestandteilen, die sich bei der Extraktion abgeschieden hatten, vereinigt. Man erhielt so 12,9 g N-(iso-Propyloxycarbonyl)-L-valin-2-(3-hydroxy)butylamid.
Fp. 152°C

2. N-(iso-Propyloxycarbonyl)-L-valin-2-(3-oxo)butylamid

Zu einem Zweiphasengemisch aus je 100 ml Dichlormethan und Wasser wurden 5,2 g (20 mmol) N-(iso-Propyloxycarbonyl)-L-valin-2-(3-hydroxy)butylamid, 0,2 g 2,2,6,6-Tetramethyl-piperidin-N-oxid, 0,12 g Kaliumbromid, 0,31 g Natriumdihydrogenphosphat und 0,36 g Dinatriumhydrogenphosphat zugefügt. Sodann wurden binnen 90 Minuten bei Raumtemperatur 11,2 g einer ca. 13 %-igen Natriumhypochloritlösung zugetropft, wobei mittels pH-Elektrode kontrolliert wurde, daß ein pH-Wert von pH 6 bis 7 aufrecht erhalten wurde. Anschließend rührte man noch 1 Stunde nach, ehe nochmals Dichlormethan zugefügt und die organische Phase abgetrennt wurde. Diese wurde zweimal mit Wasser gewaschen, getrocknet und eingeengt. Den resultierenden Rückstand reinigte man schließlich chromatographisch an Kieselgel. Man erhielt nach Einengen der gewünschten Fraktionen 3,7 g N-(iso-Propyloxycarbonyl)-L-valin-2-(3-oxo)butylamid.
Fp. 125 - 134°C
$^1$H-NMR ($CDCl_3$) : 6,6; 5,1 (N-H); 4,9; 4,6; 4,0 (je 1H); 2,1 (3 H); 2,0 (1H); 1,4 (3H); 1,2 und 0,9 (je 6H).

3. N-(iso-Propyloxycarbonyl)-L-valin-2-(3-p-chlorbenzyloxyimino)butylamid (Verbindung 2.4 in Tabelle S1)

Zu der Lösung von 0,4 g Natriumacetat und 0,9 g (6 mmol) p-Chlorbenzyloxyamin in 30 ml Ethanol und 10 ml Wasser, wurden bei 60°C eine Lösung aus 1,1 g (4 mmol) N-(iso-Propyloxycarbonyl)-L-valin-2-(3-oxo)butylamid in 20 ml Ethanol zugetropft. Nach 6stündigem Refluxieren wurde 15 Stunden bei Raumtemperatur gerührt. Man gab noch etwas Wasser hinzu und filtrierte das ausgefallene Produkt ab. Es wurde mit Hexan und Wasser gewaschen und getrocknet. So erhielt man 1,0 g (2,4 mmol) der Titelverbindung.
Fp. 144-147°C

Tabelle S1

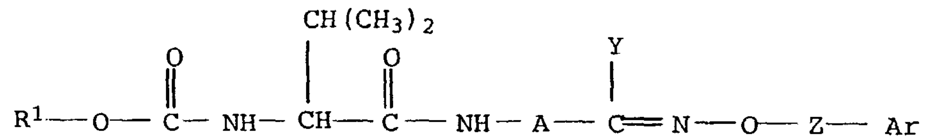

(I')

| | $R^1$ | A | Y | Z | Ar | Fp. [°C] |
|---|---|---|---|---|---|---|
| 2.1 | $CH(CH_3)_2$ | $CH(CH_3)$ | $CH_3$ | $CH_3$ | - | 161 |
| 2.2 | $CH(CH_3)_2$ | $CH(CH_3)$ | $CH_3$ | $CH_2CH_2CH(CH_3)_2$ | - | 120-8 |
| 2.3 | $CH(CH_3)_2$ | $CH(CH_3)$ | $CH_3$ | $CH_2CH_2$ | 4-Cl-$C_6H_4$ | 116 |
| 2.4 | $CH(CH_3)_2$ | $CH(CH_3)$ | $CH_3$ | $CH_2$ | 4-Cl-$C_6H_4$ | 144-7 |
| 2.5 | $CH(CH_3)_2$ | $CH(CH_3)$ | $CH_3$ | $CH_2CH_2CHCH$ | 3,4-$Cl_2$-$C_6H_3$ | 148 |
| 2.6 | $CH(CH_3)_2$ | $CH(CH_3)$ | $CH_3$ | $CH_2CCH$ | - | 143 |
| 2.7 | $CH(CH_3)_2$ | $CH(CH_3)$ | $CH_3$ | $CH_2CH_2O$ | 2-Cl-$C_6H_4$ | 119 |
| 2.8 | $CH(CH_3)_2$ | $CH(CH_3)$ | $CH_3$ | $CH_2$ | β-Naphthyl | 195 |
| 2.9 | $CH(CH_3)_2$ | $CH(CH_3)$ | $CH_3$ | $CH_2$ | 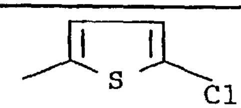 | 164 |

| | $R^1$ | A | Y | Z | Ar | Fp. [°C] |
|---|---|---|---|---|---|---|
| 2.10 | $CH(CH_3)_2$ | $CH(CH_3)$ | $CH_3$ | $CH_2CH_2$ | 3,4-$(MeO)_2$-$C_6H_3$ | 147 |
| 2.11 | $C(CH_3)_3$ | $CH(CH_3)$ | $CH_3$ | $CH_2$ | 4-Cl-$C_6H_4$- | 116 |
| 2.12 | $CH(CH_3)_2$ | $CH(CH_3)$ | $C_6H_5$ | H | - | 50 |
| 2.13 | $CH(CH_3)_2$ | $CH(CH_3)$ | $C_6H_5$ | $CH_3$ | - | 172 |
| 2.14 | $CH(CH_3)_2$ | $CH_2$ | $C_6H_5$ | $CH_2$ | 4-Cl-$C_6H_4$ | 120 |
| 2.15 | $CH(CH_3)_2$ | $CH(CH_3)$ | H | $CH_2$ | 4-Cl-$C_6H_4$ | 116 |
| 2.16 | $CH(CH_3)_2$ | $CH(C_6H_5)$ | H | $CH_3$ | - | 155-8 |
| 2.17 | $CH(CH_3)_2$ | $CH(C_6H_5)$ | H | $CH_2$ | 4-Cl-$C_6H_4$ | 138-42 |
| 2.18 | $CH(CH_3)_2$ | $CH(CH_3)$ | $CH_3$ | $CH_2$ | 2-$CH_3$-$C_6H_4$ | 172-80 |
| 2.19 | $CH(CH_3)_2$ | $CH(CH_3)$ | $CH_3$ | $CH_2$ | $C_6H_5$ | 150-2 |
| 2.20 | $CH(CH_3)_2$ | $CH(CH_3)$ | $CH_3$ | $CH_2$ | 3-MeO-$C_6H_4$ . | 126-8 |
| 2.21 | $CH(CH_3)_2$ | $CH(CH_3)$ | $CH_3$ | $CH_2$ | 3,4-$Cl_2C_6H_3$ | 178-80 |
| 2.22 | $CH(CH_3)_2$ | $CH(CH_3)$ | $CH_3$ | $CH_2$-cyclo$C_6H_{12}$ | - | 134-6 |
| 2.23 | $CH(CH_3)_2$ | $CH(CH_3)$ | $CH_3$ | $CH_2$ | 4-tBu-$C_6H_4$ | 120-3 |
| 2.24 | $CH(CH_3)_2$ | $CH(CH_3)$ | $CH_3$ | $(CH_2)_4$ | 4-F-$C_6H_4$ | 104-10 |
| 2.25 | $CH(CH_3)_2$ | $CH(CH_3)$ | $CH_3$ | $CH_2CH_2CHCH$ | 4-Cl-$C_6H_4$ | 173-5 |
| 2.26 | $CH(CH_3)_2$ | $CH(CH_3)$ | $CH_3$ | $CH_2CH_2O$ | 2-F-$C_6H_4$ | 144-6 |
| 2.27 | $CH(CH_3)_2$ | $CH(CH_3)$ | $CH_3$ | $CH(CH_3)$ | 4-Cl-$C_6H_4$ | 158-60 |
| 2.28 | $CH(CH_3)_2$ | $CH(CH_3)$ | $CH_3$ | CH(cyclo-$C_3H_5$) | 4-Cl-$C_6H_4$ | Öl |
| 2.29 | $CH(CH_3)_2$ | $CH(CH_3)$ | $CH_3$ | $CH_2C(CH_2)CH_3$ | - | 155-8 |

| | $R^1$ | A | Y | Z | Ar | Fp. [°C] |
|---|---|---|---|---|---|---|
| 2.30 | $CH(CH_3)_2$ | $CH(CH_3)$ | H | $CH_2CH_2CHCH$ | 4-Cl-$C_6H_4$ | 104-8 |
| 2.31 | $CH(CH_3)_2$ | $CH(CH_3)$ | H | $CH_2$ | 2-$CH_3$-$C_6H_4$ | 148-50 |
| 2.32 | $CH(CH_3)_2$ | $CH(CH_3)$ | H | $CH_2CH_2O$ | 2-Cl-$C_6H_4$ | 108-12 |
| 2.33 | $CH(CH_3)_2$ | $CH(CH_3)$ | H | $CH_2CH_2O$ | 3,4-$(MeO)_2$-$C_6H_3$ | Öl |
| 2.34 | $CH(CH_3)_2$ | $CH(CH_3)$ | H | $CH_2$ | 3,4-$Cl_2$-$C_6H_3$ | 138-40 |
| 2.35 | $CH(CH_3)_2$ | $CH(CH_3)$ | H | $CH_2CH_2O$ | 2-Cl-$C_6H_4$ | Öl |
| 2.36 | $CH(CH_3)_2$ | $CH(CH_3)$ | $CH_3$ | $CH_2$ | 4-MeO-$C_6H_4$ | 130-2 |
| 2.37 | $CH(CH_3)_2$ | $CH(CH_3)$ | $CH_3$ | $CH_2$ | 4-$CH_3$-$C_6H_4$ | 157-9 |
| 2.38 | $CH(CH_3)_2$ | $CH(CH_3)$ | $CH_3$ | $CH_2$ | 4-CN-$C_6H_4$ | 163-5 |
| 2.39 | $CH(CH_3)_2$ | $CH(CH_3)$ | $CH_3$ | $CH_2$ | 3-CN-$C_6H_4$ | 134 |
| 2.40 | $CH(CH_3)_2$ | $CH(CH_3)$ | $CH_3$ | $CH_2$ | 3-Cl-$C_6H_4$ | 140 |
| 2.41 | $CH(CH_3)_2$ | $CH(CH_3)$ | $CH_3$ | $CH_2$ | 2-Cl-$C_6$-$C_6H_4$ | 171-6 |
| 2.42 | $CH(CH_3)_2$ | $C(CH_3)_2CH_2$ | $CH_3$ | $CH_2$ | 2-Cl-$C_6H_4$ | Öl |
| 2.43 | $CH(CH_3)_2$ | $C(CH_3)_2CH_2$ | $CH_3$ | $CH_2$ | 3-Cl-$C_6H_4$ | Öl |
| 2.44 | $CH(CH_3)_2$ | $C(CH_3)_2CH_2$ | $CH_3$ | $CH_2$ | 4-MeO-$C_6H_4$ | Öl |
| 2.45 | $CH(CH_3)_2$ | $C(CH_3)_2CH_2$ | $CH_3$ | $CH_2$ | 3-CN-$C_6H_4$ | Öl |
| 2.46 | $CH(CH_3)_2$ | $CH(CH_3)$ | H | $CH_2$ | 4-MeO-$C_6H_4$ | 126 |
| 2.47 | $CH(CH_3)_2$ | $CH(CH_3)$ | H | $CH_2$ | 3-CN-$C_6H_4$ | 83-7 |
| 2.48 | $CH(CH_3)_2$ | $C(CH_3)_2CH_2$ | $CH_3$ | $CH_2$ | 4-$CH_3$-$C_6H_4$ | Öl |
| 2.49 | $CH(CH_3)_2$ | $CH(CH_3)$ | H | $CH_2$ | 4-$CH_3$-$C_6H_4$ | 128 |

| | $R^1$ | A | Y | Z | Ar | Fp. [°C] |
|---|---|---|---|---|---|---|
| 2.50 | $CH(CH_3)_2$ | $CH(CH_3)$ | H | $CH_2$ | $4\text{-}CN\text{-}C_6H_4$ | 112-4 |
| 2.51 | $CH(CH_3)_2$ | $CH(CH_3)$ | H | $CH_2$ | $3\text{-}Cl\text{-}C_6H_4$ | 124-7 |
| 2.52 | $CH(CH_3)_2$ | $C(CH_3)_2CH_2$ | $CH_3$ | $CH_2$ | $3\text{-}Cl\text{-}C_6H_4$ | Öl |
| 2.53 | $CH(CH_3)_2$ | $C(CH_3)_2CH_2$ | $CH_3$ | $CH_2$ | $4\text{-}CN\text{-}C_6H_4$ | Öl |

Anwendungsbeispiele

Für die folgenden Versuche zur fungiziden Wirkung der Verbindungen I wurde eine Emulsion verwendet, welche zu 10 Gew.-% aus dem Wirkstoff und zu 90 Gew.-% aus einem Gemisch aus

| | |
|---|---|
| 70 Gew.-% | Cyclohexanol, |
| 20 Gew.-% | Nekanil® LN (Lutensol® AP6, Netzmittel mit Emulgierund Dispergierwirkung auf der Basis ethoxylierter Alkylphenole) und |
| 10 Gew.-% | Uniperol® EL (nicht-ionischer Emulgator auf Basis von ethoxyliertem Ricinusöl) |

bestand. Die gewünschten Wirkstoff-Konzentrationen wurden durch Verdünnen dieser Emulsion mit Wasser eingestellt. Das Ausmaß des Krankheitsbefalls wurde visuell ermittelt.

## Patentansprüche

1. Carbamoylcarbonsäureamidoxime der Formel I

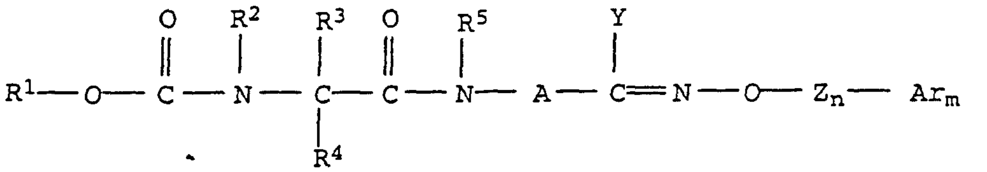

(I)

sowie deren Salze, in denen die Variablen die folgende Bedeutung haben:

$R^1$ $C_1$-$C_8$-Alkyl, $C_2$-$C_8$-Alkenyl oder $C_2$-$C_8$-Alkinyl, wobei diese Reste partiell oder vollständig halogeniert sein und/oder eine, oder unabhängig voneinander zwei oder drei der folgenden Gruppen tragen können: Cyano, $C_1$-$C_4$-Alkoxyalkyl, $C_1$-$C_4$-Halogenalkyl, $C_1$-$C_4$-Alkoxyl $C_1$-$C_4$-Halogenalkoxy, $C_1$-$C_4$-Alkylthio, $C_1$-$C_4$-Alkoxycarbonyl, $C_3$-$C_7$-Cycloalkyl, $C_3$-$C_7$-Cycloalkenyl, Aryl, Aryloxy und Heteroaryl, wobei die cyclischen Reste ihrerseits einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy und Heteroaryl,

$C_3$-$C_7$-Cycloalkyl oder $C_3$-$C_7$-Cycloalkenyl, wobei diese Reste partiell oder vollständig halogeniert sein und/oder eine, oder unabhängig voneinander zwei oder drei der folgenden Gruppen tragen können: Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy und Aryl-($C_1$-$C_4$)-alkyl, wobei die cyclischen Gruppen einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy, einen nicht-aromatischen 4- bis 8-gliedrigen Ring, welcher als Ringglieder neben Kohlenstoff noch eines oder zwei der Heteroatome Sauerstoff, Schwefel und Stickstoff enthalten kann, wobei die Kohlenstoffatome im Ring eine oder zwei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy und wobei das zweite und jedes weitere Stickstoffatom als Heteroatom im Ring Wasserstoff oder eine $C_1$-$C_4$-Alkylgruppe trägt,

Aryl oder Heteroaryl, wobei diese Reste eine, oder unabhängig voneinander zwei oder drei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy und Heteroaryl, in denen die cyclischen Substituenten ihrerseits einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Alkoxycarbonyl,

$W^1W^2C$=N-, wobei $W^1$ $C_1$-$C_8$-Alkyl bedeutet, welches partiell oder vollständig halogeniert sein und/oder eine, oder unabhängig voneinander zwei oder drei der folgenden Gruppen tragen kann: Cyano, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, Aryl, Aryloxy und Heteroaryl, wobei die cyclischen Gruppen ihrerseits einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy,

$C_2$-$C_8$-Alkenyl oder $C_2$-$C_8$-Alkinyl, wobei diese Reste partiell oder vollständig halogeniert sein und/oder eine, oder unabhängig voneinander zwei oder drei der folgenden Gruppen tragen können: Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy, wobei die cyclischen Gruppen ihrerseits einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy,

$C_3$-$C_7$-Cycloalkyl oder $C_3$-$C_7$-Cycloalkenyl, wobei diese Reste eine, oder unabhängig voneinander zwei oder drei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryl-($C_1$-$C_4$)-alkyl, wobei die Gruppen, welche Aryl enthalten, einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy,

Aryl oder Heteroaryl, wobei diese Reste eine, oder unabhängig voneinander zwei oder drei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy und

$W^2$ Wasserstoff oder unabhängig von diesen eine der Gruppen $W^1$;

$R^2$ Wasserstoff oder $C_1$-$C_8$-Alkyl oder $C_3$-$C_7$-Cycloalkyl, welche partiell oder vollständig halogeniert sein können;

$R^3$ $C_1$-$C_8$-Alkyl, $C_3$-$C_7$-Cycloalkyl, wobei diese Reste eine, oder unabhängig voneinander zwei oder drei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Alkoxycarbonyl oder Phenyl-($C_1$-$C_4$)-alkyl, wobei der Phenylrest eine, oder unabhängig voneinander zwei oder drei der folgenden Gruppen tragen kann: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy;

$R^4$ Wasserstoff oder eine der bei $R^3$ genannten Gruppen oder

$R^3$ und $R^4$ gemeinsam mit dem C-Atom an das sie gebunden sind einen 4- bis 8-gliedrigen Ring, welcher als Ringglieder neben Kohlenstoff noch ein oder zwei der Heteroatome Sauerstoff, Schwefel und Stickstoff enthalten kann, wobei die Kohlenstoffatome im Ring eine oder zwei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy und wobei Stickstoff als Heteroatom Wasserstoff oder eine $C_1$-$C_4$-Alkylgruppe trägt;

$R^5$ einen Rest $R^2$;

A $C_1$-$C_8$-Alkylen, wobei diese Reste partiell oder vollständig halogeniert sein und/oder eine, oder unabhängig voneinander zwei oder drei der folgenden Gruppen tragen können: Cyano, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Alkoxycarbonyl, Aryl und Aryloxy, oder
$C_3$-$C_7$-Cycloalkylen, wobei diese Reste partiell oder vollständig halogeniert sein und/oder eine, oder unabhängig voneinander zwei oder drei der folgenden Gruppen tragen können: Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy und Aryl-($C_1$-$C_4$)-alkyl, wobei die cyclischen Gruppen ihrerseits einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy;

Y Wasserstoff,

$C_1$-$C_8$-Alkyl, wobei dieser Rest partiell oder vollständig halogeniert sein und/oder eine, oder unabhängig voneinander zwei oder drei der folgenden Gruppen tragen kann: Cyano, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Alkoxycarbonyl, Aryl und Aryloxy,

$C_3$-$C_7$-Cycloalkyl, wobei dieser Rest partiell oder vollständig halogeniert sein und/oder eine, oder unabhängig voneinander zwei oder drei der folgenden Gruppen tragen kann: Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy und Aryl- ($C_1$-$C_4$)-alkyl, wobei die cyclischen Gruppen ihrerseits einen, oder unabhängig voneinander zwei oder drei der folgenden Substituenten tragen können: Halogen, Cyano, $C_1$-$C_4$-Alkyl, $C_1$-$C_4$-Alkoxyalkyl; $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy oder

Aryl, wobei diese Reste eine, oder unabhängig voneinander zwei oder drei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy;

Z $C_1$-$C_8$-Alkylen, wobei dieser Rest partiell oder vollständig halogeniert sein und/oder eine, oder unabhängig voneinander zwei oder drei der folgenden Gruppen tragen kann: Cyano, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Alkoxycarbonyl, Aryl und Aryloxy,

$C_1$-$C_8$-Alkylenoxy, wobei dieser Rest partiell oder vollständig halogeniert sein und/oder eine, oder unabhängig voneinander zwei oder drei der folgenden Gruppen tragen kann: Cyano, $C_1$-$C_4$-Alkylthio und $C_1$-$C_4$-Alkoxycarbonyl, Aryl und Aryloxy,

$C_3$-$C_7$-Cycloalkylen, wobei dieser Rest partiell oder vollständig halogeniert sein und/oder eine, oder unabhängig voneinander zwei oder drei der folgenden Gruppen tragen kann: Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy und Aryl- ($C_1$-$C_4$)-alkyl, wobei die cyclischen Gruppen ihrerseits einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl und Aryloxy;

n 0, 1 oder 2;

Ar Aryl oder Heteroaryl, wobei diese Reste eine, oder unabhängig voneinander, zwei oder drei der folgenden Gruppen tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aryl, Aryloxy und Heteroaryl, in denen die cyclischen Substituenten ihrerseits einen, oder unabhängig voneinander zwei oder drei der folgenden Substituenten tragen können: Halogen, Cyano, $C_1$-$C_4$-Alkyl, $C_1$-$C_4$-Alkoxyalkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Alkoxycarbonyl:

m die Zahl 1 bedeutet,

**2.** Carbamoylcarbonsäureamidoxime der Formel I gemäß Auspruch 1, wobei die folgenden Reste für sich allein oder in Kombination miteinander folgende Bedeutungen haben:

$R^1$ iso-Propyl, sec-Butyl tert.- Butyl oder Phenyl
$R^2$ wasserstoff;
$R^3$ und $R^4$ einer dieser Reste wasserstoff und der andere iso-Propyl, wobei das kohlenstoffatour, welches die Reste $R^3$ and $R^4$ trägt, die S-konfiguration aufwiest;
$R^5$ Wasserstoff;
A $CH_2$-$CH_2$, $CH_2$, $C(CH_3)_2$, $CH(CH_3)CH_2$, $CH(CH_3)$, wobei der Rest $CH(CH_3)$ racewich ist oder die R-Konfiguration aufweist ;
Y Phenyl, wasserstoff oder Methyl;
Z $CH_2O$, $CH_2$-$CH_2$, $CH(CH_3)$ oder $CH_2$ ;
n 1 oder 2
Ar Phenyl, das durch Chlor, Methyl, Methoxy oder Cyano substituiert sein kann.

**3.** Verfahren zur Herstellung von Carbamoylcarbonsäureamidoximen der Formel I gemäß Anspruch 1 oder 2, **dadurch gekennzeichnet, daß** man eine Carbamoylcarbonsäure der allgemeinen Formel II

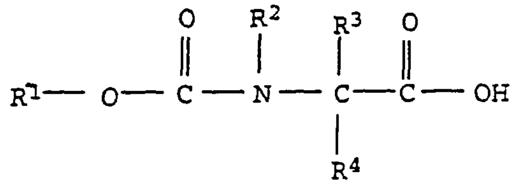 (II)

mit einem Amin der allgemeinen Formel III

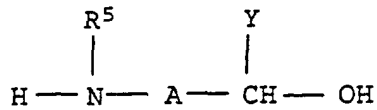 (III)

zum Amid IV

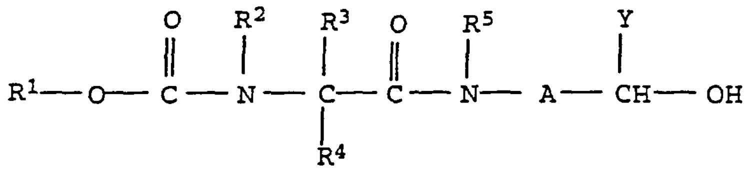 (IV)

umsetzt, IV anschließend zur Carbonylverbindung V

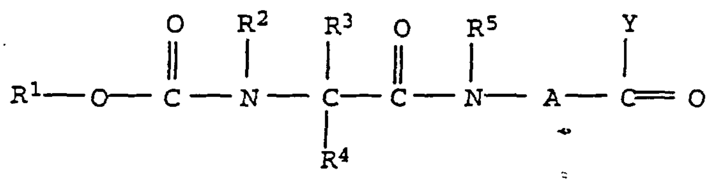 (V)

oxidiert und V mit einem Oxyamin VI

$$H_2N—O—Z_n—Ar_m \qquad (VI)$$

zur Reaktion bringt.

**4.** Verfahren zur Herstellung von Carbamoylcarbonsäureamidoximen der Formel I gemäß Anspruch 1 oder 2, **dadurch gekennzeichnet, daß** man

a) ein Carbamoylcarbonsäureamid der allgemeinen Formel I

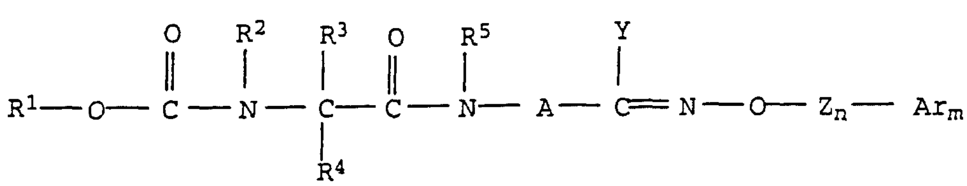 (I)

in der die Gruppe $R^1$-O-(CO) für eine Schutzgruppe steht, die in an sich bekannter Weise abgespalten werden kann, in ein Aminosäureamid VII

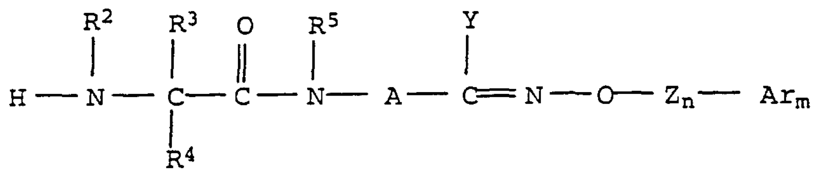

(VII)

überführt und

b) das so erhaltene Aminosäureamid VII mit einem Chlorformyloxim der allgemeinen Formel VIII

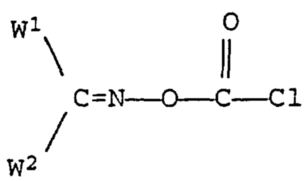

(VIII)

in Gegenwart einer Base umsetzt.

**5.** Zur Bekämpfung von Schadpilzen geeignete Mittel, enthaltend eine fungizid wirksame Menge einer Verbindung der Formel I oder eines ihrer Salze gemäß Anspruch 1 oder 2 und mindestens ein übliches Formulierungshilfsmittel.

**6.** Verfahren zur Bekämpfung von Schadpilzen, **dadurch gekennzeichnet, daß** man die Schadpilze, deren Lebensraum oder die von ihnen freizuhaltenden Pflanzen, Flächen, Materialien oder Räume mit einer wirksamen Menge einer Verbindung der allgemeinen Formel I oder einem ihrer Salze gemäß Anspruch 1 oder 2 oder einem Mittel gemäß Anspruch 5 behandelt.

**7.** Verwendung der Verbindungen der allgemeinen Formel I gemäß Anspruch 1 oder 2 zur Bekämpfung von Schadpilzen.

## Claims

**1.** Carbamoylcarboxamide oximes of the formula I

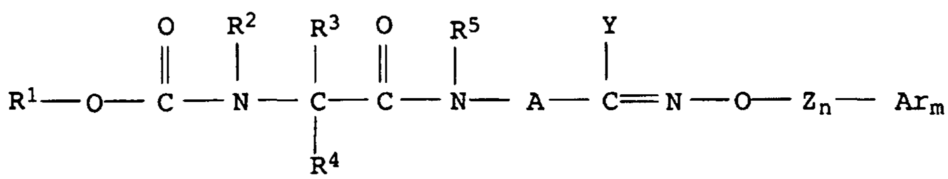

(I)

or salts thereof, where the variables have the following meanings:

$R^1$ is $C_1$-$C_8$-alkyl, $C_2$-$C_8$-alkenyl or $C_2$-$C_8$-alkynyl, it being possible for these radicals to be partially or fully halogenated and/or to carry one or independently two or three of the following groups: cyano, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, $C_3$-$C_7$-cycloalkyl, $C_3$-$C_7$-cycloalkenyl, aryl, aryloxy and hetaryl, it being in turn possible for the cyclic radicals to carry one or independently two or three of the following substituents: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl, aryloxy

and hetaryl,

$C_3$-$C_7$-cycloalkyl or $C_3$-$C_7$-cycloalkenyl, it being possible for these radicals to be partially or fully halogenated and/or to carry one or independently two or three of the following groups: cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl, aryloxy and aryl-($C_1$-$C_4$)-alkyl, it being possible for the cyclic groups to carry one or independently two or three of the following substituents: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl and aryloxy,

a non-aromatic 4- to 8-membered ring which, as ring members, in addition to carbon may contain one or two of the hetero atoms oxygen, sulfur and nitrogen, it being possible for the carbons in the ring to carry one or two of the following groups: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl and aryloxy, the second and any further nitrogen as ring heteroatom carrying hydrogen or a $C_1$-$C_4$-alkyl group,

aryl or hetaryl, it being possible for these radicals to carry one or independently two or three of the following groups: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl, aryloxy and hetaryl, it being in turn possible for the cyclic substituents to carry one or independently two or three of the following substituents: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio and $C_1$-$C_4$-alkoxycarbonyl,

$W^1W^2C$=N-, where $W^1$ is $C_1$-$C_8$-alkyl which may be partially or fully halogenated and/or carry one or independently two or three of the following groups: cyano, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, aryl, aryloxy and hetaryl, it being in turn possible for the cyclic groups to carry one or independently two or three of the following substituents: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl, aryloxy,

$C_2$-$C_8$-alkenyl or $C_2$-$C_8$-alkynyl, it being possible for these radicals to be partially or fully halogenated and/or to carry one or independently two or three of the following groups: cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl and aryloxy, it being in turn possible for the cyclic groups to carry one or independently two or three of the following substituents: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl and aryloxy,

$C_3$-$C_7$-cycloalkyl or $C_3$-$C_7$-cycloalkenyl, it being possible for these radicals to carry one or independently two or three of the following groups: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl and aryl-($C_1$-$C_4$)-alkyl, it being possible for the aryl-containing groups to carry one or independently two or three of the following substituents: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl and aryloxy,

aryl or hetaryl, it being possible for these radicals to carry one or independently two or three of the following groups: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl and aryloxy, and

$W^2$ is hydrogen or independently one of the groups $W^1$;

$R^2$ is hydrogen or is $C_1$-$C_8$-alkyl or $C_3$-$C_7$-cycloalkyl, both of which may be partially or fully halogenated;

$R^3$ is $C_1$-$C_8$-alkyl or $C_3$-$C_7$-cycloalkyl, it being possible for these radicals to carry one or independently two or three of the following groups: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio and $C_1$-$C_4$-alkoxycarbonyl or phenyl-($C_1$-$C_4$)-alkyl, it being possible for the phenyl radical to carry one or independently two or three of the following groups: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl and aryloxy;

$R^4$ is hydrogen or one of the groups listed under $R^3$ or

$R^3$ and $R^4$, together with the carbon that they are attached to, form a 4- to 8-membered ring which, as ring members, in addition to carbon may contain one or two of the hetero atoms oxygen, sulfur and nitrogen, it being possible for the carbons in the ring to carry one or two of the following groups: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl and aryloxy, nitrogen as hetero atom carrying hydrogen or a $C_1$-$C_4$-alkyl group;

$R^5$ is a radical $R^2$;

A is $C_1$-$C_8$-alkylene, it being possible for these radicals to be partially or fully halogenated and/or to carry one or independently two or three of the following groups: cyano, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio and $C_1$-$C_4$-alkoxycarbonyl, aryl and aryloxy, or
$C_3$-$C_7$-cycloalkylene, it being possible for these radicals to be partially or fully halogenated and/or to carry one or independently two or three of the following groups: cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl, aryloxy and aryl-($C_1$-$C_4$)-alkyl, it being in turn possible for the cyclic groups to carry one or independently two or three of the following substituents: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl and aryloxy;

Y is hydrogen,

$C_1$-$C_8$-alkyl, it being possible for this radical to be partially or fully halogenated and/or to carry one or independently two or three of the following groups: cyano, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio and $C_1$-$C_4$-alkoxycarbonyl, aryl and aryloxy,

$C_3$-$C_7$-cycloalkyl, it being possible for this radical to be partially or fully halogenated and/or to carry one or independently two or three of the following groups: cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl, aryloxy and aryl-($C_1$-$C_4$)-alkyl, it being in turn possible for the cyclic groups to carry one or independently two or three of the following substituents: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl and aryloxy or

aryl, it being possible for these radicals to carry one or independently two or three of the following groups: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl and aryloxy;

z is $C_1$-$C_8$-alkylene, it being possible for this radical to be partially or fully halogenated and/or to carry one or independently two or three of the following groups: cyano, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio and $C_1$-$C_4$-alkoxycarbonyl, aryl and aryloxy,

$C_1$-$C_8$-alkyleneoxy, it being possible for this radical to be partially or fully halogenated and/or to carry one or independently two or three of the following groups: cyano, $C_1$-$C_4$-alkylthio and $C_1$-$C_4$-alkoxycarbonyl, aryl and aryloxy,

$C_3$-$C_7$-cycloalkylene, it being possible for this radical to be partially or fully halogenated and/or to carry one or independently two or three of the following groups: cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl, aryloxy and aryl-($C_1$-$C_4$)-alkyl, it being in turn possible for the cyclic groups to carry one or independently two or three of the following substituents: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl and aryloxy;

n is 0, 1 or 2;

Ar is aryl or hetaryl, it being possible for these radicals to carry one or independently two or three of the following groups: halogen, cyano, $C_1$-$C_4$-alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkoxycarbonyl, aryl, aryloxy and hetaryl, it being in turn possible for the cyclic substituents to carry one or independently two or three of the following substituents: halogen, cyano, $C_1$-$C_4$-

alkyl, $C_1$-$C_4$-alkoxyalkyl, $C_1$-$C_4$-haloalkyl, $C_1$-$C_4$-alkoxy, $C_1$-$C_4$-haloalkoxy, $C_1$-$C_4$-alkylthio and $C_1$-$C_4$-alkoxycarbonyl;

m is 1.

**2.** A carbamoylcarboxamide oxime of the formula I as claimed in claim 1, where the following radicals, on their own or in combination with one another, have the following meanings:

$R^1$ is isopropyl, sec-butyl, tert-butyl or phenyl;

$R^2$ is hydrogen;

$R^3$ and $R^4$: one of these radicals is hydrogen and the other is isopropyl, the carbon carrying the radicals $R^3$ and $R^4$ being in the S configuration;

$R^5$ is hydrogen;

A is $CH_2$-$CH_2$, $CH_2$, $C(CH_3)_2$, $CH(CH_3)CH_2$, $CH(CH_3)$, the radical $CH(CH_3)$ being racemic or in the R configuration;

Y is phenyl, hydrogen or methyl;

z is $CH_2O$, $CH_2$-$CH_2$, $CH(CH_3)$ or $CH_2$;

n is 1 or 2;

Ar is phenyl which may be substituted by chlorine, methyl, methoxy or cyano.

**3.** A process for preparing carbamoylcarboxamide oximes of the formula I as claimed in claim 1 or 2, which comprises reacting a carbamoyl carboxylic acid of the general formula II

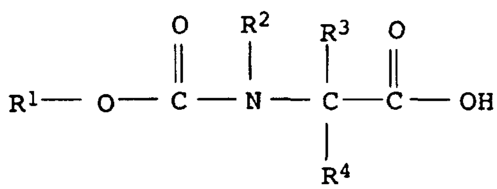

(II)

with an amine of the general formula III

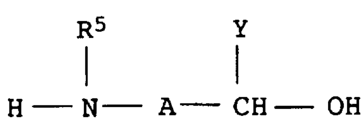

(III)

to give an amide IV

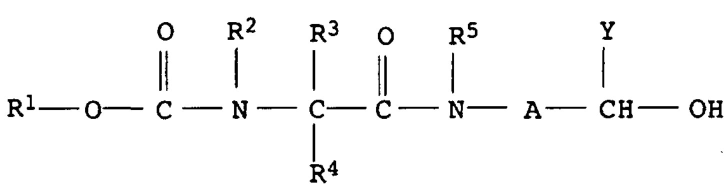

(IV)

and then oxidizing IV to give the carbonyl compound V

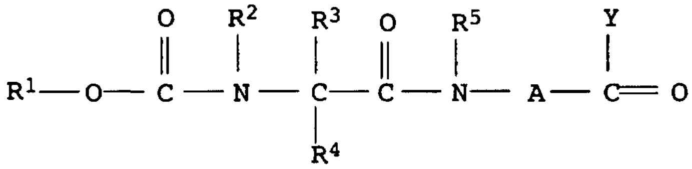 (V)

and reacting V with an oxyamine VI

$$H_2N—O—Z_n— Ar_m \quad (VI).$$

**4.** A process for preparing carbamoylcarboxamide oximes of the formula I as claimed in claim 1 or 2, which comprises:

a) converting a carbamoylcarboxamide of the general formula I

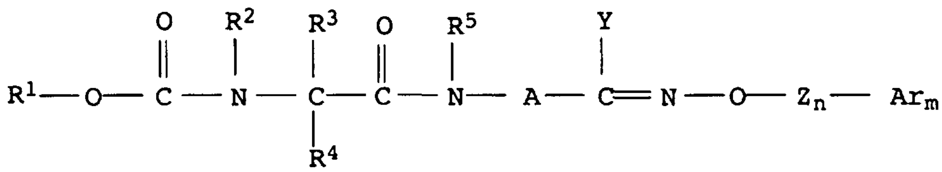 (I)

where the group $R^1$-O-(CO) is a protecting group which can be removed in a conventional manner into an amino acid amide VII

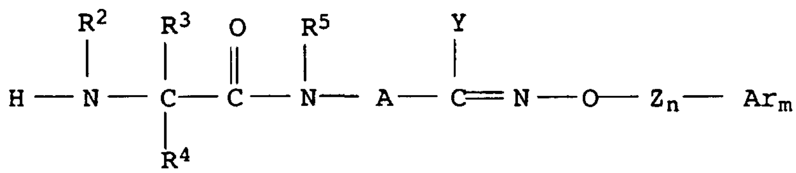 (VII)

and

b) reacting the amino acid amide VII obtained in this manner with a chloroformyl oxime of the general formula VIII

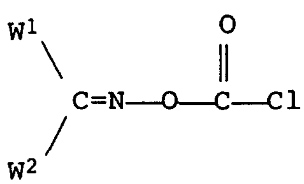 (VIII)

in the presence of a base.

**5.** Compositions suitable for controlling harmful fungi, comprising a fungicidally active amount of a compound of the formula I or a salt thereof as claimed in claim 1 or 2 and at least one customary formulation auxiliary.

**6.** A process for controlling harmful fungi, which comprises treating the harmful fungi, their habitat or the plants, areas,

materials or spaces to be kept free from them with an effective amount of a compound of the general formula I or a salt thereof as claimed in claim 1 or 2 or with a composition as claimed in claim 5.

**7.** The use of the compounds of the general formula I as claimed in claim 1 or 2 for controlling harmful fungi.

## Revendications

**1.** Carbamoylcarboxamidoximes de formule I

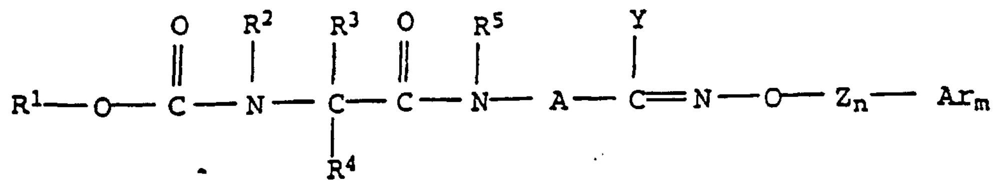

(I)

et leurs sels, les symboles de la formule ayant les significations suivantes :

$R^1$ : un groupe alkyle en C1-C8, alcényle en C2-C8 ou alcynyle en C2-C8, ces groupes pouvant être halogénés en totalité ou en partie et/ou pouvant porter un ou bien, indépendamment les uns des autres, deux ou trois des substituants suivants : cyano, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, cycloalkyle en C3-C7, cycloalcényle en C3-C7, aryle, aryloxy et hétéroaryle, les groupes cycliques pouvant eux-mêmes porter un ou bien, indépendamment les uns des autres, deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle, aryloxy et hétéroaryle,

un groupe cycloalkyle en C3-C7 ou cycloalcényle en C3-C7, ces groupes pouvant être halogénés en totalité ou en partie et/ou pouvant porter un, ou bien, indépendamment les uns des autres, deux ou trois des substituants suivants : cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle, aryloxy et aryl-alkyle en C1-C4, les groupes cycliques pouvant eux-mêmes porter un ou bien, indépendamment les uns des autres, deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle et aryloxy,

un cycle non aromatique de quatre à huit chaînons qui peut contenir en tant que chaînons cycliques, à côté du carbone, un ou deux des hétéroatomes oxygène, soufre et azote, les atomes de carbone du cycle pouvant porter un ou deux des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle et aryloxy, le deuxième atome d'azote et tout autre atome d'azote présent en tant qu'hétéroatome dans le cycle portant de l'hydrogène ou un groupe alkyle en C1-C4,

un groupe aryle ou hétéroaryle, ces groupes pouvant porter un ou, indépendamment les uns des autres, deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle, aryloxy et hétéroaryle dans lesquels les substituants cycliques peuvent eux-mêmes porter un ou bien, indépendamment les uns des autres, deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4 et (alcoxy en C1-C4)carbonyle,

un groupe $W^1W^2C=N-$ dans lequel $W^1$ représente un groupe alkyle en C1-C8 qui peut être halogéné en totalité ou en partie et/ou qui peut porter un ou, indépendamment les uns des autres, deux ou trois des substituants suivants : cyano, (alcoxy en C1-C4)alkyle, halogénoalkyle en C1-C4, alcoxy en C1-C4, halogénoalcoxy en C1-C4, alkylthio en C1-C4, aryle, aryloxy et hétéroaryle, les groupes cycliques pouvant

eux-mêmes porter un ou bien, indépendamment les uns des autres, deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle, aryloxy,

un groupe alcényle en C2-C8 ou alcynyle en C2-C8, ces groupes pouvant être halogénés en totalité ou en partie et/ou pouvant porter un ou bien, indépendamment les uns des autres, deux ou trois des substituants suivants : cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle et aryloxy, les groupes cycliques pouvant eux-mêmes porter un ou, indépendamment les uns des autres, deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle et aryloxy,

un groupe cycloalkyle en C3-C7 ou cycloalcényle en C3-C7, ces groupes pouvant porter un ou, indépendamment les uns des autres, deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle et aryl-alkyle en C1-C4, ceux de ces groupes qui contiennent une partie aryle pouvant porter un, ou, indépendamment les uns des autres, deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle et aryloxy,

un groupe aryle ou hétéroaryle, ces groupes pouvant porter un ou, indépendamment les uns des autres, deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en Cl-C4)carbonyle, aryle et aryloxy et

$W^2$ représente l'hydrogène ou bien, indépendamment de celui-ci, l'un des groupes $W^1$ ;

$R^2$ représente l'hydrogène ou un groupe alkyle en C1-C8 ou cycloalkyle en C3-C7 qui peuvent être halogénés en totalité ou en partie ;

$R^3$ représente un groupe alkyle en C1-C8, cycloalkyle en C3-C7, ces groupes pouvant porter un, ou, indépendamment les uns des autres, deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4 et (alcoxy en C1-C4)carbonyle ou phényl-alkyle en C1-C4, la partie phényle pouvant porter un, ou, indépendamment les uns des autres deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, et aryloxy ;

$R^4$ représente l'hydrogène ou l'un des groupes mentionnés en référence à $R^3$ ou bien

$R^3$ et $R^4$ forment ensemble et avec l'atome auquel ils sont reliés un cycle de quatre à huit chaînons qui peut contenir en tant que chaînons cycliques, à côté du carbone, encore un ou deux des hétéroatomes oxygène, soufre et azote,
les atomes de carbone du cycle pouvant porter un ou deux des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle et aryloxy, l'azote présent en tant qu'hétéroatome portant de l'hydrogène ou un groupe alkyle en C1-C4 ;

$R^5$ représente un groupe $R^2$ ;

A représente un groupe alkylène en C1-C8 qui peut être halogéné en totalité ou en partie et/ou peut porter un, ou, indépendamment les uns des autres, deux ou trois des substituants suivants : cyano, (alcoxy en C1-C4) alkyle, halogénoalkyle en C1-C4, alcoxy en C1-C4, halogénoalcoxy en C1-C4, alkylthio en C1-C4 et (alcoxy en C1-C4)carbonyle, aryle et aryloxy, ou bien
un groupe cycloalkylène en C3-C7, ce groupe pouvant être halogéné en totalité ou en partie et/ou pouvant porter un, ou, indépendamment les uns des autres, deux ou trois des substituants suivants : cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle, aryloxy et aryl-alkyle en C1-C4, les groupes cycliques pouvant eux-mêmes porter un ou, indépendamment les uns des autres, deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle et aryloxy ;

Y représente l'hydrogène,

un groupe alkyle en C1-C8 qui peut être halogéné en totalité ou en partie et/ou peut porter un ou, indépendamment les uns des autres, deux ou trois des substituants suivants : cyano, (alcoxy en C1-C4)alkyle, halogénoalkyle en C1-C4, alcoxy en C1-C4, halogénoalcoxy en C1-C4, alkylthio en C1-C4 et (alcoxy en C1-C4)carbonyle, aryle et aryloxy,

un groupe cycloalkyle en C3-C7, ce groupe pouvant être halogéné en totalité ou en partie et/ou pouvant porter un, ou, indépendamment les uns des autres, deux ou trois des substituants suivants : cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle, aryloxy et aryl-alkyle en C1-C4, les groupes cycliques pouvant eux-mêmes porter un, ou, indépendamment les uns des autres, deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle et aryloxy, ou bien

un groupe aryle, ce groupe pouvant porter un ou, indépendamment les uns des autres, deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle et aryloxy ;

Z représente un groupe alkylène en C1-C8 qui peut être halogéné en totalité ou en partie et/ou peut porter un, ou, indépendamment les uns des autres, deux ou trois des substituants suivants : cyano, (alcoxy en C1-C4) alkyle, halogénoalkyle en C1-C4, alcoxy en C1-C4, halogénoalcoxy en C1-C4, alkylthio en C1-C4 et (alcoxy en C1-C4)carbonyle, aryle et aryloxy,

un groupe alkylénoxy en C1-C8 qui peut être halogéné en totalité ou en partie et/ou peut porter un, ou, indépendamment les uns des autres, deux ou trois des substituants suivants : cyano, alkylthio en C1-C4 et (alcoxy en C1-C4)carbonyle, aryle et aryloxy,

un groupe cycloalkylène en C3-C7 qui peut être halogéné en totalité ou en partie et/ou peut porter un ou, indépendamment les uns des autres, deux ou trois des substituants suivants : cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle, aryloxy et aryl-alkyle en C1-C4, les groupes cycliques pouvant eux-mêmes porter un, ou, indépendamment les uns des autres, deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle et aryloxy ;

n est égal à 0, 1 ou 2 ;

Ar représente un groupe aryle ou hétéroaryle, chacun d'eux pouvant porter un, ou indépendamment les uns des autres, deux ou trois des substituants suivants : halogéno, cyano, alkyle en C1-C4, (alcoxy en C1-C4) alkyle, halogénoalkyle en C1-C4, alcoxy en C1-C4, halogénoalcoxy en C1-C4, alkylthio en C1-C4, (alcoxy en C1-C4)carbonyle, aryle, aryloxy et hétéroaryle dans lesquels les parties cycliques peuvent elles mêmes porter un, ou, indépendamment les uns des autres, deux ou trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4 et (alcoxy en C1-C4)carbonyle ;

m est égal à 1.

**2.** Carbamoylcarboxamidoximes de formule I de la revendication 1, dans laquelle les symboles ci-après, isolément ou en combinaison entre eux, ont les significations suivantes :

$R^1$ : un groupe isopropyle, sec-butyle, tert-butyle ou phényle ;

$R^2$ : l'hydrogène ;

$R^3$ et $R^4$ : l'un de ces symboles représente l'hydrogène et l'autre un groupe isopropyle, l'atome de carbone portant les substituants $R^3$ et $R^4$ étant en configuration S ;

$R^5$ : l'hydrogène ;

A : un groupe $CH_2$-$CH_2$, $CH_2$, $C(CH_3)_2$, $CH(CH_3)CH_2$, $CH(CH_3)$, le groupe $CH(CH_3)$ étant à l'état de racémique ou en configuration R ;

Y : un groupe phényle, l'hydrogène ou un groupe méthyle ;

Z : un groupe $CH_2O$, $CH_2$-$CH_2$, $CH(CH_3)$ ou $CH_2$;

n : est égal à 1 ou 2,

Ar représente un groupe phényle qui peut être substitué par le chlore, des groupes méthyle, méthoxy ou cyano.

3. Procédé pour la préparation des carbamoylcarboxamidoximes de formule I selon la revendication 1 ou 2, **caractérisé par le fait que** l'on fait réagir un acide carbamoylcarboxylique de formule générale II

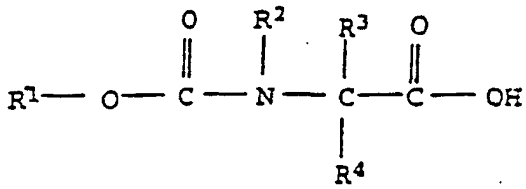

(II)

avec une amine de formule générale III

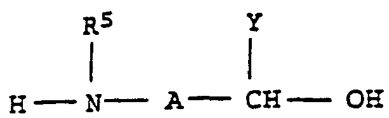

(III)

ce qui donne l'amide IV

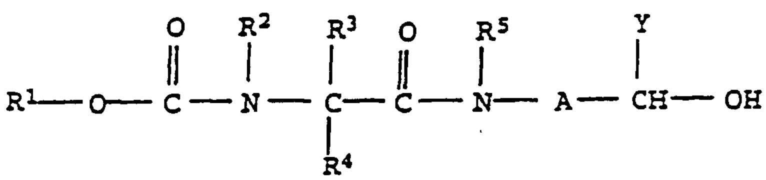

(IV)

qu'on oxyde ensuite en le dérivé carbonylé V

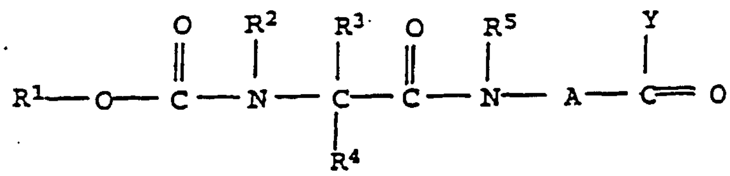

(V)

qu'on fait ensuite réagir avec une oxyamine VI

$$H_2N—O—Z_n— Ar_m \qquad (VI)$$

4. Procédé pour la préparation des carbamoylcarboxamides de formule I selon la revendication 1 ou 2, **caractérisé par le fait que**

   a) on convertit un carbamoylcarboxamide de formule générale I

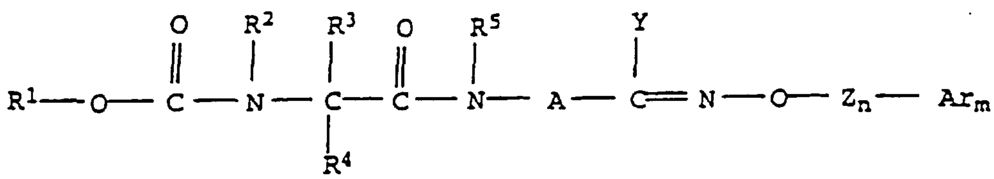

(I)

   dans laquelle le groupe $R^1$-O-(CO) est un groupe protecteur qu'on peut scinder de manière connue en soi, en un amide d'aminoacide VII

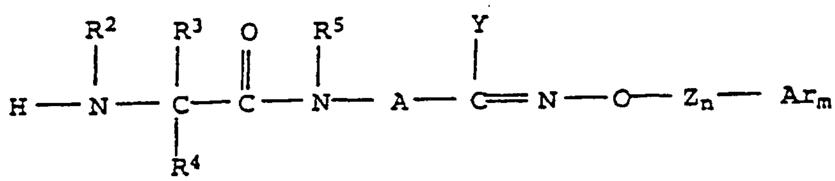

(VII)

   et

   b) on fait réagir cet amide d'aminoacide VII avec une chloroformyloxime de formule générale VIII

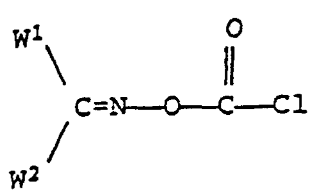

(VIII)

   en présence d'une base.

5. Produit pour combattre les mycètes nuisibles, contenant une quantité fongicide efficace d'un composé de formule I ou de l'un de ses sels selon la revendication 1 ou 2, et au moins un produit auxiliaire usuel de formulation.

6. Procédé pour combattre les mycètes nuisibles, **caractérisé par le fait que** l'on traite les mycètes nuisibles, leur habitat ou les végétaux, aires, matériaux ou locaux qu'on veut protéger contre les mycètes par une quantité efficace

d'un composé de formule générale I ou de l'un de ses sels selon la revendication 1 ou 2 ou d'un produit selon la revendications 5.

7. Utilisation des composés de formule générale I selon la revendication 1 ou 2, pour la lutte contre les mycètes nuisibles.